(19) Europäisches Patentamt / European Patent Office / Office européen des brevets

(11) EP 1 727 551 B1

(12) EUROPEAN PATENT SPECIFICATION

(45) Date of publication and mention of the grant of the patent: **09.09.2009 Bulletin 2009/37**

(21) Application number: **05728747.6**

(22) Date of filing: **18.03.2005**

(51) Int Cl.: ***A61K 31/5513*** *(2006.01)*

(86) International application number: **PCT/GB2005/001029**

(87) International publication number: **WO 2005/089771 (29.09.2005 Gazette 2005/39)**

(54) **PHARMACEUTICAL COMPOSITION COMPRISING A BENZODIAZEPINE DERIVATIVE AND A INHIBITOR OF THE RSV FUSION PROTEIN**

PHARMAZEUTISCHE ZUSAMMENSETZUNG MIT EINEM BENZODIAZEPIN-DERIVAT UND EINEM HEMMER DES RSV-FUSIONSPROTEINS

COMPOSITION PHARMACEUTIQUE COMPRENANT UN DERIVE DE BENZODIAZEPINE ET UN INHIBITEUR DE LA PROTEINE DE FUSION DU VIRUS RESPIRATOIRE SYNCYTIAL

(84) Designated Contracting States:
**AT BE BG CH CY CZ DE DK EE ES FI FR GB GR HU IE IS IT LI LT LU MC NL PL PT RO SE SI SK TR**

(30) Priority: **19.03.2004 GB 0406279**

(43) Date of publication of application: **06.12.2006 Bulletin 2006/49**

(73) Proprietor: **Arrow Therapeutics Limited London SE1 1DA (GB)**

(72) Inventors:
- **POWELL, Kenneth, Arrow Therapeutics Limited London SE1 1DA (GB)**
- **KELSEY, Richard, Arrow Therapeutics Limited London SE1 1DA (GB)**
- **CARTER, Malcoml, Arrow Therapeutics Limited London SE1 1DA (GB)**
- **DOWDELL, Verity, Arrow Therapeutics Limited London SE1 1DA (GB)**
- **ALBER, Dagmar, Arrow Therapeutics Limited London SE1 1DA (GB)**
- **HENDERSON, Elisa, Arrow Therapeutics Limited London SE1 1DA (GB)**

(74) Representative: **Bachelin, Karl Martin Raimund et al Hoffmann Eitle Patent- und Rechtsanwälte Arabellastrasse 4 81925 München (DE)**

(56) References cited:
**EP-A- 0 491 218**
**WO-A-03/053344**
**WO-A-20/04026843**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a series of anti-viral benzodiazepine derivatives according to formula (V). In particular, it relates to a series of benzodiazepine derivatives which interact with an inhibitor of the RSV fusion protein to provide an additive or synergistic therapeutic effect in treating or preventing an RSV infection.

Respiratory Syncytial Virus (RSV) is a major cause of respiratory illness in patients of all ages. In adults, it tends to cause mild cold symptoms. In school-aged children, it can cause a cold and bronchial cough. In infants and toddlers it can cause bronchiolitis (inflammation of the smaller airways of the lungs) or pneumonia. It has also been found to be a frequent cause of middle ear infections (otitis media) in pre-school children. RSV infection in the first year of life has been implicated in the development of asthma during childhood.

Current anti-RSV therapy involves the use of a monoclonal antibody to RSV, called palivizumab. Such use of palivizumab is a prophylactic, rather than therapeutic, treatment of RSV. However, although this antibody is often effective, it is expensive. Indeed, its expense means that it is unavailable for many people in need of anti-RSV therapy. There is therefore an urgent need for effective alternatives to existing anti-RSV therapy.

Small compounds which inhibit RSV replication by inhibiting the fusion (F) protein of RSV block the entry of the virus into the host cell and the exit from the host cell via syncytia formation. While these compounds have been shown to have high potency, RSV rapidly develops resistance to these compounds through mutations in the F protein (Morton, C.J. et al, 2003.Virology 311, 275-288).

PCT/GB03/04050 filed on 20 September 2003 discloses a series of benzodiazepine derivatives which inhibit RSV replication. Serial passaging experiments have indicated that resistance to these inhibitors is slow to develop and sequencing of resistant mutants did not reveal any significant changes in the F protein. It can therefore be assumed that these benzodiazepines have a common and novel mode of action, which does not involve inhibition of the F-protein.

It has now surprisingly been shown that a combination of (a) an RSV fusion protein inhibitor and (b) an anti-RSV benzodiazepine according to formula (V) is highly active against RSV. Components (a) and (b) are found to have at least an additive effect. Further, it is also a finding of the invention that the two components interact synergistically, to provide a combined effect that is greater than the sum of the effects of the individual components.

The present invention therefore provides, in a first embodiment, a pharmaceutical composition which comprises a pharmaceutically acceptable carrier or diluent and

(a) an inhibitor of the RSV fusion protein; and:
(b) a benzodiazepine derivative capable of inhibiting RSV replication.

It is a finding of the present invention that components (a) and (b) have at least an additive effect. The concepts of synergism and additivity are, of course, well known in the field of pharmacology. It is thus well established that a therapeutically useful additive combination is one in which the effect of the combination is greater than the larger of the effects produced by each of the components at the same concentrations as in the mixture. Thus, in the present case, a given formulation containing x wt% of component (a) and y wt% of component (b) has an activity which is at least as great as the activity of a formulation containing, as sole active ingredient, either x wt% component (a) or y wt% component (b).

In such additive combinations, the active ingredients are typically operating via different physiological pathways. In the present case, for example, component (a) and component (b) are believed to be inhibiting separate RSV proteins. An additive combination is therapeutically useful because it can achieve a therapeutically useful effect using lower concentrations of each active component. This enables the side-effects of the medication to be minimised. Thus, the additive combination can be formulated so that each active ingredient is present at a concentration which is subclinical in cells other than the target disease cells. The additive combination is nevertheless therapeutically effective in target cells which respond to both ingredients.

As regards component (a), an inhibitor of the RSV fusion protein can be identified by an assay comprising:

(a) labelling RSV with octadecyl rhodamine dye (R18);
(b) pre-incubating the labelled virus with Hep-2 cells seeded in a 6-well plate at 1 hour for 4°C;
(c) removing unattached virus;
(d) adding the candidate fusion protein inhibitor;
(e) incubating the 6-well plates at 37°C for 1 hour; and
(f) determining any increase in fluorescence, typically using a fluorescence microscope.

In the above assay, any increase in fluorescence signifies a fusion event. Thus, if no increase in fluorescence is detected, 100% inhibition is achieved. If the increase in fluorescence is equal to that observed with a corresponding assay in which a control of growth medium and solvent (e.g., growth medium with 10% fetal bovine serum and DMSO)

is used in step (d) in place of the candidate fusion protein inhibitor, 0% inhibition is achieved. Accordingly the % inhibition achieved with the candidate fusion protein inhibitor can be determined by quantitative assessment of the fluorescence in step (f).

As used herein, component (a) is typically a compound which achieves at least 10%, more typically at least 30%, preferably at least 50% and most preferably at least 75%, inhibition of the RSV fusion protein as determined by the above assay.

Typically, component (a) is a compound of formula (I), or a pharmaceutically acceptable salt thereof,

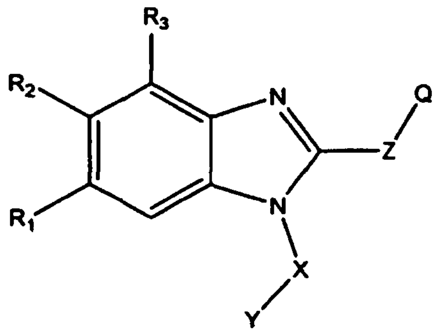

(I)

wherein:

- X is a direct link or $C_{1-6}$ alkyl; said $C_{1-6}$ alkyl being optionally substituted with halogen, oxo, cyano, hydroxyl, $OCOR_4$ or S(O)n-$C_{1-6}$ alkyl;
- Y is $R_4$, $NR_4R_5$, $NCOR_4$, =N-$OR_4$, -$CONHR_4$, $COOR_4$, -$OR_4$, aryl, heteroaryl, cyclyl or heterocyclyl, where $R_4$ and $R_5$ are H or $C_{1-6}$ alkyl;
- Z is $CR_6R_7$, where $R_6$ and $R_7$ are independently H, or straight, branched or cyclic $C_{1-6}$ alkyl;
- n is 1-2;
- $R_1$ is $CONR_4R_5$, $CO_2R_4$ or $C_{1-6}$ alkyl, said $C_{1-6}$ alkyl can be optionally substituted with $OR_4$ or $NR_8R_9$;
- $R_8$ and $R_9$ are each independently H, $C_{1-6}$ alkyl, $SO_2R_5$, $CO_2R_4$ or $COR_4$;
- $R_2$ is selected from the group consisting of $NH_2$, $CONR_6R_7$, heteroaryl, $C_{2-6}$ alkenyl, $CO_2R_4$, N=$CPh_2$, C(=NH)$NH_2$ and $C_{1-6}$ alkyl; said alkyl optionally substituted with a member selected from the group consisting of halogen, CN, $NR_{10}R_{11}$, OSO2$R_4$ and $OR_4$;
- $R_{10}$ and $R_{11}$ are each independently selected from the group consisting of H, $C_{1-6}$ alkyl, $C_{3-6}$ cycloalkyl, $CO_2R_4$, $COR_4$ and $SO_2R_4$;
- $R_3$ is selected from the group consisting of (1) $CO_2R_9$; (2) $C_{1-6}$ alkyl optionally substituted with CN, $OR_4$ or $NR_6R_7$; and (3) $C_{2-6}$ alkenyl substituted with CN;
- Q is a member selected from the group consisting of

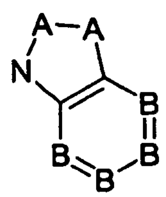

A is C or N, optionally substituted with H, halogen, $C_{1-6}$ alkyl, $C_{2-6}$ alkenyl, cyano-$C_{1-6}$- alkyl, $CO_2R_4$, aryl, benzoaminocarbonyl, hydroxybenzyl, $SO_2NR_4R_5$ or $C_{3-6}$ cycloalkyl. Where A is carbon, it may also be optionally substituted by O or S via a double bond;

B is C or N; where B is C it may be optionally substituted by H, $C_{1-6}$ alkyl, $NO_2$, CN, halogen, $COR_4$, $COOR_4$, $CONHR_4C$ (=NH)$NH_2$ or C(=NOH)$NH_2$.

Typically, at least two of $R_1$, $R_2$ and $R_3$ are hydrogen, and the other is hydrogen or -C(NH)-$NH_2$. Preferably, all of $R_1$, $R_2$ and $R_3$ are hydrogen.

Typically, either -X-Y is H, or X is a $C_1$-$C_6$ alkylene group which is unsubstituted or substituted by a hydroxy group and Y is H, OH, CN, -NR'R", -COR', -$SO_2$R' or phenyl, wherein R' and R" are the same or different and represent

a $C_1$-$C_4$ alkyl group.

Typically, Z is $-CH_2-$.

Typically, Q is a moiety

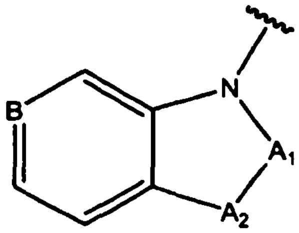

or

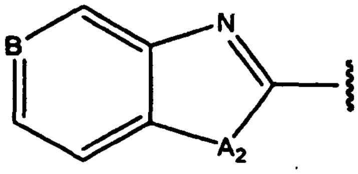

wherein B is -CH- or -N-, $A_1$ is -C(O)- or -NH- and $A_2$ is $-CH_2-$, -CHR'- or -NR"-, wherein R' is a halogen atom and R" represents a hydrogen atom or a $C_1$-$C_4$ alkyl, $C_2$-$C_4$ alkenyl, $C_3$-$C_6$ cycloalkyl, $-SO_2-(C_1-C_6$ alkyl), $-SO_2-N(C_1-C_6$ alkyl$)_2$ or $-(CO-NH)_a-(C_1-C_4$ alkyl)-phenyl group, wherein a is 0 or 1, which group is unsubstituted or is substituted with a hydroxy or cyano substituent.

Particularly preferred compounds of the invention are compounds of formula (Ia) and pharmaceutically acceptable salts thereof

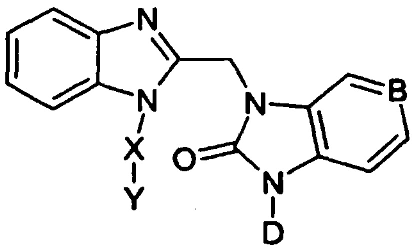

;

(Ia)

wherein

- B, X and Y are as described in formula (I) above
- D is cyclopropyl, ethyl, 4-cyanobutyl, isopropenyl, methylsulfonyl, dimethylsulfamoyl, benzylaminocarbamoyl or para-hydroxybenzyl

Component (a) can also be a compound of formula (II), or a pharmaceutically acceptable salt thereof,

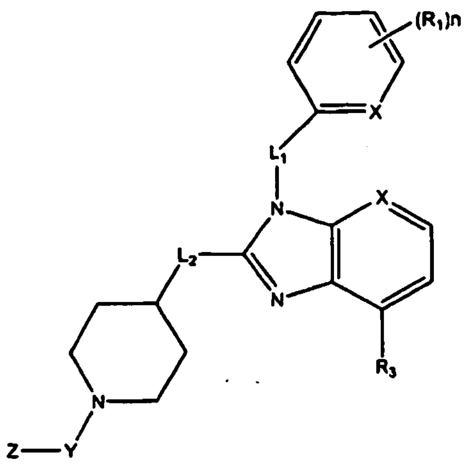

(II)

wherein:

- $L_1$ is $-CH_2-$ or $-CHR_2-CO-$
- each X is the same or different and CH or N;
- each $R_1$ is the same or different and is $C_{1-6}$ alkyl, halogen, hydroxy, phenyl or $(CH_2)_m=NH_2$;
- n is 1 or 2;
- $R_2$ is $C_{1-6}$ alkoxy or $C_{1-6}$alkoxy-phenyl;
- $R_3$ is $C_{1-6}$alkyl;
- $L_2$ is $-CH_2-$ or -NH-;
- Y is $C_{1-6}$ alkyl or $C_{1-6}$ alkenyl;
- Z is H, $N(R_4)_2$, $-C(=O)-R_5$, $-C(=CH_2)-R_5$, $-CH(OH)-R_5$, -CH(CH3)-Rs, $-CH(OCH3)-R_5$;
- each $R_4$ is the same or different and is H, $C_{1-6}$ alkyl;
- $R_5$ is $C_{1-6}$ alkyl-carbonyl, amino, hydroxyl, aryl, heteroaryl, carbocyclyl, heterocyclyl; and
- m=1-6

For the avoidance of doubt, when $L_1$ is $-CHR_2-CO-$, the carbonyl group is attached to the phenyl or pyridyl moiety.

Typically, $L_1$ is $-CH_2-$.

Typically, $L_2$ is -NH-.

Typically, $R_1$ is methyl or hydroxy. Typically, n is 2. Typically, each $R_1$ is different.

Typically, Y is $C_1$-$C_4$ alkyl.

Typically, Z is $-NH_2$.

Other preferred compounds of formula (II) are compounds of formula

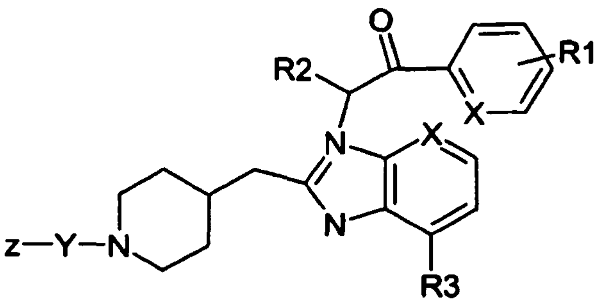

(II)

wherein:

- X is C or N;
- $R_1$ is $C_{1-6}$ alkyl, halogen, phenyl or $(CH_2)_m=NH_2$;
- $R_2$ is $C_{1-6}$ alkoxy or $C_{1-6}$alkoxy-phenyl;
- $R_3$ is $C_{1-6}$alkyl;
- Y is $C_{1-6}$ alkyl or $C_{1-6}$ alkenyl;
- Z is H, $NR_4$, -C(=O)-$R_5$, -C(=$CH_2$)-$R_5$, -CH(OH)-$R_5$, -CH(CH3)-$R_5$, -CH(OCH3)-$R_5$;
- $R_4$ is H, $C_{1-6}$ alkyl.
- $R_5$ is $C_{1-6}$ alkyl-carbonyl, amino, hydroxyl, aryl, heteroaryl, carbocyclyl, heterocyclyl
- m=1-6

Component (a) can also be a compound of formula (III), or a pharmaceutically acceptable salt thereof,

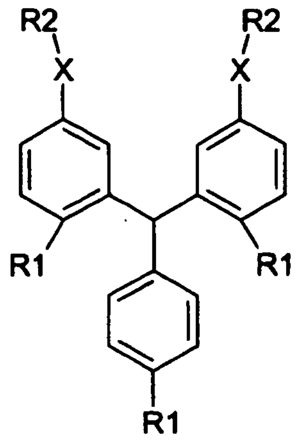

(III)

wherein

- X is -N=C- or -CH=CH-;
- $R_1$ is H, hydroxyl, alkyl, halogen, nitro or alkoxy; said alkoxy being optionally monosubstituted with carboxy, amino, monoalkylamino, dialkylamino or acetoamino;
- $R_2$ is pyrazolyl, triazolyl or tetrazolyl and optionally substituted by amino or alkyl.

Component (a) can also be a compound of formula (IV), or a pharmaceutically acceptable salt thereof.

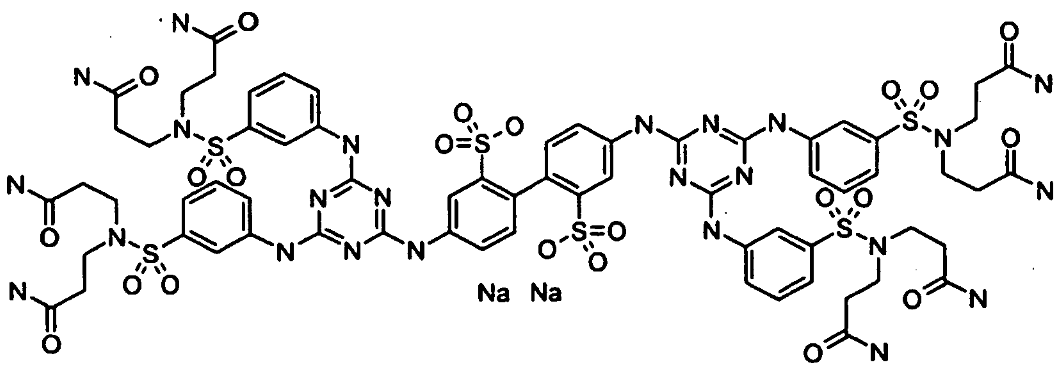

The compound of formula (IV) is 4,4'-Bis-(4,6-bis-{3-[bis-(2-carbamoyl-ethyl)-sulfamoyl]-phenylamino}-[1,3,5] triazin-2-ylamino)-biphenyl-2,2'-disulfonic acid.

Preferably, component (a) is:

1-Cyclopropyl-3-[1-(4-hydroxy-butyl)-1H-benzoimidazol-2-ylmethyl]-1,3-dihydro-imidazo[4,5-c]pyridin-2-one
{2-[2-(1,2-Dihydro-benzotriazol-1-ylmethyl)-benzoimidazol-1-yl]]ethyl}-diethylamine
{2-[2-(3-Iodo-2,3-dihydro-indazol-1-ylmethyl)-benzimidazol-1-yl]-ethyl}-dimethylamine
1-Isopropenyl-3-[1-(3-methyl-butyl)-1H-benzoimidazol-2-ylmethyl]-1,3-dihydro-benzoimidazol-2-one
1-(4-Hydroxy-benzyl)-3-[1-(3-methyl-butyl)-1H-benzoimidazol-2-ylmethyl]-1,3-dihydro-benzoimidazol-2-one
1-Isopropenyl-3-[1-(3-oxo-butyl)-1H-benzoimidazol-2-ylmethyl]-1,3-dihydro-benzoimidazol-2-one
1-Ethyl-3-[1-(2-hydroxy-2-phenyl-ethyl)-1H-benzoimidazol-2-ylmethyl]-1,3-dihydro-benzoimidazol-2-one
1-Ethyl-3-[1-(4-hydroxy-butyl)-1H-benzoimidazol-2-ylmethyl]-1,3-dihydro-benzoimidazol-2-one
7-[2-(3-Isopropenyl-2-oxo-2,3-dihydrobenzoimidazol-1-ylmethyl)-benzoimidazol-1-yl]-heptanenitril
5-{3-[1-(3-Methanesulfonyl-propyl)-1H-benzoimidazol-2-ylmethyl]-2-oxo-2,3-dihydro-benzoimidazol-1-yl}-pentanenitrile
3-[1-(3-Methyl-butyl)-1H-benzoimidazol-2-ylmethyl]-2-oxo-2,3-dihydro-benzoimidazol-1-carboxylic acid benzylamide
1-Methanesulfonyl-3-[1-(3-methyl-butyl)-1H-benzoimidazol-2-ylmethyl]-1,3-dihydro-benzoimidazol-2-one
3-[1-(3-Methyl-butyl)-1H-benzoimidazol-2-ylmethyl]-2-oxo-2,3-dihydro-benzoimidazol-1-sulfonic acid dimethylamide
1-Isopropenyl-3-(1-propyl-1H-benzoimidazol-2-ylmethyl)-1,3-dihydro-imidazo[4,5-c]pyridine-2-one
Bis(5-amidino-2-benzimidazolyl)-methane
2-{2-[1-[1-(2-Amino-ethyl)-piperidin-4-ylamino]-4-methyl-benzoimidazol-1-ylmethyl}-6-methyl-pyridin-3-ol

or a pharmaceutically acceptable salt thereof.

In a further embodiment, the composition contains an RSV fusion inhibitor, as described above, and a benzodiazepine identifiable as having anti-RSV activity by the method of Example 8.

Component (b) is a compound of formula (V), or a pharmaceutically acceptable salt thereof,

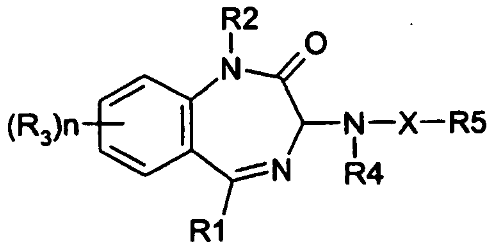

(V)

wherein:

- $R^1$ represents $C_{1-6}$ alkyl, aryl or heteroaryl;
- $R^2$ represents hydrogen or $C_{1-6}$ alkyl;
- each $R^3$ is the same or different and represents halogen, hydroxy, $C_{1-6}$ alkyl, $C_{1-6}$ alkoxy, $C_{1-6}$ alkylthio, $C_{1-6}$ haloalkyl, $C_{1-6}$ haloalkoxy, amino, mono($C_{1-6}$ alkyl)amino, di($C_{1-6}$ alkyl)amino, nitro, cyano, $-CO_2R'$, -CONR'R", -NH-CO-R', -S(O)R', $-S(O)_2R'$, $-NH-S(O)_2R'$, -S(O)NR'R" or $-S(O)_2NR'R"$, wherein each R' and R" is the same or different and represents hydrogen or $C_{1-6}$ alkyl;
- n is from 0 to 3;
- $R^4$ represents hydrogen or $C_{1-6}$ alkyl;
- X represents -CO-, -CO-NR'-, -S(O)- or $-S(O)_2-$, wherein R' is hydrogen or a $C_1$-$C_6$ alkyl group; and
- $R^5$ represents an aryl, heteroaryl or heterocyclyl group which is substituted by a $C_1$-$C_6$ hydroxyalkyl group or a $-(C_1$-$C_4$ alkyl)-$X_1$-($C_1$-$C_4$ alkyl)-$X_2$-($C_1$-$C_4$ alkyl) group, wherein $X_1$ represents -O-, -S- or -NR'-, wherein R' represents H or a $C_1$-$C_4$ alkyl group, and $X_2$ represents -CO-, -SO- or $-SO_2-$, or $R_5$ represents $-A_1$-Y-$A_2$, wherein:

  - $A_1$ is an aryl, heteroaryl, carbocyclyl or heterocyclyl group;
  - Y represents a direct bond or a $C_1$-$C_4$ alkylene, $-SO_2-$, -CO-, -O-, -S- or -NR'-moiety, wherein R' is a $C_1$-$C_6$ alkyl group; and
  - $A_2$ is an aryl, heteroaryl, carbocyclyl or heterocyclyl group.

As used herein, a $C_{1-6}$ alkyl group or moiety is a linear or branched alkyl group or moiety containing from 1 to

6 carbon atoms, such as a $C_{1-4}$ alkyl group or moiety. Examples of $C_{1-4}$ alkyl groups and moieties include methyl, ethyl, n-propyl, *i*-propyl, *n*-butyl, *i*-butyl and t-butyl. For the avoidance of doubt, where two alkyl moieties are present in a group, the alkyl moieties may be the same or different.

As used herein, a hydroxyalkyl group is typically a said alkyl group that is substituted by one or more hydroxy groups. Typically, it is substituted by one, two or three hydroxy groups. Preferably, it is substituted by a single hydroxy group. A preferred hydroxyalkyl group is $-CH_2-OH$.

As used herein, an acyl group is a $C_{2-7}$ acyl group, for example a group -CO-R, wherein R is a said $C_{1-6}$ alkyl group.

As used herein, an aryl group is typically a $C_{6-10}$ aryl group such as phenyl or naphthyl. Phenyl is preferred. An aryl group may be unsubstituted or substituted at any position. Typically, it carries 0, 1, 2 or 3 substituents.

Suitable substituents on an aryl group include halogen, $C_{1-6}$ alkyl, $C_{2-7}$ acyl, hydroxy, $C_{1-6}$ alkoxy, $C_{1-6}$ alkylthio, $C_{1-6}$ haloalkyl, $C_{1-6}$ haloalkoxy, nitro, cyano, carbamoyl, mono($C_{1-6}$ alkyl)carbamoyl, di($C_{1-6}$ alkyl)carbamoyl, amino, mono($C_{1-6}$ alkyl)amino, di($C_{1-6}$ alkyl)amino, $-CO_2R'$, -CONR'R", -S(O)R', $-S(O)_2R'$, -S(O)NR'R",$-S(O)_2NR'R"$ -NH-S$(O)_2R'$ or -NH-CO-R', wherein each R' and R" is the same or different and represents hydrogen or $C_{1-6}$ alkyl.

Preferred substituents on an aryl group include halogen, $C_{1-6}$ alkyl, $C_{2-7}$ acyl, hydroxy, $C_{1-6}$ alkoxy, $C_{1-6}$ alkylthio, $C_{1-6}$ haloalkyl, $C_{1-6}$ haloalkoxy, amino, mono($C_{1-6}$ alkyl)amino, di($C_{1-6}$ alkyl)amino, nitro, cyano, $-CO_2R'$, -S(O)R', -S$(O)_2R'$ and $-S(O)_2NR'R"$, wherein each R' and R" is the same or different and represents hydrogen or $C_{1-4}$ alkyl.

Particularly preferred substituents include fluorine, chlorine, bromine, iodine, cyano, $C_{1-4}$ alkyl, $C_{2-4}$ acyl, hydroxy, $C_{1-4}$ alkoxy, $C_{1-4}$ alkylthio, $C_{1-4}$ haloalkyl, $C_{1-4}$ haloalkoxy, amino, mono($C_{1-4}$ alkyl)amino, di($C_{1-4}$ alkyl)amino, nitro, $-CO_2R'$, $-S(O)_2R'$ and $-S(O)_2NH_2$, wherein R' represents $C_{1-2}$ alkyl. Most preferred substituents are chlorine, fluorine, cyano, $C_1$-$C_4$ alkyl and $C_1$-$C_4$ haloalkyl substituents.

As used herein, references to an aryl group include fused ring systems in which an aryl group is fused to a monocyclic carbocyclyl, heterocyclyl or heteroaryl group or to a fused group which is a monocyclic carbocyclyl, heterocyclyl or heteroaryl group which is fused to a phenyl ring. Typically, said fused ring systems are systems in which an aryl group is fused to a monocyclic carbocyclyl, heterocyclyl or heteroaryl group.

Preferred such fused ring systems are those wherein an aryl group is fused to a monocyclic heterocyclyl or heteroaryl group or to a monocyclic carbocyclic group fused to a phenyl ring, in particular those wherein an aryl group is fused to a heterocyclyl or heteroaryl group. Examples of such fused ring systems are groups in which a phenyl ring is fused to a thienyl group or to a tetrahydrofuranyl group to form a benzothienyl or dihydrobenzofuranyl group. Further examples of such fused rings are groups in which a phenyl ring is fused to a dioxanyl group, a pyrrolyl group or a 2,3-dihydroinden-1-one group to form a benzodioxinyl, indolyl or a 9H-fluoren-9-one group. Most preferably, however, an aryl group, as used herein, is not fused to a monocyclic carbocyclyl, heterocyclyl or heteroaryl group or to a said fused group.

As used herein, a carbocyclyl group is a non-aromatic saturated or unsaturated monocyclic hydrocarbon ring, typically having from 3 to 6 carbon atoms. Preferably it is a saturated hydrocarbon ring (i.e. a cycloalkyl group) having from 3 to 6 carbon atoms. Examples include cyclopropyl, cyclobutyl, cyclopentyl and cyclohexyl. It is preferably cyclopropyl, cyclopentyl or cyclohexyl, most preferably cyclopropyl. A cycloalkyl group may be unsubstituted or substituted at any position. Typically, it carries 0, 1, 2 or 3 substituents.

Suitable substituents on a carbocyclyl group include halogen, $C_{1-6}$ alkyl, $C_{2-7}$ acyl, hydroxy, $C_{1-6}$ alkoxy, $C_{1-6}$ alkylthio, $C_{1-6}$ haloalkyl, $C_{1-6}$ haloalkoxy, nitro, cyano, carbamoyl, mono($C_{1-6}$ alkyl)carbamoyl, di($C_{1-6}$ alkyl)carbamoyl, amino, mono($C_{1-6}$ alkyl)amino, di($C_{1-6}$ alkyl)amino, oxo, $-CO_2R'$, -CONR'R", -S(O)R', $-S(O)_2R'$, -S(O)NR'R", -S$(O)_2NR'R"$, $-NH-S(O)_2R'$ or -NH-CO-R', wherein each R' and R" is the same or different and represents hydrogen or $C_{1-6}$ alkyl.

Preferred substituents on an carbocyclyl group include halogen, $C_{1-6}$ alkyl, $C_{1-6}$ alkoxy, $C_{1-6}$ alkylthio, $C_{1-6}$ haloalkyl, $C_{1-6}$ haloalkoxy, mono($C_{1-6}$ alkyl)amino, di($C_{1-6}$ alkyl)amino, nitro, cyano and oxo. Particularly preferred substituents include fluorine, chlorine, bromine, $C_{1-4}$ alkyl, $C_{1-4}$ alkoxy, $C_{1-4}$ haloalkyl, nitro and oxo. Most preferably, a carbocyclyl group is unsubstituted.

As used herein, a heterocyclyl group is a non-aromatic saturated or unsaturated carbocyclic ring, typically having from 5 to 10 carbon atoms, in which one or more, for example 1, 2 or 3, of the carbon atoms is replaced by a heteroatom selected from N, O and S. Saturated heterocyclyl groups are preferred. Examples include tetrahydrofuranyl, tetrahydrothienyl, pyrrolidinyl, imidazolidinyl, pyrazolidinyl, dioxolanyl, thiazolidinyl, tetrahydropyranyl, piperidinyl, dioxanyl, piperazinyl, morpholinyl, thiomorpholinyl and thioxanyl. Further examples include dithiolanyl, oxazolidinyl, tetrahydrothiopyranyl and dithianyl. Piperazinyl, piperidinyl, thiomorpholinyl, imidazolidinyl and morpholinyl groups are preferred.

As used herein, references to a heterocyclyl group include fused ring systems in which a heterocyclyl group is fused to a phenyl group. Preferred such fused ring systems are those wherein a 5- to 6-membered heterocyclyl group is fused to a phenyl group. An example of such a fused ring system is a group wherein a 1H-imidazol-2(3*H*)-onyl group or a imidazolidin-2-onyl group is fused to a phenyl ring or a pyridine ring, to form, for example, a 1*H*-benzo[*d*]imidazol-2(3*H*)-onyl group or a 1H-imidazo[4,5-b]pyridin-2(3H)-one group. Most preferably, however, a heterocyclyl group is

monocyclic.

A heterocyclic group may be unsubstituted or substituted at any position. Typically, it carries 0, 1 or 2 substituents.

Suitable substituents on a heterocyclyl group include halogen, $C_{1-6}$ alkyl, $C_{2-7}$ acyl, hydroxy, $C_{1-6}$ alkoxy, $C_{1-6}$ alkylthio, $C_{1-6}$ haloalkyl, $C_{1-6}$ haloalkoxy, nitro, cyano, carbamoyl, mono($C_{1-6}$ alkyl)carbamoyl, di($C_{1-6}$ alkyl)carbomyl, amino, mono($C_{1-6}$ alkyl)amino, di($C_{1-6}$ alkyl)amino, oxo, $-CO_2R'$, -CONR'R", -S(O)R', $-S(O)_2R'$, -S(O)NR'R", $-S(O)_2N'R"$, $-NH-S(O)_2R'$ or -NH-CO-R', wherein each R' and R" is the same or different and represents hydrogen or $C_{1-6}$ alkyl.

Preferred substituents on a heterocyclyl group include halogen, $C_{1-6}$ alkyl, $C_{1-6}$ alkoxy, $C_{1-6}$ alkylthio, $C_{1-6}$ haloalkyl, $C_{1-6}$ haloalkoxy, mono($C_{1-6}$ alkyl)amino, di($C_{1-6}$ alkyl)amino, nitro, cyano and oxo. Particularly preferred substituents include fluorine, chlorine, bromine, $C_{1-4}$ alkyl, $C_{1-4}$ alkoxy, $C_{1-4}$ haloalkyl, nitro and oxo. Most preferably, a heterocyclyl group is unsubstituted or substituted by one or two $C_{1-2}$ alkyl or oxo groups. An example of a substituted heterocyclic group is S,S-dioxothiomorpholino.

As used herein, a halogen is typically chlorine, fluorine, bromine or iodine. It is preferably chlorine, fluorine or bromine. It is more preferably chlorine or fluorine.

As used herein, an alkoxy group is typically a said alkyl group attached to an oxygen atom. An alkylthio group is typically a said alkyl group attached to a thio group. A haloalkyl or haloalkoxy group is typically a said alkyl or alkoxy group substituted by one or more said halogen atoms. Typically, it is substituted by 1, 2 or 3 said halogen atoms. Preferred haloalkyl and haloalkoxy groups include perhaloalkyl and perhaloalkoxy groups such as $-CX_3$ and $-OCX_3$ wherein X is a said halogen atom, for example chlorine or fluorine. Particularly preferred haloalkyl groups are $-CF_3$ and $-CCl_3$. Particularly preferred haloalkoxy groups are $-OCF_3$ and $-OCCl_3$.

As used herein, a heteroaryl group is typically a 5- to 10-membered aromatic ring, such as a 5- or 6-membered ring, containing at least one heteroatom, for example 1, 2 or 3 heteroatoms, selected from O, S and N. Examples include pyridyl, pyrazinyl, pyrimidinyl, pyridazinyl, furanyl, thienyl, pyrazolidinyl, pyrrolyl, oxadiazolyl, isoxazolyl, thiadiazolyl, thiazolyl, imidazolyl and pyrazolyl groups. Further examples include oxazolyl and isothiazolyl. Preferred heteroaryl groups are pyridyl, thienyl, oxazolyl, isoxazolyl, thiazolyl, furanyl and pyrazolyl.

As used herein, references to a heteroaryl group include fused ring systems in which a heteroaryl group is fused to a phenyl group or to a monocyclic heterocyclyl group. Preferred such fused ring systems are those wherein a 5- to 6-membered heteroaryl group is fused to a phenyl group or to a 5- to 6- membered heterocyclyl group. Examples of such fused ring systems are benzofuranyl, benzothiophenyl, indolyl, benzimidazolyl, benzoxazolyl, quinolinyl, quinazolinyl, isoquinolinyl and 1H-imidazo[4,5-b]pyridin-2(3H)-one moieties. Most preferably, said fused ring system is a 1H-imidazo[4,5-b]pyridin-2(3H)-one moiety.

A heteroaryl group may be unsubstituted or substituted at any position. Typically, it carries 0, 1, 2 or 3 substituents.

Suitable substituents on a heteroaryl group include halogen, $C_{1-6}$ alkyl, $C_{2-7}$ acyl, hydroxy, $C_{1-6}$ alkoxy, $C_{1-6}$ alkylthio, $C_{1-6}$ haloalkyl, $C_{1-6}$ haloalkoxy, nitro, cyano, carbamoyl, mono($C_{1-6}$ alkyl)carbamoyl, di($C_{1-6}$ alkyl)carbamoyl, amino, mono($C_{1-6}$ alkyl)amino, di($C_{1-6}$ alkyl)amino, $-CO_2R'$, -CONR'R", -S(O)R', $-S(O)_2R'$, -S(O)NR'R",$-S(O)_2NR'R"$, $-NH-S(O)_2R'$ or -NH-CO-R', wherein each R' and R" is the same or different and represents hydrogen or $C_{1-6}$ alkyl.

Preferred substituents on a heteroaryl group include halogen, $C_{1-6}$ alkyl, $C_{1-6}$ alkoxy, $C_{1-6}$ alkylthio, $C_{1-6}$ haloalkyl, $C_{1-6}$ haloalkoxy, mono($C_{1-6}$ alkyl)amino, di($C_{1-6}$ alkyl)amino, nitro and cyano. Particularly preferred substituents include fluorine, chlorine, bromine, $C_{1-4}$ alkyl, $C_{1-4}$ alkoxy, $C_{1-4}$ haloalkyl and nitro. Most preferred substituents include fluorine, chlorine, bromine, $C_{1-2}$ alkyl and $C_{1-2}$ haloalkyl substituents.

When $R^1$ in the formula (V) is an aryl or heteroaryl group it is typically unsubstituted or substituted by one, two or three substituents selected from halogen, $C_{1-6}$ alkyl, $C_{1-6}$ alkoxy, $C_{1-6}$ alkylthio, $C_{1-6}$ haloalkyl or $C_{1-6}$ haloalkoxy. Preferably, it is unsubstituted or substituted by one or two substituents selected from fluorine, chlorine, bromine, $C_{1-4}$ alkyl, $C_{1-4}$ alkoxy, $C_{1-4}$ alkylthio, $C_{1-4}$ haloalkyl or $C_{1-4}$ haloalkoxy. More preferably, it is unsubstituted or substituted by a single fluorine, chlorine, $C_{1-2}$ alkyl, $C_{1-2}$ alkoxy, $C_{1-2}$ alkylthio, $C_{1-2}$ haloalkyl or $C_{1-2}$ haloalkoxy substituent.

Typically, $R^1$ in formula (V) is $C_{1-6}$ alkyl or aryl. Preferably, $R^1$ is $C_{1-2}$ alkyl or aryl. More preferably, $R^1$ is $C_{1-2}$ alkyl or phenyl. More preferably, $R^1$ is an unsubstituted phenyl group.

Typically, $R^2$ in formula (V) is hydrogen or $C_{1-4}$ alkyl. Preferably, $R^2$ is hydrogen.

Typically, $R^3$ in formula (V) is halogen, hydroxy, $C_{1-4}$ alkyl, $C_{1-4}$ alkoxy, $C_{1-4}$ alkylthio, $C_{1-4}$ haloalkyl, $C_{1-4}$ haloalkoxy, amino, mono($C_{1-4}$ alkyl)amino or di($C_{1-4}$ alkyl)amino. Preferably, $R^3$ is fluorine, chlorine, bromine, $C_{1-2}$ alkyl, $C_{1-2}$ alkoxy, $C_{1-2}$ alkylthio, $C_{1-2}$ haloalkyl, $C_{1-2}$ haloalkoxy, amino, mono($C_{1-2}$ alkyl)amino or di($C_{1-2}$ alkyl) amino. More preferably, $R^3$ is methyl, trifluoromethyl, fluorine, chlorine or bromine. Most preferably, $R^3$ is methyl or chlorine.

Typically, n in formula (V) is 0, 1 or 2. Preferably, n is 0 or 1. Most preferably, n is 0.

Typically, $R^4$ in formula (V) is hydrogen or $C_{1-4}$ alkyl. Preferably, $R^4$ is hydrogen or $C_{1-2}$ alkyl. More preferably, $R^4$ is hydrogen or methyl. Most preferably, $R^4$ is hydrogen

Typically, X in formula (V) is -CO-, $-S(O)_2-$ or -CO-NR'-, wherein R' represents hydrogen or a $C_1$-$C_2$ alkyl group. Preferably, X is -CO- or -CO-NR'-.

When $R^5$ in formula (V) is a heterocyclyl or heterocyclyl group which is substituted by a $C_1$-$C_6$ hydroxyalkyl

group or a -($C_1$-$C_4$ alkyl)-$X_1$-($C_1$-$C_4$ alkyl)-$X_2$-($C_1$-$C_4$ alkyl) group, the heterocyclyl or heteroaryl group is typically a 5- or 6- membered ring. Preferably, it is a 5- or 6- membered heteroaryl group, for example a furanyl group.

Typically, the $C_1$-$C_6$ hydroxyalkyl group in formula (V) is a -$CH_2$-OH group. Typically, $X_1$ in the formula (V) is -NR'-, wherein R' is hydrogen or $C_1$-$C_2$ alkyl. Typically, $X_2$ in formula (V) is -S(O)$_2$-.

Typically, $A_1$ in formula (V) is an aryl or heteroaryl group. Preferably, $A_1$ is a monocyclic aryl or heteroaryl group, a naphthyl group or a heteroaryl group fused to a monocyclic oxo substituted heterocyclyl group. More preferably, $A_1$ is a phenyl group, a monocyclic 5- or 6- membered heteroaryl group or a 5- to 6- membered heteroaryl group fused to a monocyclic oxo substituted 5- to 6- membered heterocyclyl group (for example an oxo substituted imidazolidine group). Most preferably, $A_1$ is a phenyl, pyridyl, furanyl, thiazolyl, oxazolyl, isoxazolyl, thienyl or 1H-imidazo[4,5-b]pyridin-2-(3H)-one moiety.

Typically, the moiety $A_1$ in formula (V) is unsubstituted or substituted by 1 or 2 substituents selected from halogen, cyano, nitro, $C_1$-$C_4$ alkyl, $C_1$-$C_4$ haloalkyl and $C_1$-$C_4$ alkoxy substituents. Preferably, the substituents are selected from halogen, cyano, $C_1$-$C_2$ alkyl, $C_1$-$C_2$ haloalkyl and $C_1$-$C_2$ alkoxy substituents.

Typically, Y in formula (V) represents a direct bond, a $C_1$-$C_2$ alkylene group, -$SO_2$- or -O-.

Typically, $A_2$ in formula (V) is a phenyl, 5- to 6- membered heteroaryl, 5- to 6-membered heterocyclyl or $C_3$-$C_6$ cycloalkyl group. Preferably, $A_2$ is a piperazinyl, pyridyl, morpholinyl, thiomorpholinyl, pyrrolidinyl, piperidinyl, pyrazinyl, cyclopropyl or phenyl group.

Typically, when $A_2$ in formula (V) is a heterocyclyl group it is attached to the moiety Y via a N atom.

Typically, the moiety $A_2$ in formula (V) is unsubstituted or substituted by one or two substituents which are selected from $C_1$-$C_4$ alkyl and halogen substituents when $A_2$ is a heteroaryl or aryl group and which are selected from $C_1$-$C_4$ alkyl, halogen and oxo substituents when $A_2$ is a carbocyclic or heterocyclyl group.

Most preferably, $A_2$ in formula (V) is a piperazinyl, pyridyl, morpholinyl, pyrrolidinyl, piperidinyl, pyrazinyl, cyclopropyl, phenyl or S,S-dioxo-thiomorpholino group, which group is unsubstituted or is substituted by a $C_1$-$C_2$ alkyl group.

Preferred compounds of formula (V) are those in which:

- $R^1$ is $C_{1-6}$ alkyl or aryl;
- $R^2$ is hydrogen or $C_{1-4}$ alkyl;
- $R^3$ is halogen, hydroxy, $C_{1-4}$ alkyl, $C_{1-4}$ alkoxy, $C_{1-4}$ alkylthio, $C_{1-4}$ haloalkyl, $C_{1-4}$ haloalkoxy, amino, mono($C_{1-4}$ alkyl)amino or di($C_{1-4}$ alkyl)amino or, preferably, $R^3$ is fluorine, chlorine, bromine, $C_{1-2}$ alkyl, $C_{1-2}$ alkoxy, $C_{1-2}$ alkylthio, $C_{1-2}$ haloalkyl, $C_{1-2}$ haloalkoxy, amino, mono($C_{1-2}$ alkyl)amino or di ($C_{1-2}$ alkyl)amino;
- h is 0, 1 or 2;
- $R^4$ is hydrogen or $C_{1-4}$ alkyl;
- X is -CO-, -CO-NR' or -S(O)$_2$-, wherein R' is hydrogen or a $C_1$-$C_2$ alkyl group; and
- $R^5$ is a 5- or 6- membered heterocyclyl or heteroaryl ring which is substituted by a $C_1$-$C_6$ hydroxyalkyl group or a -($C_1$-$C_4$ alkyl)-$X_1$-($C_1$-$C_4$ alkyl)-$X_2$-($C_1$-$C_4$ alkyl) group, wherein $X_1$ and $X_2$ are as defined above, or $R^5$ represents -$A_1$-Y-$A_2$, wherein:
  - $A_1$ is an aryl or heteroaryl group;
  - Y is a direct bond, a $C_1$-$C_2$ alkylene group, -$SO_2$- or -O-; and
  - $A_2$ is an aryl, heteroaryl, heterocyclyl or carbocyclyl group,

the aryl moiety in the $R^1$ group being unsubstituted or substituted by 1, 2 or 3 substituents selected from halogen, $C_1$-$C_6$ alkyl, $C_1$-$C_6$ alkoxy, $C_1$-$C_6$ alkylthio, $C_1$-$C_6$ haloalkyl and $C_1$-$C_6$ haloalkoxy groups,
the $A_1$ moiety being unsubstituted or substituted by 1 or 2 substituents selected from halogen, cyano, nitro, $C_1$-$C_4$ alkyl, $C_1$-$C_4$ haloalkyl and $C_1$-$C_4$ alkoxy substituents; and
the $A_2$ moiety being unsubstituted or substituted by one or two substituents which are selected from $C_1$-$C_4$ alkyl and halogen substituents when $A_2$ is a heteroaryl or aryl group and which are selected from $C_1$-$C_4$ alkyl, halogen and oxo substituents when $A_2$ is a carbocyclic or heterocyclyl group.

Further preferred compounds of formula (V) are those wherein:

- $R^1$ is $C_{1-2}$ alkyl or phenyl;
- $R^2$ is hydrogen or $C_{1-4}$ alkyl;
- $R^3$ is methyl, trifluoromethyl, fluorine, chlorine or bromine;
- n is 0 or 1;
- $R^4$ is hydrogen or $C_{1-2}$ alkyl;
- X is -CO-, -CO-NR'- or -S(O)$_2$, wherein R' is hydrogen or a $C_1$-$C_2$ alkyl group; and
- $R^5$ is a 5- or 6- membered heterocyclyl or heteroaryl group which is substituted by a $C_1$-$C_6$ hydroxyalkyl group or a -($C_1$-$C_4$ alkyl)-NR'-($C_1$-$C_4$ alkyl)-$SO_2$-($C_1$-$C_4$ alkyl) group, wherein R' is hydrogen or $C_1$-$C_2$ alkyl, or $R^5$ represents

- $A_1$-Y-$A_2$, wherein:

  - $A_1$ is a phenyl group, a monocyclic 5- or 6- membered heteroaryl group or a 5- or 6- membered heteroaryl group fused to a monocyclic oxo-substituted 5- to 6-membered heterocyclyl group;
  - Y represents a direct bond, a $C_1$-$C_2$ alkylene moiety, -$SO_2$- or -O-; and
  - $A_2$ is a phenyl, 5- to 6- membered heteroaryl, 5- to 6- membered heterocyclyl or $C_3$-$C_6$ cycloalkyl group,

the phenyl moiety in the $R^1$ group being unsubstituted or substituted by one or two substituents selected from fluorine, chlorine, bromine, $C_{1-4}$ alkyl, $C_{1-4}$ alkoxy, $C_{1-4}$ alkylthio, $C_{1-4}$ haloalkyl or $C_{1-4}$ haloalkoxy;
the $A_1$ moiety being unsubstituted or substituted by 1 or 2 substituents selected from halogen, cyano, nitro, $C_1$-$C_4$ alkyl, $C_1$-$C_4$ haloalkyl and $C_1$-$C_4$ alkoxy substituents; and
the $A_2$ moiety being unsubstituted or substituted by 1 or 2 substituents which are selected from $C_1$-$C_4$ alkyl, halogen and oxo substituents when $A_2$ is a heterocyclyl or cycloalkyl group and which are selected from $C_1$-$C_4$ alkyl and halogen substituents when $A_2$ is a phenyl or heteroaryl group.

Particularly preferred compounds of the invention are compounds of formula (Va) and pharmaceutically acceptable salts thereof

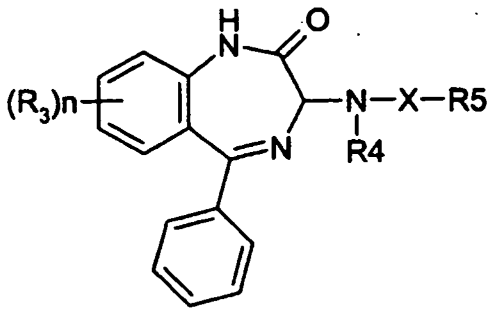

(Va)

wherein:

- X is -CO- or -CO-NH-; and
- $R^5$ is a 5- to 6- membered heteroaryl group, for example a furanyl group,

which is substituted by -$CH_2$-OH or -($C_1$-$C_4$ alkyl)-N($CH_3$)-($C_1$-$C_4$ alkyl)-$SO_2$-($C_1$-$C_4$ alkyl) or $R_5$ represents -$A_1$-Y-$A_2$, wherein:

- $A_1$ is a phenyl, pyridyl, furanyl, thiazolyl, oxazolyl, isoxazolyl, thienyl or 1H-imidazo[4,5-b]pyridin-2-(3H)-one moiety, which is unsubstituted or substituted by 1 or 2 substituents selected from halogen, cyano, $C_1$-$C_2$ alkyl, $C_1$-$C_2$ haloalkyl and $C_1$-$C_2$ alkoxy substituents;
- Y is a direct bond, a $C_1$-$C_2$ alkylene group, -$SO_2$- or -O-; and
- $A_2$ is a piperazinyl, pyridyl, morpholinyl, pyrrolidinyl, piperidinyl, pyrazinyl, cyclopropyl, phenyl or S,S-dioxo-thiomorpholino group, which is unsubstituted or substituted by a $C_1$-$C_2$ alkyl group.

In the compounds of formula (Va), typically n is 0 and $R_4$ is hydrogen. Preferably, in the compounds of formula (Va), $A_1$ is a phenyl or furanyl group which is unsubstituted or substituted by a chlorine atom. Preferably, Y is a direct bond or a methylene group. Preferably, $A_2$ is a morpholino or S,S-dioxo-thiomorpholino group.

Compounds of the formula (V) containing one or more chiral centre may be used in enantiomerically or diasteroisomerically pure form, or in the form of a mixture of isomers. For the avoidance of doubt, the chemical structures depicted herein are intended to embrace all stereoisomers of the compounds shown, including racemic and non-racemic mixtures and pure enantiomers and/or diastereoisomers.

Preferred compounds of formula (V) are optically active isomers. Thus, for example, preferred compounds of formula (V) containing only one chiral centre include an R enantiomer in substantially pure form, an S enantiomer in substantially pure form and enantiomeric mixtures which contain an excess of the R enantiomer or an excess of the S enantiomer. For the avoidance of doubt, the compounds of the formula (V) can, if desired, be used in the form of solvates.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as citric, fumaric, maleic, malic, ascorbic, succinic, tartaric, benzoic, acetic, methanesulphonic, ethanesulphonic, benzenesulphonic or p-toluenesulphonic acid. Pharmaceutical acceptable bases include alkali metal (e.g. sodium or potassium) and alkaline earth metal (e.g. calcium or magnesium) hydroxides and organic bases such as alkyl amines, aralkyl amines or heterocyclic amines.

Particularly preferred compounds of formula (V) include:

6-(4-Methyl-piperazin-1-yl)-N-(2-oxo-5-phenyl-2,3-dihydro-1H-benzo[e][1,4] diazepin-3-yl)-nicotinamide;
3,4,5,6-Tetrahydro-2H-[1,2']bipyridinyl-5'-carboxylic acid (2-oxo-5-phenyl-2,3-dihydro-1H-benzo[e] [1,4]diazepin-3-yl)-amide;
(S)-2-(1,1,Dioxo-1λ6-thiomorpholin-4-yl)-N-(2-oxo-5-phenyl-2,3-dihydro-1H-benzo[e][1,4]diazepin-3-yl-benzamide;
(S)-2-Chloro-4-morpholin-4-yl-N-(2-oxo-5-phenyl-2,3-dihydro-1H-benzo[e][1,4] diazepin-3-yl)-benzamide;
(S)-2-(1,1-Dioxo-1λ6-thiomorpholin-4-yl)-4-fluoro-(2-oxo-5-phenyl-2,3-dihydro-1H-benzo[e][1,4]diazepin-3-yl-benzamide;
(S)-5-Chloro-2-(1,1-dioxo-1λ6-thiomorpholin-4-yl)-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-5-fluoro-N-(2-oxo-5-phenyl-2,3-dihydro-1H-benzo[e][1,4]diazepin-3-yl)-benzamide;
(S)-5-(4-Methyl-piperazin-1-ylmethyl)-furan-2-carboxylic acid (2-oxo-5-phenyl-2,3-dihydro-1H-benzo[e][1,4]diazepin-3-yl)-amide;
(S)-5-Pyrrolidin-1-ylmethyl-furan-2-carboxylic acid (2-oxo-5-phenyl-2,3-dihydro-1H-benzo[e][1,4]diazepin-3-yl)-amide;
(S)-5-Piperidin-1-ylmethyl-furan-2-carboxylic acid (2-oxo-5-phenyl-2,3-dihydro-1H-benzo[e][1,4]diazepin-3-yl)-amide;
(S)-5-Dimethylaminomethyl-furan-2-carboxylic acid (2-oxo-5-phenyl-2,3-dihydro-1H-benzo[e][1,4]diazepin-3-yl)-amide;
(S)-4-Fluoro-N-(2-oxo-5-phenyl-2,3-dihydro-1H-benzo[e][1,4]diazepin-3-yl)-2-piperidin-1-yl-benzamide;
(S)-4-Fluoro-2-morpholino-4-yl-N-(2-oxo-5-phenyl-2,3-dihydro-1H-benzo[e][1,4] diazepin-3-yl)-benzamide;
(S)-4-Cyano-N-(2-oxo-5-phenyl-2,3-dihydro-1H-benzo[e][1,4]diazepin-3-yl)-2-pyrrolidin-1-yl-benzamide;
(S)-4-Cyano-N-(2-oxo-5-phenyl-2,3-dihydro-1H-benzo[e][1,4]diazepin-3-yl)-piperidine-1-yl-benzamide;
(S)-N-(2-Oxo-5-phenyl-2,3-dihydro-1H-benzo[e][1,4]diazepin-3-yl)-2-pyrrolidin-1-yl-4-trifluoromethyl-benzamide;
(S)-N-(2-Oxo-5-phenyl-2,3-dihydro-1H-benzo[e][1,4]diazepin-3-yl)-2-piperidin-1-yl-4-trifluoromethyl-benzamide;
(S)-2-Morpholin-4-yl-N-(2-oxo-5-phenyl-2,3-dihydro-1H-benzo[e][1,4]diazepin-3-yl)-4-trifluoromethyl-benzamide;
(S)-N-(2-Oxo-5-phenyl-2,3-dihydro-1H-benzo[e][1,4]diazepin-3-yl)-2-pyrrolidin-1-yl-5-trifluoromethyl-benzamide;
(S)-2-Morpholin-4-yl-N-(2-oxo-5-phenyl-2,3-dihydro-1H-benzo[e][1,4]diazepin-3-yl)-5-trifluoromethyl-benzamide;
(S)-2-Morpholin-4-yl-N-(2-oxo-5-phenyl-2,3-dihydro-1H-benzo[e][1,4]diazepin-3-yl)-nicotinamide;
(S)-2-(1,1-Dioxo-1λ6-thiomorpholin-4-yl)-N-(2-oxo-5-phenyl-2,3-dihydro-1H-benzo[e][1,4]diazepin-3-yl)-nicotinamide;
(S)-2-(1,1-Dioxo-1λ6-thiomorpholin-4-yl)-2-methyl-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-4-methyl-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-6-methyl-N-(2-oxo-5-phenyl-2,3-dihydro-1H-benzo[e][1,4]diazepin-3-yl)-benzamide;
(S)-2-Chloro-6-(1,1-dioxo-1λ6-thiomorpholin-4-yl)-N-(2-oxo-5-phenyl-2,3-dihydro-1H-benzo[e][1,4]diazepin-3-yl)-benzamide;
(S)-3-Cyclopropyl-2-oxo-2,3-dihydro-imidazo[4,5-b]pyridine-1-carboxylic acid (2-oxo-5-phenyl-2,3-dihydro-1H-benzo[e][1,4]diazepin-3-yl)-amide;
(S)-3-(4-Methyl-piperazine-1-sulfonyl)-N-(2-oxo-5-phenyl-2,3-dihydro-1H-benzo[e][1,4]diazepin-3-yl)-benzamide;
(S)-4-(4-Methyl-piperazin-1-yl)-N-(2-oxo-5-phenyl-2,3-dihydro-1H-benzo[e][1,4] diazepin-3-yl)-benzamide;
(S)-N-(2-Oxo-5-phenyl-2,3-dihydro-1H-benzo[e][1,4]diazepin-3-yl)-3-(piperidine-1-sulfonyl)-benzamide;
(S)-3-(Morpholine-4-sulfonyl)-N-(2-oxo-5-phenyl-2,3-dihydro-1H-benzo[e][1,4] diazepin-3-yl)-benzamide;
(S)-5-Morpholin-4-ylmethyl-furan-2-carboxylic acid (2-oxo-5-phenyl-2,3-dihydro-1H-benzo[e][1,4]diazepin-3-yl)-amide;
(S)-5-Hydroxymethyl-furan-2-carboxylic acid (2-oxo-5-phenyl-2,3-dihydro-1H-benzo[e][1,4]diazepin-3-yl)-amide;
(S)-5-(1,1-Dioxo-1λ6-thiomorpholin-4-ylmethyl)-furan-2-carboxylic acid (2-oxo-5-phenyl-2,3-dihydro-1H-benzo[e] [1,4]diazepin-3-yl)-amide;
(S)-2-Chloro-4-(1,1-dioxo-1λ6-thiomorpholin-4-yl)-N-(2-oxo-5-phenyl-2,3-dihydro-1H-benzo[e][1,4]diazepin-3-yl)-benzamide;
(S)-2-Chloro-5-(1,1-dioxo-1λ6-thiomorpholin-4-yl)-N-(2-oxo-5-phenyl-2,3-dihydro-1H-benzo[e][1,4]diazepin-3-yl)-benzamide;
(S)-5-{[(2-Methanesulfonyl-ethyl)-methyl-amino]-methyl}-furan-2-carboxylic acid (2-oxo-5-phenyl-2,3-dihydro-1H-benzo[e][1,4]diazepin-3-yl-amide;
(S)-2-Pyridin-3-yl-thiazole-4-carboxylic acid (2-oxo-5-phenyl-2,3-dihydro-1H-benzo[e][1,4]diazepin-3-yl)-amide;
(S)-2-Pyridin-4-yl-thiazole-4-carboxylic acid (2-oxo-5-phenyl-2,3-dihydro-1H-benzo[e][1,4]diazepin-3-yl)-amide;
(S)-4-Methyl-2-pyrazin-2-yl-thiazole-5-carboxylic acid (2-oxo-5-phenyl-2,3-dihydro-1H-benzo[e][1,4]diazepin-3-yl)-amide;
(S)-2-Morpholin-4-ylmethyl-furan-3-carboxylic acid (2-oxo-5-phenyl-2,3-dihydro-1H-benzo[e][1,4]diazepin-3-yl)-amide;
(S)-3-Morpholin-4-ylmethyl-N-(2-oxo-5-phenyl-2,3-dihydro-1H-benzo[e][1,4] diazepin-3-yl)-benzamide;
(S)-5-Morpholin-4-ylmethyl-isoxazole-3-carboxylic acid (2-oxo-5-phenyl-2,3-dihydro-1H-benzo[e][1,4]diazepin-3-yl)-amide;
(S)-3-Morpholin-4-ylmethyl-furan-2-carboxylic acid (2-oxo-5-phenyl-2,3-dihydro-1H-benzo[e][1,4]diazepin-3-yl)-amide;
(S)-5-Pyridin-2-yl-thiophene-2-carboxylic acid (2-oxo-5-phenyl-2,3-dihydro-1H-benzo[e][1,4]diazepin-3-yl)-amide;
(S)-2-Methyl-4-(morpholin-4-sulfonyl)-furan-3-carboxylic acid (2-oxo-5-phenyl-2,3-dihydro-1H-benzo[e][1,4]diazepin-3-yl)-amide;
(S)-6-Morpholin-4-yl-N-(2-oxo-5-phenyl-2,3-dihydro-1H-benzo[e][1,4]diazepin-3-yl)-nicotinamide;
(S)-3-Morpholin-4-ylmethyl-thiophene-2-carboxylic acid (2-oxo-5-phenyl-2,3-dihydro-1H-benzo[e][1,4]diazepin-3-yl)-amide;
(S)-5-Morpholin-4-ylmethyl-thiophene-2-carboxylic acid (2-oxo-5-phenyl-2,3-dihydro-1H-benzo[e][1,4]diazepin-3-yl)-amide;
2-Morpholin-4-yl-N-(2-oxo-5-phenyl-2,3-dihydro-1H-benzo[e][1,4]diazepin-3-yl)-benzamide;
(S)-5-Phenyl-oxazole-4 carboxylic acid (2-oxo-5-phenyl-2,3-dihydro-1H-benzo[e][1,4]diazepin-3-yl)-amide;
1-(2-Oxo-5-phenyl-2,3-dihydro-1H-benzo[e][1,4]diazepin-3-yl)-3-(4-phenoxy-phenyl)-urea; an N-oxide of any of the above compounds;

and pharmaceutically acceptable salts thereof.

The compounds of formulae (I), (II), (III) and (IV) are known compounds. They are disclosed, for example, in WO 00/195910, WO 00/004900, WO 03/053344, US-A-4324794 and WO 01/00612, and can be prepared by the processes set out in those documents.

WO 00/195910, WO 00/004900, WO 03/053344, US-A-4324794 and WO 01/00612 are incorporated herein by reference. Any of the compounds disclosed as fusion inhibitors in those documents can be used in the present invention.

Compounds of formula (V) may be prepared by reacting glyoxylic acid ($HCO-CO_2H$), benzotriazole and an appropriate benzyl carbamate at reflux in toluene, under Dean-Stark conditions giving the key protected amino acid of formula (II')

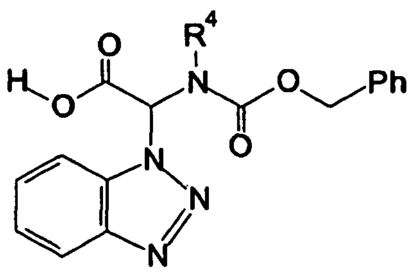

(II')

The thus obtained amino acid of formula (II') can then be reacted with a suitable chlorinating agent, such as oxalyl chloride, followed by reaction with a 2-aminobenzophenone of formula (III')

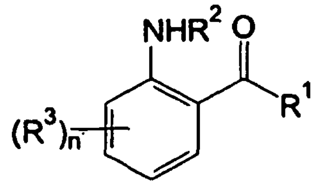

(III')

to give the intermediate amide of formula (IV')

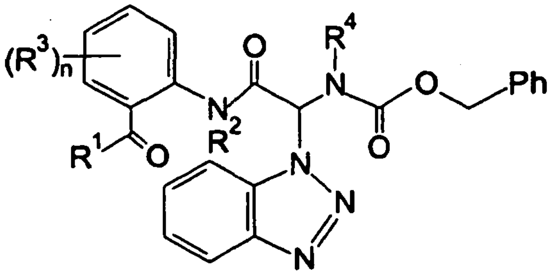

(IV')

which need not be characterized.

The compound of formula (IV') can then be subjected to ammonolysis followed by

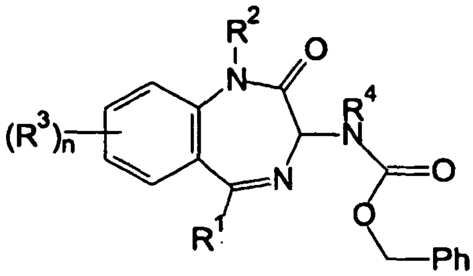

(V')

ring closure in acetic acid containing ammonium acetate to obtain the protected benzodiazepine of formula (V')

The compound of formula (V') can then be deprotected using hydrogen bromide in acetic acid to yield the deprotected amine of formula (VI').

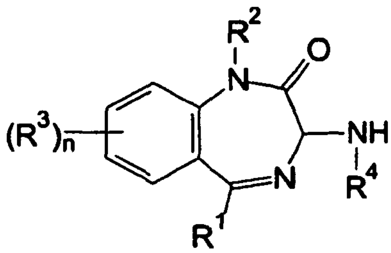

(VI')

Compounds of formula (V), in which X is -CO- or -CO-NR' can be prepared by reacting a compound of formula (VI'), as defined above, with an acid anhydride in a suitable solvent, preferably pyridine at ambient temperature, or with an acid chloride in a suitable solvent in the presence of a base, preferably in THF at ambient temperature with triethylamine present. Alternatively, the compounds can be produced by reaction of a compound of formula (VI') with an acid in a suitable solvent in the presence of a base and a coupling agent, preferably in THF at ambient temperature with triethylamine and *O*-benzotriazol-1-yl-N, N, N', N'-tetramethyluronium hexafluorophosphate (HBTU) present.

If the acid chloride used is an amino carbonyl chloride, the compound of formula (V) is a urea. In the case where R' in the X moiety is hydrogen, such compounds may also be prepared by the reaction of a compound of formula (VI') with an isocyanate. This reaction is preferably carried out in THF at ambient temperature. Alternatively, the isocyanate may be prepared *in situ* from the relevant amine and phosgene, in the presence of a base, usually triethylamine, again in THF. Compounds in which R' is other than hydrogen can, of course, be prepared by reacting a corresponding compound in which R' is hydrogen with an appropriate alkylating agent, for example L-($C_1$-$C_6$ alkyl) wherein L is a leaving group, for example chlorine.

Compounds of formula (V), in which X is -$S(O)_2$- may be prepared by the reaction of a compound of formula (VI') with a suitable sulfonyl chloride. Similarly, compounds of formula (V), in which X is -S(O)- may be prepared by the reaction of a compound of formula (VI') with a suitable sulfinyl chloride

In the preparation of the benzodiazepine skeleton, commercially available aminobenzophenone compounds of formula (III') can be used where possible. Compounds of formula (III') which are not commercially available can be prepared by known methods, for example by reaction of a Weinreb type amide of formula (VII')

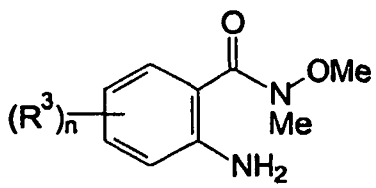

(VII')

with a group R'-Li or a Grignard reagent such as R'-MgBr. Preferably this reaction is carried out in THF at -100°C.

Compounds of formula (VII') are known compounds or can be prepared by analogy with known methods. For example, they can be prepared from the reaction of isatoic anhydrides of formula (VIII')

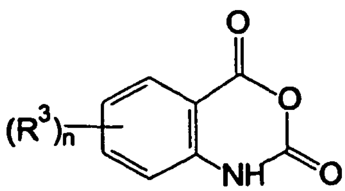

(VIII')

with N,O-dimethyl hydroxylamine under standard reaction conditions.

The starting materials of formula (II'), (III'), (VII'), and (VIII') are known compounds, or may be prepared by analogy with known methods.

Further synthetic manipulation of the thus obtained compounds of formula (V) may be carried out by conventional methods to achieve further compounds of formula (V). The benzodiazepines of formula (V) can be salified by treatment with an appropriate acid or base.

Although the described route to the claimed compounds of formula (V) provides an adequate synthesis for laboratory scale preparations, an alternative route was sought which has potential as a manufacturing route. The same starting material (2-amino-benzophenone) (1) is used in both, however in the alternative route, the benzodiazepine ring system is formed by reaction initially with bromoacetyl bromide (or an equivalent reagent) followed by ring closure with ammonia. These reactions are carried out in a suitable solvent, such as dichloromethane, and at a suitable temperature which may range from -20 to 150°C. In order to protect the NH functionality, at this stage the unsubstituted benzodiazepine is reacted with a base, and an alkylating agent. For instance sodium hydride in DMF followed by addition of 4-methoxybenzyl chloride gives rise to the intermediate (2) shown below. Further reaction of this material with a base (e.g. potassium tert-butoxide ) in a suitable solvent (e.g. THF or DMF) followed by quenching with isoamyl nitrite (or an alternative similar reagent) furnishes the oxime intermediate (3) which may be converted into the racemic primary amine by methods which include the use of hydrogen and a suitable catalyst. This amine then undergoes a Dynamic Kinetic Resolution (DKR) procedure by which the racemic amine in the presence of a suitable optically active acid, and a suitable aldehyde gives rise to precipitation of the salt of the desired (S)-amine (4) in good yield and exceptionally high enantiomeric excess. A suitable acid for this conversion can be e.g. Camphorsulfonic acid, Boc-phenyl alanine or the like, and a suitable aldehyde may be a benzaldehyde such as 3,5-dichloro salicylaldehyde.

The optically amine thus formed may then be transformed into a desired derivative, such as an amide or urea. The amide formations may be carried out using a suitable carboxylic acid and a coupling reagent, or a carbonyl chloride or other suitable reagent, and the ureas prepared using either a suitable isocyanate, or alternatively reaction with phosgene followed by a suitable amine.

These derivatives thus formed may then have the protecting group removed. This may be carried out in the presence of a Lewis Acid, such as aluminium chloride, boron trifluoride, titanium tetrachloride, or the like. These reactions are carried out in a suitable inert solvent, such as dichloromethane. Reaction temperatures may range from -20 to 150°C, but are typically carried out at room temperature or below.

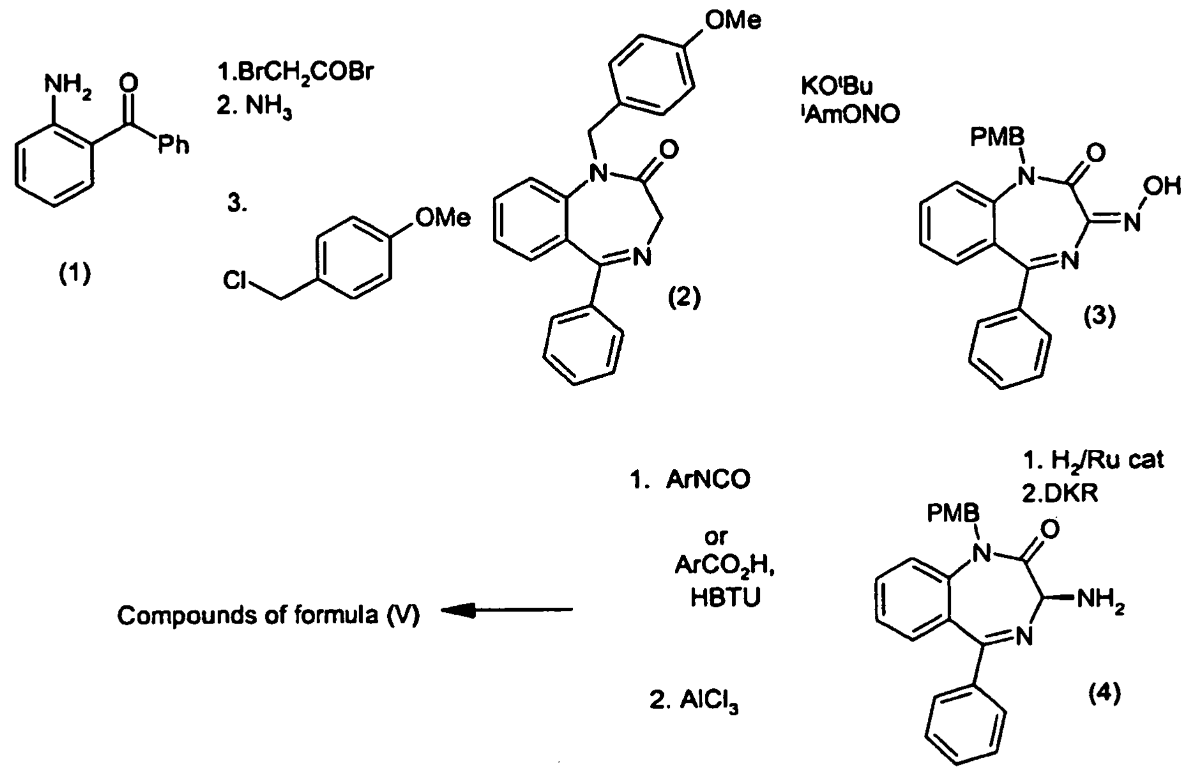


In a particularly preferred embodiment of the invention, component (a) is 1-cyclopropyl-3-[1-(4-hydroxy-butyl)-1H-benzoimidazol-2-ylmethyl]-1,3-dihydro-imidazo[4,5-c]pyridin-2-one, 2-[2-(1,2-dihydro-benzotriazol-1-ylmethyl)-benzoimidazol-1-yl]]ethyl}-diethyl-anune, {2-[2-(3-iodo-2,3-dihydro-indazol-1-ylmethyl)-benzimidazol-1-yl]-ethyl}-dimethyl-amine or a pharmaceutically acceptable salt thereof and component (b) is (S)-2-Chloro-4-morpholin-4-yl-N-(2-oxo-5-phenyl-2,3-dihydro-1H-benzo[e][1,4]diazepin-3-yi)-benzamide or 5-(1,1-dioxo-1λ6-thiomorpholin-4-ylmethyl)-furan-2-carboxytic acid (2-oxo-5-phenyl-2,3-dihydro-1H-benzo[e][1,4]diazepin-3-yl)amide or a pharmaceutically acceptable salt thereof.

The present invention also provides a pharmaceutical composition according to the invention, for use in the treatment of the human or animal body. Also provided is the use of (a) a said RSV fusion protein inhibitor and (b) a said benzodiazepine derivative, in the manufacture of a medicament for use in treating or preventing an RSV infection.

The present invention also provides a method of treating or preventing an RSV infection in a patient, which method comprises the administration to said patient of (a) a said RSV fusion protein inhibitor and (b) a said benzodiazepine derivative.

Typically, the amount of component (a) in the composition of the invention is from 0.025 wt% to 10 wt%, preferably from 0.25 wt% to 5 wt%, more preferably from I wt% to 3.5 wt%, for example about 2.5 wt%, based on the total weight of the composition.

Typically, the amount of component (b) in the composition of the invention is from 0.025 wt% to 10 wt%, preferably from 0.25 wt% to 5 wt%, more preferably from 1 wt% to 3.5 wt%, for example about 2.5 wt%, based on the total weight of the composition.

Typically, the total amount of components (a) and (b) in the composition of the invention is from 0.05 to 20

wt%, preferably from 0.5 to 10 wt%, more preferably from 2 to 7 wt%, for example about 5 wt%, based on the total weight of the composition.

RSV is prevalent among children younger than two years of age, adults suffering from asthma, chronic obstructive pulmonary disorder (COPD) or immunodeficiency and the elderly. It is a particularly serious risk amongst children who suffer from chronic lung disease. Accordingly, the said composition or medicament is typically for use in treating a patient who is a child under two years of age, patients with asthma, COPD or immunodeficiency the elderly or persons in long term care facilities. Typically, said child suffers from chronic lung disease.

Further, anti-RSV prophylaxis is recommended for infants bom at 32 weeks of gestation or earlier, until they reach 6 months of age, the elderly, persons with immunedeficiency and those in long term care facilities. Accordingly, the said composition or medicament is typically for use in preventing RSV infection in an infant less than 6 years of age, who was born after 32 weeks of gestation or less, the elderly, persons with immunosufficiency and those in long term care facilities.

As described above, RSV strains upon exposure to fusion inhibitors known in the art rapidly develop resistance. In order to minimize the risk of development of resistance to fusion inhibitors it is desirable to combine them with another inhibitor of RSV replication with a different mode of action. To our knowledge, the benzodiazepine derivatives disclosed above are the first class of compounds with a novel mode of action. Accordingly, the compositions of the invention are characterized by a very low resistance profile, which makes them particularly suitable for therapeutic and prophylactic applications.

The present invention also covers situations where components (a) and (b) are administered separately. Thus, for example, component (a) can be administered up to 24 hours before component (b). Alternatively, component (b) can be administered up to 24 hours before component (a). More usually, when components (a) and (b) are administered separately, they are administered within 12 hours, preferably within 6 hours, of each other.

The present invention therefore also provides a product comprising (a) a said RSV fusion protein inhibitor and (b) a said benzodiazepine derivative for separate, simultaneous or sequential use in the treatment of the human or animal body. Typically, said product is for separate, simultaneous or sequential use in treating or preventing an RSV infection.

Also provided is the use of a said RSV fusion protein inhibitor in the manufacture of a medicament for use in treating or preventing an RSV infection by co-administration with a said benzodiazepine derivative. The present invention also provides the use of a said benzodiazepine derivative in the manufacture of a medicament for use in treating or preventing an RSV infection, by co-administration with a said RSV fusion protein inhibitor.

When components (a) and (b) are administered separately, they are typically formulated as described above. The amount of active ingredient in each separate formulation will, of course, correspond to the amount of component (a) or (b) given above for the combined formulation. Thus, when components (a) and (b) are administered separately, a first formulation is typically provided which contains from 0.025 wt% to 10 wt%, preferably from 0.25 wt% to 5 wt%, more preferably from 1 wt% to 3.5 wt%, for example about 2.5 wt%, of a said RSV fusion protein inhibitor, based on the total weight of the formulation. Similarly, a second formulation is typically provided which contains from 0.025 wt% to 10 wt%, preferably from 0.25 wt% to 5 wt%, more preferably from 1 wt% to 3.5 wt%, for example around 2.5 wt%, of a said benzodiazepine derivative, based on the total weight of the formulation. The two formulations can be administered separately in any order.

Preferably, the compositions and medicaments of the invention have an activity greater than the combined individual activities of compounds (a) and (b). Thus, components (a) and (b) typically interact synergistically. Preferably, therefore, in the formulations and the medicaments of the invention, component (a) and component (b) are each present in an amount producing a synergistic therapeutic effect in treating or preventing an RSV infection.

The anti-RSV compositions of the invention may be administered in a variety of dosage forms. Thus, they can be administered orally, for example as tablets, troches, lozenges, aqueous or oily suspensions, dispersible powders or granules. The compounds of the invention may also be administered parenterally, whether subcutaneously, intravenously, intramuscularly, intrasternally, transdermally or by infusion techniques. The compounds may also be administered as suppositories.

In a preferred embodiment, administration is by intravenous, intranasal or intrabronchial means. In particular, formulations for treating or preventing RSV can advantageously be administered intranasally. The present invention therefore also provides an inhaler or nebuliser containing a medicament which comprises (i) a composition of the invention comprising component (a) and component (b), as defined above, and (ii) a pharmaceutically acceptable carrier or diluent.

The anti-RSV compositions of the invention are typically formulated for administration with a pharmaceutically acceptable carrier or diluent. For example, solid oral forms may contain, together with the active compound(s), diluents, e.g. lactose, dextrose, saccharose, cellulose, conn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates,

laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

Preferably, the anti-RSV compositions of the invention are solubilised in a earner containing (a) a pharmaceutically acceptable oil selected from esterification or polyether product of glycerides with vegetable oil fatty acids of chain length $C_8$-$C_{10}$ and (b) a pharmaceutically acceptable surfactant selected from oleate and laurate esters of a polyalcohol copolymerized with ethylene oxide. Particularly preferred carriers contain Labrafil as the oil and Tween 20 or Tween 80 as the surfactant.

The anti-RSV compositions of the invention may also be suspended in PEG 400 for oral administration.

A therapeutically effective amount of an anti-RSV composition of the invention is administered to a patient. A typical dose is from about 0.001 to 50 mg, typically 0.5 to 30 mg, preferably 1 to 20 mg active ingredient per kg of body weight, according to the activity of the specific composition, the age, weight and conditions of the subject to be treated, the type and severity of the disease and the frequency and route of administration. Preferably, daily dosage levels are from 5 mg to 2 g active ingredient.

The following Examples illustrate the invention. They do not however, limit the invention in any way. In this regard, it is important to understand that the particular assays used in the Examples section are designed only to provide an indication of antiviral activity. There are many assays available to determine the activity of given compounds against RSV, and a negative result in any one particular assay is therefore not determinative.

## EXAMPLES

Intermediate 1

2-Chloro-4-(1.1-dioxo-1λ6-thiomorpholin-4-yl)-benzoic acid

A mixture of 4-amino-2-chlorobenzoic acid (172mg) and ethenesulfonyl-ethene (0.15ml) in water (3ml) containing sodium carbonate (212mg) was heated to 100C for 18h. The mixture was allowed to cool and was acidified with 2N HCl. The off-white precipitate was collected and dried (263mg)
LC/MS RT= 4.09mins, ES- 288,290

Intermediate 2

2-Chloro-5-(1.1-dioxo-1λ6-thiomorpholin-4-y1)-benzoic acid

A mixture of 5-amino-2-chlorobenzoic acid (172mg) and ethenesulfonyl-ethene (0.15ml) in water (3ml) was heated to 100C for 18h. The mixture was allowed to cool and was extracted with dichloromethane. The dried extracts were evaporated giving a pale brown solid (265mg)
LC/MS RT= 4.13mins, ES- 288,290

Intermediate 3

2-(1,1-Dioxo-1λ6-thiomorpholin-4-yl)-nicotinic acid

This material was prepared as described for Intermediate 1 except that 2-amino-nicotinic acid (138mg) was used. The title compound was isolated as an off-white solid (93mg)

Intermediate 4

2-(1,1-Dioxo-1λ6-thiomorpholin-4-yl)-3-methyl-benzoic acid

This material was prepared as described for Intermediate 2 except that 2-amino-3-methylbenzoic acid (302mg) was used. The title compound was isolated as a pale brown solid (486mg)

Intermediate 5

2-(1,1-Dioxo-1λ6-thiomomholin-4-yl)-4-methyl-benzoic acid

This material was prepared as described for Intermediate 2 except that 2-amino-4-methylbenzoic acid (302mg) was used. The title compound was isolated as a brown solid (430mg)

Intermediate 6

2-(1,1-Dioxo-1λ6-thiomorpholin-4-yl-6-methyl-benzoic acid

This material was prepared as described for Intermediate 2 except that 2-amino-6-methylbenzoic acid (302mg) was used. The title compound was isolated as a brown solid (490mg)

Intermediate 7

3-(4-Methyl-piperazine-1-sulfonyl)-benzoic acid

A solution of 3-chlorosulfonyl-benzoic acid (89mg) 4-dimethylamino-pyridine (catalytic amount) and N-methyl-piperazine (0.045ml) in dichloromethane (10ml) was heated to reflux for 2h. The solvent was then evaporated and the crude material used without purification or characterisation in the next synthetic step.

Intermediate 8

3-Piperidine-1-sulfonyl-benzoic acid

This material was prepared as described for Intermediate 7 except that piperidine was used as the nucleophile. As for Intermediate 7 the material was used crude.

Intermediate 9

3-(Morpholine-4-sulfonyl)-benzoic acid

This material was prepared as described for Intermediate 7 except that morpholine was used as the nucleophile. As for Intermediate 7 the material was used crude.

Intermediate 10

2-Chloro-6-(1,1-dioxo-1λ6-thiomorpholin-4-yl)-benzoic acid

This material was prepared as described for Intermediate 2 except that 2-amino-6-chlorobenzoic acid (343mg) was used. The title compound was isolated as a buff solid (405mg)

Intermediate 11

5-Chloro-2-(1,1-dioxo-1λ6-thiomoroholin-4-yl)-benzoic acid

This material was prepared as described for Intermediate 2 except that 2-amino-5-chlorobenzoic acid (200mg) was used. The title compound was isolated as a white solid (233mg) $^1$t NMR (DMSO, δ) 3.25 (brs, 4H) 3.47 (brs, 4H) 7.31 (d, 1H) 7.54 (dd, 1H) 7.71 (d, 1H) LC/MS RT = 4.66 min Found $ES^+$ = 290,292

Intermediate 12

2-(1.1-Dioxo-1λ6-thiomorpholin-4-yl)-5-fluoro-benzoic acid

This material was prepared as described for Intermediate 2 except that 2-amino-5-fluorobenzoic acid (200mg) was used. The title compound was isolated as a white solid (310mg) $^1$H NMR (DMSO, δ) 3.28 (m, 4H) 3.42 (m, 4H) 7.33-7.56 (m, 3H)
LC/MS RT = 4.28 min Found $ES^-$ = 272

Intermediate 13

4-Fluoro-2-thiomorpholin-4-yl-benzoic acid

A mixture of 2,4-difluoro-benzoic acid (0.5g), thiomorpholine (0.33ml) and triethylamine (0.88ml) in acetonitrile (2ml) was heated to 200C in a microwave reactor for 20mins. The residue was partitioned between water and dichloromethane. The dried organic layer was evaporated and then purified on a silica gel SPE cartridge. Elution with dichloromethane followed by a gradient of dichloromethane:ethanol:0.880 ammonia; 800:8:1 to 200:8:1 gave the title material as a white solid (292mg)
$^1$H NMR (DMSO, δ) 2.81 (m, 4H) 3.27 (m, 4H) 7.11 (m, 1H) 7.40 (dd, 1H) 7.95 (m, 1H)

Intermediate 14

2-(1,1-Dioxo-4-oxy-1λ6-thiomorpholin-4-yl)-4-fluoro-benzoic acid

Intermediate 11 (262mg) and potassium peroxymonosulfate (1.34g) in methanol (5ml) and water (2.5ml) was stirred at room temperature for 6h. The precipitate formed was collected by filtration then dissolved in aqueous sodium bicarbonate. Acidification to pH3 with 1M HC1 led to the formation of a white precipitate which was collected and dried (194mg)
$^1$H NMR (DMSO, δ) 3.2-3.48 (brm, 4H) 3.59 (t, 2H) 3.89 (t, 2H) 6.96 (m, 1H) 7.30 (dd, 1H) 7.85 (m, 1H)

Intermediate 15

6-Chloro-N-(2-oxo-5-phenyl-2,3-dihvdro-1H-benzo[e][1,4]diazepin-3-yl)-nicotinarnide

A mixture of racemic 3-amino-5-phenyl-1,3-dihydro-benzo[e][1,4]diazepin-2-one (1g), O-benzotriazol-1-yl-N, N,N',N'-tetramethyluronium hexafluorophosphate (1.51g), triethylamine (0.83ml) and 6-chloro-nicotinic acid (0.63g) in dry DMF (20ml) was stirred at room temperature for 1.5h. Water (200ml) was then added and the mixture stirred vigorously for 10mins. The colourless precipitate was collected by filtration and dried (1.1g)
$^1$H NMR (DMSO, δ) 5.50 (d, 1H) 7.28-7.71 (m, 10H) 8.42 (dd, 1H) 9.01 (d, 1H) 9.99 (d, 1H) 10.95 (s, 1H)
LC/MS RT= 4.96mins, ES+ 391,393

Intermediate 16

Thiomorpholine-1,1-dioxide

9.98 g of thiomorpholine and 14.8 g of triflic anhydride were stirred together in DCM at room temperature for 2 hours. The reaction was then partitioned between 1 M $K_2CO_{3(aq)}$ and DCM. The organic layer was separated and dried by passing through a hydrophobic frit, then concentrated *in vacuo.* 13.82 g of the resultant oil was stirred with 85.2 g of oxone in 50 mL of methanol and 50 mL of water for 18 h at room temperature. The reaction was then filtered and washed with methanol and the filtrate concentrated. This was then partitioned between water and EtOAc and the aqueous layer washed 3 times with EtOAc. The combined organic extracts were then dried ($MgSO_4$) and concentrated to produce a white solid. This was then stirred at room temperature with 40 g of $K_2CO_3$ in 80 mL of methanol for 18 h. The methanol was then removed *in vacuo* and the remains partitioned between DCM and sat. $K_2CO_{3(aq)}$. The combined organic extracts were passed through a hydrophobic frit and concentrated *in vacuo* to produce the title compound, 3.51 g.
$^1$H NMR ($CDCl_3$, δ) 1.54 (s, 1H), 2.93-2.97 (m, 4H), 3.24-3.28 (m, 4H).

Intermediate 17

5-{[(2-Methanesulfonyl-ethyl)-methyl-amino]-methyl}-furan-2-carboxylic acid ethyl ester

0.5 g of 5-chloromethyl-furan-2-carboxylic acid ethyl ester and 20 ml of 2 M methylamine solution in THF were stirred at room temperature for 5 days under nitrogen. The solution was then concentrated and purified by SPE. The resultant oil was heated at 200 °C in a microwave with 0.2mL of methanesulfonyl-ethene in 3 mL of acetonitrile for 1h. The solution was concentrated and purified by chromatography to produce the title compound as a colourless oil.
LC/MS RT = 3.55 min, Found $ES^+$ = 290
$^1H$ NMR ($CDCl_3$, δ) 1.29 (t, 3H), 2.25 (s, 3H), 2.92-2.88 (m, 2H), 2.99 (s, 3H), 3.06-2.99 (t, 2H), 3.6 (s, 2H), 4.26 (q, 2H), 6.28 (d, 1H), 7.04 (d, 1H).

Intermediate 18

5-Dimethylaminomethyl- furan-2-carboxylic acid

0.16ml of a 2 M solution of dimethylamine was added to a stirred suspension of 19.2 mg of sodium hydride in 2 mL of DMF under a nitrogen atmosphere at room temperature for 30 min. Then a solution of 5-chloromethyl-furan-2-carboxylic acid ethyl ester in 2 mL of DMF was added dropwise over a period of 30 min. The reaction was then allowed to stir for 2 days. The solvent was then removed *in vacuo* and 5 mL of EtOH and 0.35ml of 2 M NaOH added and stirred at 80°C for 40 min. Upon return the reaction was acidified below pH 5.0 and the solvent removed *in vacuo* to produce the title compound to be hydrolysed and then used crude in the next stage

Intermediates 19-23 were prepared in an analogous manner and were used without characterisation in the next synthetic step

Intermediate 19

5-Morpholin-4-ylmethyl-furan-2-carboxylic acid

Intermediate 20

5-(1,1-Dioxo-1λ6-thiomolpholin-4-ylmethyl)-furan-2-carbosylic acid

Intermediate 21

5-(4-Methyl-piperazin-1-ylmethyl)-furan-2-carboxylic acid

Intermediate 22

5-(Piperidin-1-ylmethyl)-furan-2-carboxylic acid

Intermediate 23

5-(Pyrrolidin-1-ylmethyl)-furan-2-carboxylic acid

Intermediate 24

3-Cyclopropyl-1,3-dihydro[4,5-b]pyridin-2-one

A mixture of 2-chloro-3-nitro-pyridine (2g), cyclopropylamine (1.13ml) and potassium carbonate (3.48g) in acetonitrile (30ml) was stirred at room temperature for 18h. The mixture was then partitioned between water and ethyl acetate. The dried extracts were evaporated giving a bright yellow solid (2.1 g)
This material was then hydrogenated at atmospheric pressure in ethanol (150ml) over palladium on carbon catalyst (10%, 100mg). When hydrogen uptake had ceased the mixture was filtered through celite and evaporated giving a dark gum (1.7g)
This material was then dissolved in dry THF (40ml) and was treated with carbonyl diimidazole (2.2g) at reflux for 2.5h. The mixture was then partitioned between water and ethyl acetate. The dried organic extract was evaporated leaving a dark gum, which was crystallised from ethyl acetate/petrol giving a colourless solid (1.2g)

$^1H$ NMR (DMSO, δ) 0.97-1.04 (m, 4H) 2.92 (m, 1H) 6.97 (dd, 1H) 7.22 (dd,1H) 7.92 (dd, 1H) 10.95 (brs, 1H)

Intermediate 25

2-Morpholin-4-ylmethyl-furan-3-carboxylic acid methyl ester

A mixture of 2-chloromethyl-furan-3-carboxylic acid methyl ester (100mg) and morpholine (0.08ml) in acetonitrile (4ml) was stirred at room temperature for 2h. The mixture was then partitioned between dichloromethane and aqueous sodium bicarbonate solution. The dried organic layer was evaporated giving a yellow oil (75mg)
$^1H$ NMR ($CDCl_3$, δ) 2.57 (m, 4H) 3.74 (m, 4H) 3.86 (s, 3H) 3.97 (s, 2H) 6.70 (d, 1H) 7.38 (d, 1H)

Intermediate 26

3-Morpholin-4-ylmethyl-benzoic acid methyl ester

This material was prepared as for Intermediate 25. The product was a colourless oil (210mg)
$^1H$ NMR ($CDCl_3$, δ) 2.43 (m, 4H) 3.53 (s, 2H) 3.70 (m, 4H) 3.91 (s, 3H) 7.39 (t, 1 H) 7.42 (dd,1H) 7.93 (dt, 1H) 7.99 (brs, 1H)

Intermediate 27

5-Morpholin-4-ylmethyl-isoxazole-3-carboxylic acid methyl ester

5-Methyl-isoxazole-3-carboxylic acid methyl ester (200mg), N-bromosuccinimide (252mg) and bezoyl peroxide (30mg) in dry chloroform (4ml) was stirred and heated to 85C for 5h. The solution was cooled to room temperature and was treated with morpholine (0.27ml). Stirring was continued for 20h and the mixture was then partitioned between water and dichloromethane. The dried organic extract was evaporated and the residue purified on a silica gel SPE cartridge. Elution with dichloromethane followed by dichloromethane:ethano1:0.880 ammonia; 200:8:1 gave a colourless oil (50mg)
$^1H$ NMR ($CDCl_3$, δ) 2.46 (m, 4H) 3.64 (m, 4H) 3.67 (s, 2H) 3.90 (s, 3H) 6.55 (s, 1H)
Intermediates 28-30 were prepared in an analogous method to Intermediate 25

Intermediate 28

3-Morpholin-4-ylmethyl-furan-2-carboxylic acid methyl ester

This compound was isolated as a yellow oil (189mg)
$^1H$ NMR ($CDCl_3$, δ) 2.45 (m, 4H) 3.65 (m, 4H) 3.71 (s, 2H) 3.85 (s, 3H) 6.56 (d, 1H) 7.45 (d, 1H)

Intermediate 29

3-Morpholin-4-ylmethyl-thiophene-2-carboxylic acid methyl ester

This compound was isolated as yellow oil (197mg).
$^1H$ NMR ($CDCl_3$, δ) 2.50 (m, 4H) 3.69 (s, 2H) 3.72 (m, 4H) 3.86 (s, 3H) 6.90 (d, 1H) 7.64 (d, 1H)

Intermediate 30

5-Morpholin-4-ylmethyl-thiophene-2-carboxylic acid methyl ester

This compound was isolated as a yellow oil (214mg).
$^1H$ NMR ($CDCl_3$, δ) 2.44 (m, 4H) 3.64 (m, 4H) 3.79 (s, 3H) 3.84 (s, 2H) 7.15 (d, 1H) 7.36 (d, 1H)
Intermediates 25-30 were hydrolysed to the corresponding carboxylic acids before use in the final coupling step of the synthetic sequence

Intermediate 31

4-Fluoro-2-morpholin-4-yl-benzoic acid

2,4-Difluoro-benzoic acid (50mg) and morpholine (0.03ml) in acetonitrile (0.5ml) were heated in a microwave

at 200C for 15mins. The solvent was evaporated leaving a dark gum which was used without purification in the next synthetic step.

Intermediate 32

4-Fluoro-2-piperidin-1-yl-benzoic acid

This was prepared in an analogous procedure to Intermediate 31.

Intermediates 33-5 were prepared in an analogous procedure to Intermediate 31 except that 2-fluoro-4-trifluoromethyl-benzoic acid was used.

Intermediate 33

2-Pyrrolidin-1-yl-4-trifluoromethyl-benzoic acid

Intermediate 34

2.Piperidin-1-yl-4-tfifluoromethyl-benzoic acid

Intermediate 35

2-Morpholin-4-yl-4-trifluoromethyl-benzoic acid

Intermediates 36 and 37 were prepared in an analogous procedure to Intermediate 31 except that 2-fluoro-5-trifluoromethyl-benzoic acid was used.

Intermediate 36

2-Pyrrolidin-1-yl-5-trifluoromethyl-benzoic acid

Intermediate 37

2-Morpholin-4-yl-5-trifluoromethyl-benzoic acid

Intermediates 38 and 39 were prepared in an analogous procedure to Intermediate 31 except that 4-cyano-2-fluoro-benzoic acid was used.

Intermediate 38

4-Cyano-2-pyrrolidin-1-yl-benzoic acid

Intermediate 39

4-Cyano-2-piperidin-1-yl-benzoic acid

Example 1.

6-(4-Methyl-piperazin-1-yl)-N-(2-oxo-5-phenyl-2,3-dihydro-1H-benzo[e][1,4]diazepin-3-yl)-nicotinamide

Intermediate 15 (50mg) and N-methylpiperazine (0.022ml) in acetonitrile (1ml) containing triethylamine (0.027ml) was heated in a microwave at 200°C for 1 Omins.The mixture was then partitioned between water and dichloromethane. The dried organic layer was evaporated and the residue purified on a silica gel SPE cartridge. Gradient elution with 5-10% methanol in dichloromethane gave a colourless solid (10mg)
1H NMR (DMSO, d) 2.28 (s, 3H) 2.45 (m, 4H) 3.68 (m, 4H) 5.56 (d, 1H) 6.93 (d, 1H) 7.32-7.72 (m, 10H) 8.20 (dd, 1 H) 8.82 (d, 1H) 9.42 (d, 1H) 10.94 (s, 1 H)
RT= 3.94mins, ES+ 455

Example 2

3,4,5,6-Tetrahydro-2H-[1,2']bipyridinyl-5'-carboxylic acid (2-oxo-5-phenyl-2.3-dihydro-1H-benzo[e][1,4]diazepin-3-yl)-amide

This material was prepared as for Example 1 except that piperidine was used as the nucleophile. The product was a colourless solid (15mg)
1H NMR (DMSO, d) 1.54-1.63 (brm, 6H) 3.65 (m, 4H) 5.48 (d, 1H) 6.86 (d, 1 H) 7.25-7.65 (m, 10H) 8.11 (dd, 1H) 8.75 (d, 1H) 9.32 (d, 1H)
RT= 4.54 mins, ES+ 440

Example 3

(S)-2-(1,1-Dioxo-1λ6-thiomorpholin-4-yl)-N-(2-oxo-5-phenyl-2,3-dihydro-1H-benzo[e][1,4]diazepin-3-yl-benzamide

(S)-3-Amino-5-phenyl-1,3-dihydm-benzo[e][1,4]diazepin-2-one (100mg), O-benzotriazol-1-yl-N,N,N',N'-tetramethyluronium hexafluorophosphate (150mg), 2-(1,1-dioxo-1λ6-thiomorpholin-4-yl)-benzoic acid (102mg) and triethylamine (0.083ml) in dry DMF (1ml) was stirred at room temperature for 1 h. Water (10ml) was then added and stirring continued for 10mins. The colourless precipitate was collected by filtration and then partitioned between dichloromethane and water. The dried organic phase was evaporated and the residue purified on a silica gel SPE cartridge. Elution with ethyl acetate: petrol 1:1 gave the title compound as a colourless solid (140mg)
$^1$H NMR (DMSO, δ) 3.49 (brs, 8H) 5.48 (d, 1H) 7.31-7.95 (m, 13H) 10.86 (d, 1H) 11.18 (s, 1H)

Example 4

(S)-2-Chloro-4-morpholin-4-yl-N-(2-oxo-5-phenyl-2,3-dihydro-1H-benzo[e][1,4]diazepin-3-yl)-benzamide

This material was prepared as for Example 3 except that 2-chloro-4-morpholin-4-yl-benzoic acid (86mg) was used. The title compound was a colourless solid (112mag).
$^1$H NMR (DMSO, δ) 3.21 (m, 4H) 3.70 (t, 4H) 5.36 (d, 1H) 6.90-6.97 (m, 2H) 7.21-7.66 (m, 10H) 9.21 (d, 1H) 10.86 (s, 1H)

Example 5

(S)-2-(1,1-Dioxo-4-oxy-1λ6-thiomorpholin-4-yl)-4-fluoro-(2-oxo-5-phenyl-2,3-dihydro-1H-benzo[e][1,4]diazepin-3-yl-benzamide

This material was prepared as for Example 3 except that 2-(1,1-dioxo-4-oxy-1λ6-thiomorpholin-4-yl)-benzoic acid (Intermediate 14, 30mg) was used. The title compound was a colourless solid (29mg).
1H NMR (DMSO, d) 3.32-3.98 (m, 8H) 5.34 (d, 1H) 6.99 (dt, 1H) 7.16-7.65 (m, 11H) 9.51 (d,1H) 10.98 (s, 1H)
RT= 5.09mins, ES+ 523

Example 6

(S)-5-Chloro-2-(1,1-dioxo-1λ6-thiomorpholin-4-yl)-N-(2-oxo-5-phenyl-2,3-dihydro-1H-benzo[e][1,4]diazepin-3-yl)-benzamide

This material was prepared as for Example 3 except that 5-Chloro-2-(1,1-dioxo-1λ6-thiomorpholin-4-yl)-benzoic acid (Intermediate 11, 58mg) was used. The title compound was a colourless solid (70mg).
1H NMR (DMSO, d) 3.54 (s, 8H) 5.53 (d, 1H) 7.37-7.75 (m, 11H) 7.90 (d, 1H) 10.84 (d, 1H) 11.24 (s,1H)
RT= 5.38mins, ES+ 523,525

Example 7

(S)-2-(1,1-Dioxo-1λ6-thiomorpholin-4-yl)-5-fluoro-N-(2-oxo-5-phenyl-2,3-dihydro-1H-benzo[e][1,4]diazepin-3-yl)-benzamide

This material was prepared as for Example 3 except that 5-Fluoro-2-(1,1-dioxo-1λ6-thiomorpholin-4-yl)-benzoic acid (Intermediate 12, 54mg) was used. The title compound was a colourless solid (70mg).

1H NMR (DMSO, d) 3.49 (m,8H) 5.47 (d,1H) 7.34-7.69 (m, 12H) 11.12 (d,1H) 11.20 (s, 1H)
RT= 5.19mins, ES+ 507

Example 8

(S)-5-(4-Methyl-piperazin-1-ylmethyl)-furan-2-carboxylic acid (2-oxo-5-phenyl-2,3-dihydro-1H-benzo[e][1,4]diazepin-3-yl)-amide

This material was prepared as for Example 3 except that 5-(4-Methyl-piperazin-1-ylmethyl)-furan-2-carboxylic acid (Intermediate 21) was used. The title compound was a colourless solid (15mg).
1H NMR (CDCl3, d) 2.23 (s, 3H), 2.43-2.51 (m, 8H), 3.56 (s, 2H), 5.65 (d, 1H), 6.29 (d, 1H), 7.05-7.51 (m, 11H), 7.92 (d, 1H).
RT=4.10mins,ES+ 458

Example 9

(S)-5-Pyrrolidin-1-ylmethyl-furan-2-carboxylic acid (2-oxo-5-phenyl-2,3-dihydro-1H-benzo[e][1,4]diazepin-3-yl)-amide

This material was prepared as for Example 3 except that 5-(pyrrolidin-1-ylmethyl)-furan-2-carboxylic acid (Intermediate 23) was used. The title compound was a colourless solid (52mg).
1H NMR (CDCl3, d) 1.76-1.77 (m, 4H), 2.60-2.62 (m, 4H), 3.71 (s, 2H), 5.64 (d, 1H), 6.31 (d, 1H), 7.05-7.50 (m, 10H), 7.98 (d, 1H), 8.04 (s, 1H).
RT = 4.09 mins, ES+ 403

Example 10

(S)-5-Piperidin-1-ylmethyl-furan-2-carboxylic acid (2-oxo-5-phenyl-2,3-dihydro-1H-benzo[e][1,4]diazepin-3-yl)-amide

This material was prepared as for Example 3 except that 5-(piperidin-1-ylmethyl)-furan-2-carboxylic acid (Intermediate 22) was used. The title compound was a colourless solid (21 mg).
1H NMR (CDCl3, d) 1.36-1.45 (m, 2H), 1.53-1.60 (m, 4H), 2.45-2.55 (m, 4H), 3.62 (s, 2H), 5.65 (d, 1H), 6.34 (d, 1H), 7.06-5.52 (m, 10H), 7.81-7.89 (m, 1H), 7.96 (d, 1H).
RT = 4.16 mins, ES+ 443

Example 11

(S)-5-Dimethylaminomethyl-furan-2-carboxylic acid (2-oxo-5-phenyl-2,3-dihydro-1H-benzo[e][1,4]diazepin-3-yl)-amide

This material was prepared as for Example 3 except that 5-dimethylaminomethyl-furan-2-carboxylic acid (Intermediate 18) was used. The title compound was a colourless solid (5mg).
1H NMR (DMSO, d) 2.35 (s, 6H), 3.69 (s, 2H), 5.56 (d, 1H), 6.65 (d, 1H), 7.48-7.85 (m, 10H), 9.1 (d, 1H), 11.13 (s, 1H).
RT = 4.09 mins, ES+ 403

Example 12

(S)-4-Fluoro-N-(2-oxo-5-phenyl-2,3-dihydro-1H-benzo[e][1,4]diazepin-3-yl)-2-piperidin-1-yl-benzamide

This material was prepared as for Example 3 except that 4-fluoro-2-pipendin-l-yl-benzoic acid (Intermediate 32) was used. The title compound was a colourless solid (58mg).
1H NMR (DMSO, d) 1.62-1.67 (m, 2H) 1.91-1.99 (m, 4H) 3.08-3.16 (m, 4H) 5.56 (d, 1H) 7.15-7.79 (m, 11H) 8.10-8.13 (m, 1H) 11.08 (s and d, 2H)
RT= 6.02mins, ES+ 457

Example 13

(S)-4-Fluoro-2-morpholino-4-yl-N-(2-oxo-5-phenyl-2,3-dihydro-1H-benzo[e][1,4]diazepin-3-yl)-benzamide

This material was prepared as for Example 3 except that 4-fluoro-2-morpholin-4-yl-benzoic acid (Intermediate

31) was used. The title compound was a colourless solid (19mg).
1H NMR (DMSO, d) 2.94-3.00 (m, 4H) 3.71-3.82 (m, 4H) 5.35 (d, 1H) 6.98-7.85 (m, 12H) 10. 52 (d, 1H) 10.90 (s, 1H)
RT= 5.34mins, ES+ 459

Example 14

(S)-4-Cyano-N-(2-oxo-5-phenyl-2,3-dihydro-1H-benzo[e][1,4]diazepin-3-yl)-2-pyrrolidin-1-yl-benzamide

This material was prepared as for Example 3 except that 4-cyano-2-pyrrolidin-1-yl-benzoic acid (Intermediate 38) was used. The title compound was a colourless solid (13mg).
1H NMR (DMSO, d) 1.87 (brs, 4H) 3.29 (brs, 4H) 5.37(d, 1H) 7.01-7.65 (m, 12H) 9.60 (d, 1H) 10.88 (s, 1H)
RT= 5.45mins, ES+ 450

Example 15

(S)-4-Cyano-N-(2-oxo-5-phenyl-2,3-dihydro-1H-benzo[e][1,4]diazepin-3-yl)-piperidine-1-yl-benzamide

This material was prepared as for Example 3 except that 4-cyano-2-piperidin-1-yl-benzoic acid (Intermediate 39) was used. The title compound was a colourless solid (27mg).
1H NMR (DMSO, d) 1.32-1.36 (m, 2H) 1.58-1.67 (m, 4H) 2.81-2.89 (m, 4H) 5.25 (d, 1H) 7.10-7.83 (m, 12H) 10.70 (d, 1H) 10.81 (s, 1H)
RT= 5.88mins, ES+ 464

Example 16

(S)-N-(2-Oxo-5-phenyl-2,3-dihydro-1H-benzo[e][1,4]diazepin-3-yl)-2 pyrrolidin-1-yl-4-trifluoromethyl-benzamide

This material was prepared as for Example 3 except that 2-pyrrolidin-1-yl-4-trifluoromethylbenzoic acid (Intermediate 33) was used. The title compound was a colourless solid (5mg).
1H NMR (DMSO, d) 1.89-1.92 (brs, 4H) 3.29-3.32 (brs, 4H) 5.40 (d, 1H) 6.88 (s, 1H) 6.94 (d, 1H) 7.24-7.67 (m, 10H) 9.56 (d, 1H) 10.89 (s, 1H)
RT= 5.91mins, ES+ 493

Example 17

(S)-N-(2-Oxo-5-phenyl-2,3-dihydro-1H-benzo[e][1,4]diazepin-3-yl)-2-piperidin-1-yl-4-trifluoromethyl-benzamide

This material was prepared as for Example 3 except that 2-piperidin-1-yl-4-trifluoromethylbenzoic acid (Intermediate 34) was used. The title compound was a colourless solid (14mg).
1H NMR (DMSO, d) 1.53-1.57 (m, 2H) 1.80-1.91 (m, 4H) 3.00-3.14 (m, 4H) 5.46 (d, 1H) 7.30-7.72 (m, 11H) 8.09 (d, 1H) 10.98 (d, 1H) 10.99 (s, 1H)
RT=6.39mins, ES+ 507

Example 18

(S)-2-Morpholin-4-yl-N-(2-oxo-5-phenyl-2,3-dihydro-1H-benzo[e][1,4]diazepin-3-yl)-4-trifluoromethyl-benzamide

This material was prepared as for Example 3 except that 2-morpholin-4-yl-4-trifluoromethylbenzoic acid (Intermediate 35) was used. The title compound was a colourless solid (14mg).
1H NMR (DMSO, d) 3.18-3.24 (m, 4H) 3.90-3.96 (m, 4H) 5.52 (d, 1H) 7.36-8.10 (m, 12H) 10.59 (d, 1H) 11.10 (s, 1H)
RT= 5.72mins, ES+ 509

Example 19

(S)-N-(2-Oxo-5-phenyl-2,3-dihydro-1H-benzo[e][1,4]diazepin-3-yl)-2-pyrrolidin-1-yl-5-trifluoromethyl-benzamide

This material was prepared as for Example 3 except that 2-pyrrolidin-1-yl-5-trifluoromethylbenzoic acid (Intermediate 36) was used. The title compound was a colourless solid (8mg).

1H NMR (DMSO, d) 2.00-2.02 (brs, 4H) 3.40-3.43 (brs, 4H) 5.48 (d, 1H) 6.90 (d, 1H) 7.34-7.74 (m, 11H) 9.71 (d, 1H) 10.98 (s, 1H)
RT= 5.84 mins, ES+ 493

Example 20

(S)-2-Morpholin-4-yl-N-(2-oxo-5-phenyl-2,3-dihydro-1H-benzo[e][1,4]diazepin-3-yl)-5-trifluoromethyl-benzamide

This material was prepared as for Example 3 except that 2-motpholin-4-yl-5-trifluommethyl-benzoic acid (Intermediate 37) was used. The title compound was a colourless solid (19mg).
1 H NMR (DMSO, d) 3.13-3.18 (m, 4H) 3.85-3.90 (m, 4H) 5.46 (d, 1H) 7.30-7.69 (m, 10H) 7.88 (dd, 1H) 8.04 (d, 1H) 10.37 (d, 1H) 11.04 (s, 1H)
RT= 5.72mins, ES+ 509

Example 21

(S)-2-Morpholin-4-yl-N-(2-oxo-5-phenyl-2,3-dihydro-1H-benzo[e][1,4]diazepin-3-yl)-nicotinamide

This material was prepared as for Example 3 except that 2-morpholin-4-yl-nicotinic acid was used. The title compound was a colourless solid (45mg).
1H NMR (DMSO, d) 3.30-3.36 (m, 4H) 3.82-3.85 (m, 4H) 5.45 (d, 1H) 7.14-7.17 (m, 1 H) 7.19-7.71 (m, 9H) 8.07 (dd, 1H) 8.44 (dd, 1H) 10.00 (d, 1H) 11.05 (s, 1H)
RT= 4.86mins, ES+ 442

Example 22

(S)-2-(1,1-Dioxo-1λ6-thiomorpholin-4-yl)-N-(2-oxo-5-phenyl-2,3-dihydro-1H-benzo[e][1,4]diazepin-3-yl)-nicotinamide

This material was prepared as for Example 3 except that 2-(1,1-dioxo-1λ6-thiomorpholin-4-yl)-nicotinic acid (Intermediate 3) was used. The title compound was a colourless solid (10mg).
1H NMR (DMSO, d) 3.25 (t, 2H) 3.40 (t, 2H) 3.75-3.88 (m, 4H) 5.47 (d, 1H) 6.67-6.72 (m, 1H) 7.28-7.67 (m, 8H) 8.24-8.38 (m, 3H) 9.56 (d, 1H) 10.92 (s, 1H)
RT= 4.43mins, ES+ 508

Example 23

(S)-2-(1,1-Dioxo-1λ6-thiomorpholin-4-yl)-3-methyl-N-(2-oxo-5-phenyl-2,3-dihydro-1H-benzo[e][1,4]diazepin-3-yl)-benzamide

This material was prepared as for Example 3 except that 2-(1,1-dioxo-1λ6-thiomorpholin-4-yl)-3-methyl-benzoic acid (Intermediate 4) was used. The title compound was a colourless solid (65mg).
1H NMR (DMSO, d) 2.36 (s, 3H) 3.24 (brs, 4H) 3.49 (brs, 4H) 5.43 (d, 1H) 7.11-7.68 (m, 12H) 9.61 (d, 1H) 10.99 (s, 1H)
RT= 5.04mins, ES+ 503

Example 24

(S)-2-(1,1-Dioxo-1λ6-thiomorphlin-4-yl)-4-methyl-N-(2-oxo-5-phenyl-2,3-dihydro-1H-benzo[e][1,4]diazepin-3-yl)-benzamide

This material was prepared as for Example 3 except that 2-(1,1-dioxo-1λ6-thiomorpholin-4-yl)-4-methyl-benzoic acid (Intermediate 5) was used. The title compound was a colourless solid (72mg).
1H NMR (DMSO, d) 2.39 (s, 3H) 3.44-3.54 (brm, 8H) 5.46 (d, 1H) 7.14 (d, 1H) 7.31-7.69 (m, 10H) 7.86 (d, 1H) 10.94 (d, 1H) 11.17 (s, 1H)
RT= 5.20mins, ES+ 503

Example 25

(S)-2-(1,1-Dioxo-1λ6-thiomorpholin-4-yl)-6-methyl-N-(2-oxo-5-phenyl-2,3-dihydro-1H-benzo[e][1,4]diazepin-3-yl)-benzamide

This material was prepared as for Example 3 except that 2-(1,1-dioxo-1λ6-thiomorpholin-4-yl)-6-methyl-benzoic acid (Intermediate 6) was used. The title compound was a colourless solid (32mg).
1H NMR (DMSO, d) 2.27 (s, 3H) 3.24-3.27 (m, 4H) 3.41-3.43 (m, 4H) 5.56 (d, 1H) 7.03 (d, 1H) 7.11 (d, 1H) 7.25-7.68 (m, 10H) 9.44 (d, 1H) 10.96 (s, 1H)
RT=5.03mins, ES+ 503

Example 26

(S)-2-Chloro-6-(1,1-dioxo-1λ6-thiomorpholin-4-yl)-N-(2-oxo-5-phenyl-2,3-dihydro-1H-benzo[e][1,4]diazepin-3-yl)-benzamide

This material was prepared as for Example 3 except that 2-chloro-6-(1,1-dioxo-1λ6-thiomorpholin-4-yl)-benzoic acid (Intermediate 10) was used. The title compound was a colourless solid (51mg).
1 H NMR (DMSO, d) 3.43-3.47 (m, 4H) 3.59-3.61 (m, 4H) 5.63 (d, 1H) 7.39-7.83 (m, 12H) 9.86 (d, 1H) 11.14 (s, 1H)
RT= 5.07mins, ES+ 523, 525

Example 27

(S)-3-Cyclopropyl-2-oxo-2,3-dihydro-imidazo[4,5-b]pyridine-1-carboxylic acid (2-oxo-5-phenyl-2,3-dihydro-1H-benzo[e][1,4]diazepin-3-yl)-amide

3-Cyclopropyl-1,3-dihydro[4,5-b]pyridin-2-one (Intermediate 24, 35mg), triethylamine (0.028ml) and triphosgene (20mg) were stirred at room temperature in dichloromethane (3ml) for 1h. (S)-3-Amino-5-phenyl-1,3-dihydro-benzo[e][1,4]diazepin-2-one (50mg) was then added, and stirring continued for 18h. The solvent was evaporated and the residue purified on a silica gel SPE cartridge. Elution with dichloromethane:ethano1:0.880 ammonia; 200:8:1 gave a colourless solid (3mg)
1H NMR (DMSO, d) 0.88-1.09 (m, 4H) 2.92 (m ,1H) 5.25 (d, 1H) 7.06-7.71 (m, 10H) 8.08 (m, 2H) 9.94 (d,1H) 11.08(s,1H)
RT= 4.90mins, ES+ 453

Example 28

(S)-3-(4-Methyl-piperazine-1-sulfonyl)-N-(2-oxo-5-phenyl-2,3-dihydro-1H-benzo[e][1,4]diazepin-3-yl)-benzamide

This material was prepared as for Example 3 except that 3-(4-methyl-piperazine-1-sulfonyl)-benzoic acid (Intermediate 7) was used. The title compound was a pale yellow solid (23mg).
1H NMR (CDCl3, d) 2.19 (s, 3H), 2.39-2.43 (m, 4H), 2.95-3.05 (m, 4H), 5.68 (d, 1H), 6.5 (s, 1H), 7.13 (t, 2H), 7.19 (s, 1H), 7.32-7.83 (m, 8H), 8.08-8.11 (m, 2H), 8.28-8.29 (m, 1H).
RT = 4.25 mins, ES+ 518

Example 29

(S)-4-(4-Methyl-piperazin-1-yl)-N-(2-oxo-5-phenyl-2,3-dihydro-1H-benzo[e][1,4]diazepin-3-yl)-benzamide

This material was prepared as for Example 3 except that 4-(4-methyl-piperazine-1-yl)-benzoic acid was used. The title compound was a colourless solid (46mg).
1H NMR (CDCl3, d) 2.30 (s, 3H), 2.50-2.54 (m, 4H), 3.26-3.30 (m, 4H), 5.70 (d, 1H), 6.86 (d, 2H), 7.14 (t, 1H), 7.17-7.50 (m, 8H), 7.74 (d, 1H), 7.80 (d, 2H), 8.25-8.40 (m, 1H).
RT = 4.16 mins, ES+ 454

Example 30

(S)-N-(2-Oxo-5-phenyl-2,3-dihydro-1H-benzo[e][1,4]diazepin-3-yl)-3-(piperidine-1-sulfonyl)-benzamide

This material was prepared as for Example 3 except that 3-piperidine-1-sulfonyl-benzoic acid (Intermediate 8) was used. The title compound was a colourless solid (35mg).
1H NMR (CDCl3, d) 1.35-1.38 (m, 2H), 1.57-1.65 (m, 4H), 2.91-2.99 (m, 4H), 5.70 (d, 1H), 7.14 (t, 2H), 7.19 (s, 2H), 7.31-7.84 (m, 7H), 8.04-8.12 (m, 2H), 8.28-8.29 (m, 1H), 8.41 (s, 1H).
RT = 5.47 mins, ES+ 503

Example 31

(S)-3-(Morpholine-4-sulfonyl)-N-(2-oxo-5-phenyl-2,3-dihydro-1H-benzo[e][1,4]diazepin-3-yl)-benzamide

This material was prepared as for Example 3 except that 3-(morpholine-4-sulfonyl)-benzoic acid (Intermediate 9) was used. The title compound was a colourless solid (29mg).
1H NMR (CDCl3, d) 2.97-3.00 (m, 4H), 3.66-3.70 (m, 4H), 5.68 (d, 1H), 7.10-8.18 (m, 13H), 8.29-8.31 (m, 2H).
RT = 5.06 mins, ES+ 505

Example 32

(S)-5-Morpholin-4-ylmethyl-furan-2-carboxylic acid (2-oxo-5-phenyl-2,3-dihydro-1H-benzo[e][1,4]diazepin-3-yl)-amide

This material was prepared as for Example 3 except that 5-morpholin-4-ylmethyl-furan-2-carboxylic acid (Intermediate 19) was used. The title compound was a colourless solid (35mg).
1H NMR (CDCl3, d) 2.46-2.49 (m, 4H), 3.55 (s, 2H), 3.66-3.70 (m, 4H), 5.65 (d, 1H), 6.30 (d, 1H), 7.06-7.51 (m, 10H), 7.95 (d, 1H), 8.38 (s, 1H).
RT = 4.28 mins, ES+ 445

Example 33

(S)-5-Hydroxymethyl-furan-2-carboxylic acid (2-oxo-5-phenyl-2,3-dihydro-1H-benzo[e][1,4]diazepin-3-yl)-amide

This material was prepared as for Example 3 except that the hydrolysis product of 5-chloromethyl-furan-2-carboxlic acid ethyl ester was used. The title compound was a colourless solid (48mg).
1H NMR (CDCl3, d) 2.78 (s, 1H), 4.55-4.56 (m, 2H), 5.63 (d, 1H), 6.25 (d, 1H), 7.00 (d, 1H), 7.09 (t, 2H), 7.15-7.49 (m, 7H), 8.10 (d, 1H), 8.46 (s, 1H).
RT = 4.54 mins, ES+ 376

Example 34

(S)-5-(1,1-Dioxo-1λ6-thiomorpholin-4-ylmethyl)-furan-2-carboxylic acid (2-oxo-5-nhenyl-2,3-dihydro-1H-benzo[e][1,4] diazepin-3-yl)-amide

This material was prepared as for Example 3 except that 5-(1,1-Dioxo-1λ6-thiomoipholin-4-ylmethyl)-furan-2-carboxylic acid (Intermediate 20) was used. The title compound was a colourless solid (192mg).
1H NMR (CDCl3, d) 3.00-3.10 (m, 8H), 3.68 (s, 2H), 5.65 (d, 1H), 6.32 (d, 1H), 7.06-7.50 (m, 10H), 7.95 (d, 1H), 8.08-8.16 (s, 1H).
RT = 4.65 mins, ES+ 493

Example 35

(S)-2-Chloro-4-(1,1-dioxo-1λ6-thiomorpholin-4-yl)-N-(2-oxo-5-phenyl-2,3-dihydro-1H-benzo[e][1,4]diazepin-3-yl)-benzamide

This material was prepared as for Example 3 except that 2-chloro-4-(1,1-dioxo-1λ6-thiomorpholin-4-yl)-benzoic acid (Intermediate 1) was used. The title compound was a colourless solid (41mg).
1H NMR (DMSO, d) 3.15 (brs, 4H) 3.92 (brs, 4H) 5.41 (d, 1H) 7.10-7.68 (m, 12H) 9.26 (d, 1H) 10.92 (s, 1H)

RT= 4.70mins, ES+ 523, 525

Example 36

(S)-2-Chloro-5-(1,1-dioxo-1λ6-thiomorpholin-4-yl)-N-2-oxo-5-phenyl-2,3-dihydro-1H-benzo[e][1,4]diazepin-3-yl)-benzamide

This material was prepared as for Example 3 except that 2-chloro-5-(1,1-dioxo-1λ6-thiomotpholin-4-yl)-benzoic acid (Intermediate 2) was used. The title compound was a colourless solid (69mg).
1H NMR (DMSO, d) 3.14 (brs, 4H) 3.81 (brs, 4H) 5.37 (d, 1H) 7.08-7.63 (m, 12H) 9.56 (d, 1H) 10.84 (s, 1H)
RT= 4.76mins, ES+ 523,525

Example 37

(S)-5-{[(2-Methanesulfonyl-ethyl)-methyl-amino]-methyl}-furan-2-carboxylic acid (2-oxo-5-phenyl-2,3-dihydro-1H-benzo[e][1,4]diazepin-3-yl-amide

This material was prepared as for Example 3 except that 5-{[(2-methanesulfonyl-ethyl)-methyl-amino]-methyl}-furan-2-carboxylic acid ethyl ester (Intermediate 17) was used. The title compound was a colourless solid (87mg).
1H NMR (DMSO, d) 2.05 (s, 3H), 2.61 (t, 2H), 2.84 (s, 3H), 3.12 (t, 2H), 3.48 (s, 2H), 5.21 (d, 1H), 6.34 (d, 1H), 7.05-7.39 (m, 9H), 7.50 (td, 1H), 8.77 (d, 1H), 10.78 (s, 1H).
RT = 4.78 mins, ES+ 495

Example 38

(S)-2-Pyridin-3-yl-thiazole-4-carboxylic acid (2-oxo-5-phenyl-2,3-dihydro-1H-benzo[e][1,4]diazepin-3-yl)-amide

This material was prepared as for Example 3 except that 2-pyridin-3-yl-thiazole-4-carboxylic acid was used. The title compound was a colourless solid (55mg).
1H NMR (DMSO, d) 5.64 (d, 1H) 7.48-7.86 (m, 10H) 8.66 (dt, 1H) 8.73 (s, 1H) 8.93 (dd,1H) 9.31 (d, 1H) 9.47 (d, 1H) 11.28 (s, 1H)
RT=4.70mins, ES+ 440

Example 39

(S)-2-Pyridin-4-yl-thiazole-4-carboxylic acid 2-oxo-5-phenyl-2,3-dihydro-1H-benzo[e][1,4]diazepin-3-yl)-amide

This material was prepared as for Example 3 except that 2-pyridin-4-yl-thiazole-4-carboxylic acid was used. The title compound was a colourless solid (54mg).
1H NMR (DMSO, d) 5.36 (d, 1H) 7.19-7.58 (m, 9H) 7.96 (dd, 2H) 8.53 (s, 1H) 8.69 (dd, 2H) 9.02 (d, 1H) 11.01 (s, 1H)
RT= 4.69mins, ES+ 440

Example 40

(S)-4-Methyl-2-pyrazin-2-yl-thiazole-5-carboxylic acid (2-oxo-5-phenyl-2,3-dihydro-1H-benzo[e][1,4]diazepin-3-yl)-amide

This material was prepared as for Example 3 except that 4-methyl-2-pyrazin-2-yl-thiazole-5-carboxylic acid was used. The title compound was a colourless solid (67mg).
1H NMR (DMSO, d) 2.56 (s, 3H) 5.25 (d, 1H) 7.10-7.49 (m, 9H) 8.58-8.63 (s+dd, 2H) 9.16 (d, 1H) 9.38 (d, 1H) 10.78 (s, 1H)
RT= 4.82mins, ES+ 455

Example 41

(S)-2-Morpholin-4-ylmethyl-furan-3-carboxylic acid 2-oxo-5-phenyl-2,3-dihydro-1H-benzo[e][1,4]diazepin-3-yl)-amide

This material was prepared as for Example 3 except that 2-morpholin-4-ylmethyl-furan-3-carboxylic acid (Intermediate 25) was used. The title compound was a colourless solid (24mg).

1 H NMR (DMSO, d) 2.58 (brm, 4H) 3.67 (brm, 4H) 3.91 (s, 2H) 5.45 (d, 1H) 6.88 (d,1H) 7.33-7.75 (m, 10H) 10.95 (s, 1H) 11.01 (d, 1H)
RT= 5.04mins, ES+ 445

Example 42

(S)-3-Morpholin-4-ylmethyl-N-(2-oxo-5-phenyl-2,3-dihydro-1H-benzo[e][1,4]diazepin-3-yl)-benzamide

This material was prepared as for Example 3 except that 3-morpholin-4-ylmethyl-benzoic acid (Intermediate 26) was used. The title compound was a colourless solid (24mg).
1H NMR (DMSO, d) 2.39 (brm, 4H) 3.55 (s, 2H) 3.60 (brm, 4H) 5.51 (d, 1H) 7.28-7.71(m, 11H) 7.93 (s, 1H) 7.97 (s, 1H) 9.50 (d, 1H) 10.93 (s, 1H)
RT= 4.86mins, ES+ 455

Example 43

(S)-5-Morpholin-4-ylmethyl-isoxazole-3-carboxylic acid (2-oxo-5-phenyl-2,3-dihydro-1H-benzo[e][1,4]diazepin-3-yl)-amide

This material was prepared as for Example 3 except that 5-morpholin-4-ylmethyl-isoxazole-3-carboxylic acid (Intermediate 27) was used. The title compound was a colourless solid (11mg).
1H NMR (DMSO, d) 2.93 (m, 4H) 3.46 (m, 4H) 3.66 (brs, 2H) 5.26 (d, 1H) 6.77 (s, 1H) 7.13-7.38 (m, 9H) 9.17 (d, 1H) 10.90 (s, 1H)
RT= 4.75mins, ES+ 446

Example 44

(S)-3-Morpholin-4-ylmethyl-furan-2-carboxylic acid 2-oxo-5-phenyl-2,3-dihydro-1H-benzo[e][1,4]diazepin-3-yl-amide

This material was prepared as for Example 3 except that 3-morpholin-4-ylmethyl-furan-2-carboxylic acid (Intermediate 28) was used. The title compound was a colourless solid (20mg).
1H NMR (DMSO, d) 2.52 (brm, 4H) 3.62 (brs, 4H) 3.67 (m, 2H) 5.39 (d, 1H) 6.67 (d, 1H) 7.25-7.71 (m, 9H) 7.84 (d, 1H) 10.93 (s, 1H) 11.34 (d, 1H)
RT= 4.96mins, ES+ 445

Example 45

(S)-5-Pyridin-2-yl-thiophene-2-carboxylic acid (2-oxo-5-phenyl-2,3-dihydro-1H-benzo[e][1,4]diazepin-3-yl)-amide

This material was prepared as for Example 3 except that 5-pyridin-2-yl-thiophene-2-carboxylic acid was used. The title compound was a colourless solid (32mg).
1 H NMR (DMSO, d) 5.58 (d, 1H) 7.37-7.77 (m, 10H) 7.96-7.99 (m, 2H) 8.10 (d, 1H) 8.32 (d, 1H) 8.67 (d, 1H) 9.81 (d, 1H) 11.03 (s, 1H)
RT= 4.91 mins, ES+ 439

Example 46

(S)-2-Methyl-4-(morpholin-4-sulfonyl)-furan-3-carboxylic acid (2-oxo-5-phenyl-2,3-dihydro-1H-benzo[e][1,4]diazepin-3-yl)-amide

This material was prepared as for Example 3 except that 2-methyl-4-(morpholin-4-sulfonyl)-furan-3-carboxylic acid was used. The title compound was a colourless solid (75mg).
1H NMR (DMSO, d) 2.77 (s, 3H) 3.26 (m, 4H) 3.85 (m, 4H) 5.60 (d, 1H) 7.43-7.83 (m, 9H) 8.23 (s, 1H) 9.68 (d, 1H) 11.07 (s, 1H)
RT= 4.90mins, ES+ 509

Example 47

(S)-6-Morpholin-4-yl-N-(2-oxo-5-phenyl-2,3-dihydro-1H-benzo[e][1,4]diazepin-3-yl)-nicotinamide

This material was prepared as for Example 3 except that 6-morpholin-4-nicotinic acid was used. The title compound was a colourless solid (28mg).
1H NMR (DMSO, d) 3.58-3.61 (m, 4H) 3.70-3.73 (m, 4H) 5.51 (d, 1H) 6.89 (d, 1H) 7.24-7.71 (m, 9H) 8.19 (dd, 1H) 8.80 (d, 1H) 9.39 (d, 1H) 10.89 (s, 1H)
RT= 4.59mins, ES+ 442

Example 48

(S)-3-Morpholin-4-ylmethyl-thiophene-2-carboxylic acid (2-oxo-5-phenyl-2,3-dihydro-1H-benzo[e][1,4]diazepin-3-yl)-amide

This material was prepared as for Example 3 except that 3-morpholin-4-ylmethyl-thiophene-2-carboxylic acid (Intermediate 29) was used. The title compound was a colourless solid (34mg).
1H NMR (DMSO, d) 2.43 (m, 4H) 3.59 (m, 4H) 3.70 (s, 2H) 5.45 (d, 1H) 7.05 (d, 1H) 7.24-7.70 (m, 9H) 8.05 (d, 1H) 9.54 (d, 1H) 10.92 (s, 1H)
RT= 5.02mins, ES+ 461

Example 49

(S)-5-Morpholin-4-ylmethyl-thiophene-2-carboxylic acid (2-oxo-5-phenyl-2,3-dihydro-1H-benzo[e][1,4]diazepin-3-yl)-amide

This material was prepared as for Example 3 except that 5-morpholin-4-ylmethyl-thiophene-2-carboxylic acid (Intermediate 30) was used. The title compound was a colourless solid (41mg).
1H NMR (DMSO, d) 2.28 (brm, 4H) 3.38 (brm, 4H) 3.56 (s, 2H) 5.16 (d, 1H) 6.90 (d, 1H) 7.04-7.44 (m, 9H) 7.52 (d, 1H) 10.68 (s, 1H) 11.82 (d, 1H)
RT= 5.33mins, ES+ 461

Example 50

2-Morpholin-4-yl-N-(2-oxo-5-phenyl-2,3-dihydro-1H-benzo[e][1,4]diazepin-3-yl)-benzamide

This material was prepared as for Intermediate 15 except that 2-morpholin-4-yl-benzoic acid (49mg) was used. The product was a colourless solid (33mg)
1H NMR (DMSO, d) 3.01-3.12 (m, 4H) 3.86-3.93 (m, 4H) 5.44 (d, 1H) 7.21-7.71 (m, 12H) 7.93 (dd, 1H) 10.99 (d, 1H) 11.02 (s, 1H)
RT=5.47, ES+441

Example 51

(S)- 5-Phenyl-oxazole-4-carboxylic acid (2-oxo-5-phenyl-2,3-dihydro-1H-benzo[e][1,4]diazepin-3-yl)-benzamide

(S)-3-Amino-5-phenyl-1,3-dihydro-benzo[e][1,4]diazepin-2-one (60mg), triethylamine (0.037ml) and 5-phenyl-oxazole-4-carbonyl chloride (50mg) in THF (3ml) were stirred at room temperature for 2h. The mixture was then partitioned between water and dichloromethane. The dried organic phase was evaporated and the residue purified on a silica gel SPE cartridge. Elution with dichloromethane:ethano1:0.880 ammonia; 400:8:1 gave the title compound as a colourless solid (42mg).
$^1$H NMR (DMSO, $\delta$) 5.40 (d, 1H) 7.27-7.70 (m, 12H) 8.22-8.26 (m, 2H) 8.72 (s, 1H) 8.88 (d, 1H) 11.14 (s, 1H)
RT=5.22, ES+423.49

Example 52

1-(2-Oxo-5-phenyl-2,3-dihydro-1H-benzo[e][1,4]diazepin-3-yl)-3-(4-phenoxy-phenyl)-urea

Racemic 3-Amino-5-phenyl-1,3-dihydro-benzo[e][1,4]diazepin-2-one (30mg) and 1-isocyanato-4-phenoxy-benzene (0.022ml) in dry THF (4ml) was stirred at room temperature for 18h. The mixture was then partitioned between water and dichloromethane. The dried organic layer was evaporated and the residue triturated from dichloromethane/diethyl ether giving the title compound as a white solid (25mg)
1H NMR (DMSO, d) 5.23 (d, 1H) 6.98-7.03 (m ,3H) 7.11 (t, 1H) 7.33-7.58 (m ,13H) 7.71 (dt, 1H) 9.18 (s, 1H) 11.03 (brs, 1H) RT=5.57, ES+463.45

Example 53

3-[1-(3-Methyl-butyl)-1H-benzoimidazol-2-ylmethyl]-2-oxo-2,3-dihydro-benzoimidazol-1-sulfonic acid dimethylamide, 1-Methanesulfonyl-3-[1-(3-methyl-butyl)-1H-benzoimidazol-2-ylmethyl]-1,3-dihydro-benzoimidazol-2-one, 3-[1-(3-Methyl-butyl)-1H-benzoimidazol-2-ylmethyl]-2-oxo-2,3-dihydro-benzoimidazol-1-carboxylic acid benzylamide, 5-{3-[1-(3-Methanesulfonyl-propyl)-1H-benzoimidazol-2-ylmethyl]-2-oxo-2,3-dihydro-benzoimidazol-1-yl}-pentanenitrile, 7-[2-(3-Isopropenyl-2-oxo-2,3-dihydrobenzoimidazol-1-ylmemyl)-benzoimidazol-1-yl]-heptanenitril, 1-Ethyl-3-[1-(4-hydroxy-butyl)-1H-benzoimidazol-2-ylmethyl]-1,3-dihydro-benzoimidazol-2-one, 1-Ethyl-3-[1-(2-hydroxy-2-phenyl-ethyl)-1H-benzoimidazol-2-ylmethyl]-1,3-dihydro-benzoimidazol-2-one, 1-Isopropenyl-3-[1-(3-oxobutyl)-1H-benzoimidazol-2-ylmethyl]-1,3-dihydro-benzoimidazol-2-one, 1-(4-Hydroxybenzyl)-3-[1-(3-methyl-butyl)-1H-benzoimidazol-2-ylmethyl]-1,3-dihydro-benzoimidazol-2-one, 1-Isopropenyl-3-[1-(3-methyl-butyl)-1H-benzoimidazol-2-ylmethyl]-1,3-dihydro-benzoimidazol-2-one, 1-Cyclopropyl-3-[1-(4-hydroxy-butyl)-1H-benzoimidazol-2-ylmethyl]-1,3-dihydro-imidazo[4,5-c]pyridin-2-one and 1-Isopropenyl-3-(1-propyl-1H-benzoimidazol-2-ylmethyl)-1,3-dihydro-imidazo[4,5-c]pyridine-2-one are prepared as described in WO00195910

Example 54

{2-[2-(1,2-Dihydro-benzotriazol-1-ylmethyl)-benzoimidazol-1-yl]]ethyl}-diethyl-amine is prepared as described in WO00004900.

Example 55

{2-[2-(3-Iodo-2,3-dihydro-indazol-1-ylmethyl)-benzimidazol-1-yl]-ethyl}-dimethyl-amine is prepared as described in WO03053344.

Example 56

Bis(5-amidino-2-benzimidazolyl)-methane is prepared as described in US4,324794.

Example 57

2-{2-[1-[1-(2-Amino-ethyl)-piperidin-4-ylamino]-4-methyl-benzoimidazol-1-ylmethyl}-6-methyl-pyridin-3-ol is prepared as described in WO0100612.

Activity Example 1

Determination of RSV fusion inhibitor activity

RSV enters the host cell via attachment to and fusion with the host cell membrane. The effect of an inhibitor on the specific virus-cell fusion event can be qualitatively determined by using a fluorescence de-quenching system.

The design of this assay takes advantage of the fact that RSV binds to cells at 4°C and at 37°C but that fusion may only occur at concentrations above 18°C.

RSV labelled with octadecyl rhodamine dye (R18) is pre-incubated with Hep-2 cells seeded in a 6-well plate for 1 hour at 4°C to allow binding to occur. Unattached virus is removed by washing the cell monolayer. The inhibitor is then added to the virus-cell complexes prior to transferring the plates to 37°C for 1 hour in order to induce fusion.

Virus-cell fusion can be observed directly under a fluorescence microscope. Fluorescence emission is quenched when 2 identical fluorophores are in close proximity. Upon fusion of the labelled virus with the cell membrane, the distance

between fluorophores is increased due to dye spread and there is a decrease in quenching. This is observed as an increase in fluorescence intensity of R18. It therefore follows that inhibition of fusion would lead to a decrease in fluorescence of R18 compared to untreated control. Where the fluorescent yield of R18 in the presence of inhibitor is comparable to the untreated control this would suggest the inhibitor were not exerting its effects on the fusion protein.

Activity Example 2

Determination of RSV replication inhibitor activity

The inner 60 wells of 96 well tissue culture plates are seeded with Hep-2 cells at $4x10^4$ cells/well for compound activity and toxicity studies in 100μl of medium and incubated at 37°C overnight or until nearing confluency.

Cells are infected with 25 μl RSV, e.g. the RSS strain, previously titrated to give 80% cell kill. To each well 25μM of test compound are added. The final DMSO concentration is 0.5%. Some 200 μl of sterile distilled water is added to the outer wells of the plate and incubated at 37°C for 6 days. Some 0.25 μl/ml PMS are added to stock XTT solution, final conc. 25 μM PMS. Then 25 μl warmed XTT/PMS solution is added to each well and incubated for 1 hour at 37°C.

Maximum $OD_{450nm}$ reading (uninfected, untreated control cells) corresponds to 100% inhibition. Minimum $OD_{450nm}$ readings (infected control cells) corresponds to 0% inhibition. Log10 concentration is plotted against $OD_{450nm}$ and $IC_{50}$ values are calculated from either reading 50% value from graph or using regression analysis.

Activity Example 3

Synergistic action between RSV fusion inhibitor and anti-RSV benzodiazepines

ELISA experiments were carried out on the combined effect of potent benzodiazepine RSV replication inhibitor 2-chloro-4-morpholin-4-yl-N-(2-oxo-5-phenyl-2,3-dihydro-1H-benzo[e][1,4]diazepin-3-yl)-benzamide or 5-(1,1-dioxo-1λ16-thiomorpholin-4-ylmethyl)-furan-2-carboxylic acid (2-oxo-5-phenyl-2,3-dihydro-1H-benzo[e][1,4]diazepin-3-yl)-amide (compound A) with one RSV fusion inhibitor selected from 1-cyclopropyl-3-[1-(4-hydroxybutyl)-1H-benzoimidazol-2-ylmethyl]-1,3-dihydro-imidazo[4,5-c]pyridin-2-one (compound B) or 1-isopropenyl-3-(1-propyl-1H-benzoimidazol-2-ylmethyl)-1,3-dihydro-imidazo[4,5-c]pyridine-2-one (compound B)

ELISA PROTOCOL

Mouse monoclonal antibodies to the phosphoprotein (P), nucleocapsid (N) & fusion (F) proteins of RSV and a rabbit anti-mouse- horseradwash peroxidase (HRP) conjugated secondary antibody were used to demonstrate a reduction in RSV antigen via conversion of the o-phenylene diamine dihydrochloride (OPD) substrate to a coloured product. This was quantified by optical density (OD) measurement.

Method

This assay was set up using all 96 wells of flat-bottomed 96-well plates. The outer wells were not subjected to any greater amount of evaporation than the inner wells during the 3 day assay period. (ie. No "edge effect" seen).
Plates were set up one day before addition of virus and compounds. The assay then ran for 3 days with ELISA development taking place on the $4^{th}$ day.

**Day 0**

Set up of Assay Plates

All 96 wells of a microtitre plate were seeded at a density of $5x10^3$ Hep-2 cells/well in 100μl/well of Growth Medium (GM) consisting of Dulbecco's MEM (DMEM) with Glutamax-1, Sodium Pyruvate, 1000 mg/l glucose and pyridoxine (Invitrogen, catalogue number 21885-025) and supplemented with 10%FBS. (See Plate 1).
In tissue culture, the cells adhere to the tissue culture flask and were grown at 37°C, 5% $CO_2$ until 90% confluent.
Monolayers were washed with 20ml sterile PBS to remove serum and treated with 1ml trypsin to detach cells from the flask. Cells were suspended in a small known volume of growth media and counted using a haemocytometer. The cell suspension was made up to the desired concentration in growth medium and added to wells by multichannel pipette. Brief, gentle shaking encouraged the cells to disperse more evenly across the well.

**Plate 1**

| | | | | | | | | | | | |
|---|---|---|---|---|---|---|---|---|---|---|---|
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |
| cells | cells | cells | cells | cells | cells | cells | cells | cells | cells | cells | cells |

Plates were kept undisturbed at 37°C in a 5% $CO_2$ atmosphere for 24hrs during which time the cells settle to form an even cell monolayer.

**Day 1**

Addition of Virus

A frozen vial of RSV (RSS strain provided by Virogen Ltd) stock solution was removed from the -80 freezer or liquid nitrogen store and diluted to a known Multiplicity of Infection (m.o.i) in Growth Medium.
The m.o.i. was calculated by prior titration of the virus stock (by the ELISA assay method) as the virus input required to achieve a window of at least 0.8 OD units between infected and uninfected control wells.

$$\textbf{Multiplicity of Infection} = \frac{\textbf{plaque forming units per well (pfu/well)}}{\textbf{number of cells per well}}$$

50µl of diluted virus was added to infected wells by multichannel pipette; 50µl of Growth Medium was added to uninfected, cell control wells by multichannel pipette.
Sides of plates were marked with stripes to identify plates in the event of lids becoming separated.
Plates were incubated at 37°C for 1hr to allow virus adsorption.

Compound Dilutions

Compound "A" was titrated horizontally across the plate and Compound "B" was titrated vertically down the plate, creating a chequerboard. The 2 compounds were titrated at either ½-log or doubling dilutions either across (horizontally) or down the plate (vertically) in the presence of virus. Each compound dilution was set up in duplicates or triplicates. For triplicates 3 identical plates were set up. Duplicates were set up as dublicate wells on the same plate. The dilution range covered concentrations from just above the compound IC50 to below the compound IC50 and included a 0µM control for each compound.
Compounds were made up in a separate microtitre plate at 8x strength in GM containing 2% DMSO (a final DMSO concentration in the assay of 0.5%). 25µl of the Compound "A" dilution series and 25µl of the Compound "B" dilution series were then transferred to the appropriate wells of the assay plate by multichannel pipette, according to the marked out chequerboard.
25µl of GM (containing 2% DMSO) was added to wells receiving 0µM Compound "A" or 0µM Compound "B". 50µl GM (containing 2% DMSO) was added to wells containing neither compound.
Virus infected, untreated wells served as the virus control (VC); Uninfected, untreated wells serve as the cell control (CC). The difference in absorbance between CC and VC wells constitutes the assay window.
Plates were incubated at 37°C, 5% $CO_2$ for 3 days.

**ELISA Stage**

**Day 4**

Media was tapped out from wells directly into Virkon (1% solution in water) and plates were washed by immersing in a plastic box containing PBS.
50μl/well of 75%/25% vol/vol acetone/methanol fixative was added by multichannel pipette and left for 3mins.
Acetone/methanol was discarded from wells into Virkon and wells were washed with PBS as above.

Some 200μl of blocking solution (2% Marvel in PBS containing 0.05% Tween) was added per well by multichannel pipette. Plates were incubated at 37°C in a shaking incubator for 60mins.

Block solution was discarded down the sink and diluted primary antibody was added directly to wells (ie. no washing required).
RSV mouse monoclonal antibody NCL-RSV3 (Novocastra ) was diluted 1/400 in PBS/2% Marvel/0.05% Tween and 50μl was added per well. Plates were incubated at 37°C in a shaking incubator for 90mins.

Antibody was discarded down the sink and plates were washed 4 times by immersion in PBS/0.05% Tween.
DAKo rabbit anti-mouse HRP conjugate (DAKO catalogue number P0260) was diluted 1/1000 in PBS/2% Marvel/0.05% Tween and 50μl was added per well. Plates were incubated at 37°C in a shaking incubator for 60mins.

Antibody was discarded down the sink and plates were washed 6 times by immersion in PBS/0.05% Tween.

Substrate (SigmaFast OPD) was prepared in advance by dissolving 1 urea tablet in 20mL water. 1 OPD tablet was added to the urea solution just prior to use (NB. OPD was light sensitive) and vortexed to mix. 50μl of substrate was added per well.

The reaction was stopped by addition of 25μl/well of 20% sulphuric acid, once sufficient colour had developed but while cell control background was still low (~5 minutes).

Plates were read on a SpectraMax (Molecular Devices) spectrophotometer at wavelength 490nm and utilize the SOFTmax Pro software package.

The wells were emptied, washed in tap water and the monolayers stained with 50μl/well of 2% crystal violet in 20% methanol/water for at least 1 hour. The wells were then washed and air-dried and the monolayers examined under the microscope for indications of cell toxicity.

Results

SOFTmax data files were exported to Excel. Data handling used Excel templates written in-house for plotting dose response curves graphically and calculating IC50 values from the curves obtained.

All replicate wells were meaned. The assay window was calculated by subtracting the meaned cell control (CC) from the meaned virus control (VC). For each compound, the meaned CC was subtracted from the meaned values for each concentration point. The % of control was then calculated for each concentration point as a percentage of the window. % of control was plotted against compound concentration. A straight line was fitted to the curve and the slope and intercept functions were used to calculate the IC50.

The IC50 for Compound "A" was calculated for each background concentration of Compound "B". Similarly, the IC50 for Compound "B" was calculated for each background concentration of Compound "A".

Example 3a

2-Chloro-4-morpholin-4-yl-N-(2-oxo-5-phenyl-2,3-dihydro-1H-benzo[e][1,4]diazepin-3-yl)-benzamide (Compound A) in combination with 1-cyclopropyl-3-[1-(4-hydroxy-butyl)-1H-benzoimidazol-2-ylmethyl]-1,3-dihydro-imidazo[4,5-c]pyridin-2-one (Compound B)

Compound A has an ELISA IC50 of 1.6μM against the RSV RSS strain.
Compound B has an ELISA IC50 of 0.015μM against the RSV RSS strain.

In combination, at concentrations of Compound A below its IC50 the IC50 of Compound B is reduced from 0.15μM to at least 0.003μM (5 -fold decrease). At concentrations of Compound B below its IC50 the IC50 of Compound A is reduced from 1.6μM to at least 1μM (1.6-fold decrease).

Example 3b

5-(1,1-Dioxo-1λ6-thiomorpholin-4-ylmethyl)-furan-2-carboxylic acid (2-oxo-5-phenyl-2,3-dihydro-1H-benzo[e][1,4]diazepin-3-yl)-amide (Compound A) in combination with 1-cyclopropyl-3-[1-(4-hydroxy-butyl)-1H-benzoimidazol-2-ylmethyl]-1,3-dihydro-imidazo[4,5-c]pyridin-2-one (Compound B)

Compound A has an ELISA IC50 of 3.5μM against the RSV RSS strain.
Compound B has an ELISA IC50 of 0.06μM against the RSV RSS strain.

In combination, at concentrations of Compound A below its IC50, the IC50 of Compound B is reduced from 0.06μM to at least 0.006μM (10-fold decrease). At concentrations of Compound B below its IC50 the IC50 of compound A is reduced from 3.5μM to at least 0.312μM (11.2-fold decrease).

The formula below can be used to identify a synergistic interaction.

*FIC = Fractional Inhibitory concentration*

Compares the activity of a compound in combination (Compound A + Compound B) with the activity of the compound alone (Compound A or Compound B).

$$\mathbf{FIC} = \frac{\text{Lowest IC50 Cpd } A^{\text{COMBINATION}}}{\text{IC50 Cpd } A^{\text{ALONE}}} + \frac{\text{Lowest IC50 Cpd } B^{\text{COMBINATION}}}{\text{IC50 Cpd } B^{\text{ALONE}}}$$

| | | |
|---|---|---|
| where FIC value | < 0.5 | SYNERGY |
| | 0.5-1.0 | ADDITION |
| | 1.0-2.0 | INDIFFERENCE |
| | >2.0 | ANTAGONISM |

FIC for 2-Chloro-4-morpholin-4-yl-N-(2-oxo-5-phenyl-2,3-dihydro-1H-benzo[e][1,4]diazepin-3-yl)-benzamide in combination with 1-cyclopropyl-3-[1-(4-hydroxybutyl)-1H-benzoimidazol-2-ylmethyl]-1,3-dihydro-imidazo[4,5-c]pyridin-2-one: 0.3

FIC for 5-(1,1-Dioxo-1λ6-thiomoipholin-4-ylmethyl)-furan-2-carboxylic acid (2-oxo-5-phenyl-2,3-dihydro-1H-benzo[e][1,4]diazepin-3-yl)-amide (Compound A) in combination with 1-cyclopropyl- 3-[1-(4-hydroxy-butyl)-1H-benzoimidazol-2-ylmethyl]-1,3-dihydro-imidazo[4,5-c]pyridin-2-one: 0.14

## Claims

**1.** A pharmaceutical composition which comprises a pharmaceutically acceptable carrier or diluent and:

(a) an inhibitor of the RSV fusion protein; and
(b) a benzodiazepine derivative capable of inhibiting RSV replication,

wherein component (b) is a compound of formula (V), or a pharmaceutically acceptable salt thereof,

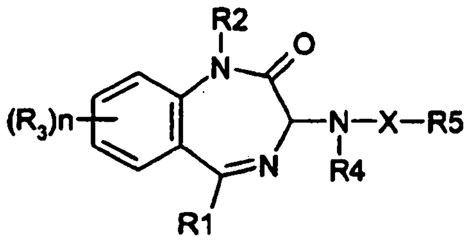

(V)

wherein:

- $R^1$ represents $C_{1-6}$ alkyl, aryl or heteroaryl;
- $R^2$ represents hydrogen or $C_{1-6}$ alkyl;
- each $R^3$ is the same or different and represents halogen, hydroxy, $C_{1-6}$ alkyl, $C_{1-6}$ alkoxy, $C_{1-6}$ alkylthio, $C_{1-6}$ haloalkyl, $C_{1-6}$ haloalkoxy, amino, mono($C_{1-6}$ alkyl)amino, di($C_{1-6}$ alkyl)amino, nitro, cyano, $-CO_2R'$, -CONR'R", -NH-CO-R',
- S(O)R', $-S(O)_2R'$, $-NH-S(O)_2R'$, -S(O)NR'R" or $-S(O)_2NR'R"$, wherein each R' and R" is the same or different and represents hydrogen or $C_{1-6}$ alkyl;
- n is from 0 to 3;
- $R^4$ represents hydrogen or $C_{1-6}$ alkyl;
- X represents -CO-, -CO-NR'-, -S(O)- or $-S(O)_2-$, wherein R' is hydrogen or a $C_1$-$C_6$ alkyl group; and
- $R^5$ represents an aryl, heteroaryl or heterocyclyl group which is substituted by a $C_1$-$C_6$ hydroxyalkyl group or a -($C_1$-$C_4$ alkyl)-$X_1$-($C_1$-$C_4$ alkyl)-$X_2$-($C_1$-$C_4$ alkyl) group, wherein $X_1$ represents -O-, -S- or -NR'-, wherein R' represents H or a $C_1$-$C_4$ alkyl group and $X_2$ represents -CO-, -SO- or $-SO_2-$, or $R_5$ represents $-A_1-Y-A_2$, wherein:

  - $A_1$ is an aryl, heteroaryl, carbocyclyl or heterocyclyl group;
  - Y represents a direct bond or a $C_1$-$C_4$ alkylene, $-SO_2-$, -CO-, -O-, -S- or -NR'-moiety, wherein R' is a $C_1$-$C_6$ alkyl group; and
  - $A_2$ is an aryl, heteroaryl, carbocyclyl or heterocyclyl group.

**2.** A composition according to claim 1 wherein wherein $R^1$ is $C_{1-2}$ alkyl or phenyl.

**3.** A composition according to either claim 1 or claim 2, wherein wherein $R^2$ is hydrogen

**4.** A composition according to any one of claims 1 to 3 wherein $R^3$ is halogen, hydroxy, $C_{1-4}$ alkyl, $C_{1-4}$ alkoxy, $C_{1-4}$ alkylthio, $C_{1-4}$ haloalkyl, $C_{1-4}$ haloalkoxy, amino, mono($C_{1-4}$ alkyl)amino or di($C_{1-4}$ alkyl)amino.

**5.** A composition according to claim 4 wherein $R^3$ is fluorine, chlorine, bromine, $C_{1-2}$ alkyl, $C_{1-2}$ alkoxy, $C_{1-2}$ alkylthio, $C_{1-2}$ haloalkyl, $C_{1-2}$ haloalkoxy, amino, mono($C_{1-2}$ alkyl)amino or di ($C_{1-2}$ alkyl)amino.

**6.** A composition according to any of claims 1-5, wherein $R^4$ is hydrogen or $C_{1-2}$ alkyl.

**7.** A composition according to any one of claims 1-6, wherein X is -CO- or -CO-NR'- wherein R' represents hydrogen or a $C_1$-$C_2$ alkyl group.

**8.** A composition according to any one of claims 1-7, wherein $R^5$ is a 5- or 6- membered heterocyclyl, aryl or heteroaryl ring which is substituted by a $C_1$-$C_6$ hydroxyalkyl group or a -($C_1$-$C_4$ alkyl)-$X_1$-($C_1$-$C_4$ alkyl)-$X_2$-($C_1$-$C_4$ alkyl) group, wherein $X_1$ and $X_2$ are as defined in claim 1.

**9.** A composition according to claim 8, wherein $R^5$ is a 5- or 6- membered heteroaryl group which is substituted by a $-CH_2-OH$ or -($C_1$-$C_4$ alkyl)-NR'-($C_1$-$C_4$ alkyl)-$S(O)_2$-($C_1$-$C_4$ alkyl) substituent, wherein R' is hydrogen or $C_1$-$C_2$ alkyl.

**10.** A composition according to claims 1-9, wherein $A_1$ is an aryl or heteroaryl group.

**11.** A composition according to claim 10, wherein $A_1$ is a phenyl, group, a monocyclic 5- or 6- membered heteroaryl group or a 5- to 6- membered heteroaryl group fused to a monocyclic oxo-substituted 5- to 6- membered heterocyclyl

group.

**12.** A composition according to claims 1-11 wherein $A_1$ is unsubstituted or substituted by 1 or 2 substituents selected from halogen, cyano, nitro, $C_1$-$C_4$ alkyl, $C_1$-$C_4$ haloalkyl and $C_1$-$C_4$ alkoxy substituents.

**13.** A composition according to claims 1-12, wherein Y represents a direct bond, a $C_1$-$C_2$ alkylene group, $-SO_2-$ or -O-.

**14.** A composition according to claims 1-13 wherein $A_2$ is a phenyl, 5- to 6- membered heteroaryl, 5- to 6- membered heterocyclyl or $C_3$-$C_6$ cycloalkyl group.

**15.** A composition according to claims 1-14, wherein when $A_2$ is a heterocyclyl group it is attached to the moiety Y via a N atom.

**16.** A composition according to claims 1-15, wherein $A_2$ is unsubstituted or is substituted by 1 or 2 substituents which are selected from $C_1$-$C_4$ alkyl and halogen substituents when $A_2$ is a heteroaryl or aryl group and which are selected from $C_1$-$C_4$ alkyl, halogen and oxo substituents when $A_2$ is a carbocyclic or heterocyclyl group.

**17.** A composition according to claims 1-16, wherein $A_2$ is a piperazinyl, pyridyl, morpholinyl, pyrrolidinyl, piperidinyl, pyrazinyl, cyclopropyl, phenyl or S,S-dioxo-thiomorpholino group, which is unsubstituted or substituted by a $C_1$-$C_2$ alkyl group.

**18.** A composition according to any one of claims 1-17 wherein the benzodiazepine derivative of formula (V) is a benzodiazepine derivative of formula (Va):

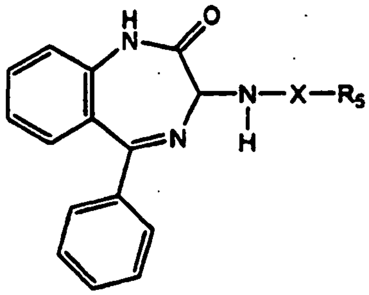

(Va)

wherein:

- X is -CO- or -CO-NH-; and
- $R^5$ is a 5- to 6- membered heteroaryl group, for example a furanyl group, which is substituted by $-CH_2-OH$ or $-(C_1$-$C_4$ alkyl$)-N(CH_3)-(C_1$-$C_4$ alkyl$)-SO_2-(C_1$-$C_4$ alkyl$)$ or $R_5$ represents $-A_1-Y-A_2$, wherein:

  - $A_1$ is a phenyl, pyridyl, furanyl, thiazolyl, oxazolyl, isoxazolyl, thienyl or 1H-imidazo[4,5-b]pyridin-2-(3H)-one moiety, which is unsubstituted or substituted by 1 or 2 substituents selected from halogen, cyano, $C_1$-$C_2$ alkyl, $C_1$-$C_2$ haloalkyl and $C_1$-$C_2$ alkoxy substituents;
  - Y is a direct bond, a $C_1$-$C_2$ alkylene group, $-SO_2-$ or -O-; and
  - $A_2$ is a piperazinyl, pyridyl, morpholinyl, pyrrolidinyl, piperidinyl, pyrazinyl, cyclopropyl, phenyl or S,S-dioxo-thiomorpholino group, which is unsubstituted or substituted by a $C_1$-$C_2$ alkyl group.

**19.** A composition according to claim 1, wherein the benzodiazepine derivative of formula (V) is:

6-(4-Methyl-piperazin-1-yl)-N-(2-oxo-5-phenyl-2,3-dihydro-1H-benzo[e][1,4] diazepin-3-yl)-nicotinamide;
3,4,5,6-Tetrahydro-2H-[1,2']bipyridinyl-5'-carboxylic acid (2-oxo-5-phenyl-2,3-dihydro-1H-benzo[e][1,4]diazepin-3-yl)-amide;
(S)-2-(1,1-Dioxo-1λ6-thiomorpholin-4-yl)-N-(2-oxo-5-phenyl-2,3-dihydro-1H-benzo[e][1,4]diazepin-3-yl-benzamide;
(S)-2-Chloro-4-morpholin-4-yl-N-(2-oxo-5-phenyl-2,3-dihydro-1H-benzo[e][1,4] diazepin-3-yl)-benzamide;
(S)-2-(1,1-Dioxo-1λ6-thiomorpholin-4-yl)-4-fluoro-(2-oxo-5-phenyl-2,3-dihydro-1H-benzo[e][1,4]diazepin-3-yl-benzamide;
(S)-5-Chloro-2-(1,1-dioxo-1λ6-thiomorpholin-4-yl)-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-5-fluoro-N-(2-oxo-5-phenyl-2,3-dihydro-1H-benzo[e][1,4]diazepin-3-yl)-benzamide;
(S)-5-(4-Methyl-piperazin-1-ylmethyl)-furan-2-carboxylic acid (2-oxo-5-phenyl-2,3-dihydro-1H-benzo[e][1,4]diazepin-3-yl)-amide;
(S)-5-Pyrrolidin-1-ylmethyl-furan-2-carboxylic acid (2-oxo-5-phenyl-2,3-dihydro-1H-benzo[e][1,4]diazepin-3-yl)-amide;
(S)-5-Piperidin-1-ylmethyl-furan-2-carboxylic acid (2-cxo-5-phenyl-2,3-dihydro-1H-benzo[e][1,4]diazepin-3-yl)-amide;
(S)-5-Dimethylaminomethyl-furan-2-carboxylic acid (2-oxo-5-phenyl-2,3-dihydro-1H-benzo[e][1,4]diazepin-3-yl)-amide;
(S)-4-Fluoro-N-(2-oxo-5-phenyl-2,3-dihydro-1H-benzo[e][1,4]diazepin-3-yl)-2-piperidin-1-yl-benzamide;
(S)-4-Fluoro-2-morpholino-4-yl-N-(2-oxo-5-phenyl-2,3-dihydro-1H-benzo[e][1,4] diazepin-3-yl)-benzamide;
(S)-4-Cyano-N-(2-oxo-5-phenyl-2,3-dihydro-1H-benzo[e][1,4]diazepin-3-yl)-2-pyrrolidin-1-yl-benzamide;
(S)-4-Cyano-N-(2-oxo-5-phenyl-2,3-dihydro-1H-benzo[e][1,4]diazepin-3-yl)-piperidine-1-yl-benzamide;
(S)-N-(2-Oxo-5-phenyl-2,3-dihydro-1H-benzo[e][1,4]diazepin-3-yl)-2-pyrrolidin-1-yl-4-trifluoromeflhyl-benzamide;
(S)-N-(2-Oxo-5-phenyl-2,3-dihydro-1H-benao[e][1,4]diazepin-3-yl)-2-piperidin-1-yl-4-trifluoromethyl-benzamide;
(S)-2-Morpholin-4-yl-N-(2-oxo-5-phenyl-2,3-dihydro-1H-benzo[e][1,4]diazepin-3-yl)-4-trifluoromethyl-benzamide;
(S)-N-(2-Oxo-5-phenyl-2,3-dihydro-1H-benzo[e][1,4]diazepin-3-yl)-2-pyrrolidin-1-yl-5-trifluoromethyl-benzamide;
(S)-2-Morpholin-4-yl-N-(2-oxo-5-phenyl-2,3-dihydro-1H-benzo[e][1,4]diazepin-3-yl)-5-trifluoromethyl-benzamide;
(S)-2-Morpholin-4-yl-N-(2-oxo-5-phenyl-2,3-dihydro-1H-benzo[e][1,4]diazepin-3-yl)-nicotinamide;
(S)-2-(1,1-Dioxo-1λ6-thiomorpholin-4-yl)-N-(2-oxo-5-phenyl-2,3-dihydro-1H-benzo[e][1,4]diazepin-3-yl)-nicotinamide;
(S)-2-(1,1-Dioxo-1λ6-thiomorpholm-4-yl)-2-methyl-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-4-methyl-N-(2-oxo-5-phenyl-2,3-dihydro-1H-benzo[e][1,4]diazepin-3-yl)-benzamide;
(S)-2-(1,1-Dioxo-1λ6-thiomorpholin-4-yl)-6-methyl-N-(2-oxo-5-phenyl-2,3-dihydro-1H-benzo[e][1,4]diazepin-3-yl)-benzamide;
(S)-2-Chloro-6-(1,1-dioxo-1λ6-thiomorpholin-4-yl)-N-(2-oxo-5-phenyl-2,3-dihydro-1H-benzo[e][1,4]diazepin-3-yl)-benzamide;
(S)-3-Cyclopropyl-2-oxo-2,3-dihydro-imidazo[4,5-b]pyridine-1-carboxylic acid (2-oxo-5-phenyl-2,3-dihydro-1H-benzo[e][1,4]diazepin-3-yl)-amide;
(S)-3-(4-Methyl-piperazine-1-sulfonyl)-N-(2-oxo-5-phenyl-2,3-dihydro-1H-benzo[e][1,4]diazepin-3-yl)-benzamide;
(S)-4-(4-Methyl-piperazin-1-yl)-N-(2-oxo-5-phenyl-2,3-dihydro-1H-benzo[e][1,4] diazepin-3-yl)-benzamide;
(S)-N-(2-Oxo-5-phenyl-2,3-dihydro-1H-benzo[e][1,4]diazepin-3-yl)-3-(piperidine-1-sulfonyl)-benzamide;
(S)-3-(Morpholine-4-sulfonyl)-N-(2-oxo-5-phenyl-2,3-dihydro-1H-benzo[e][1,4] diazepin-3-yl)-benzamide;
(S)-5-Morpholin-4-ylmethyl-furan-2-carboxylic acid (2-oxo-5-phenyl-2,3-dihydro-1H-benzo[e][1,4]diazxpin-3-yl)-amide;
(S)-5-Hydroxymethyl-furan-2-carboxylic acid (2-oxo-5-phenyl-2,3-dihydro-1H-benzo[e][1,4]diazepin-3-yl)-amide;
(S)-5-(1,1-Dioxo-1λ6-thiomorpholin-4-ylmethyl)-furan-2-carboxylic acid (2-oxo-5-phenyl-2,3-dihydro-1H-benzo[e][1,4]diazepin-3-yl)-amide;
(S)-2-chloro-4-(1,1-dioxo-1λ6-thiomorpholin-4-yl)-N-(2-oxo-5-phenyl-2,3-dihydro-1H-benzo[e][1,4]diazepin-3-yl)-benzamide;
(S)-2-Chloro-5-(1,1-dioxo-1λ6-thiomorpholin-4-yl)-N-(2-oxo-5-phenyl-2,3-dihydro-1H-benzo[e][1,4]diazepin-3-yl)-benzamide;
(S)-5-{[(2-Methanesulfonyl-ethyl)-methyl-amino]-methyl}-furan-2-carboxylic acid (2-oxo-5-phenyl-2,3-dihydro-1H-benzo[e][1,4]diazepin-3-yl-amide;
(S)-2-Pyridin-3-yl-thiazole-4-carboxylic acid (2-oxo-5-phenyl-2,3-dihydro-1H-benzo[e][1,4]diazepin-3-yl)-amide;
(S)-2-Pyridin-4-yl-thiazole-4-carboxylic acid (2-oxo-5-phenyl-2,3-dihydro-1H-benzo[e][1,4]diazepin-3-yl)-amide;
(S)-4-Methyl-2-pyrazin-2-yl-thiazole-5-carboxylic acid (2-oxo-5-phenyl-2,3-dihydro-1H-benzo[e][1,4]diazepin-3-yl)-amide;
(S)-2-Morpholin-4-ylmethyl-furan-3-carboxylic acid (2-oxo-5-phenyl-2,3-dihydro-1H. benzo[e][1,4]diazepin-3-yl)-amide;
(S)-3-Morpholin-4-ylmethyl-N-(2-oxo-5-phenyl-2,3-dihydro-1H-benzo[e][1,4] diazepin-3-yl)-benzamide;
(S)-5-Morpholin-4-ylmethyl-isoxazole-3-carboxylic acid (2-oxo-5-phenyl-2,3-dihydro-1H-benzo[e][1,4]diazepin-3-yl)-amide;
(S)-3-Morpholin-4-ylmtethyl-furan-2-carboxylic acid (2-oxo-5-phenyl-2,3-dihydro-1H-benzo[e][1,4]diazepin-3-yl)-amide;
(S)-5-Pyridin-2-yl-thiophene-2-carboxylic acid (2-oxo-5-phenyl-2,3-dihydro-1H-benzo[e][1,4]diazepin-3-yl)-amide;
(S)-2-Methyl-4-(morpholin-4-sulfonyl)-furan-3-carboxylic acid (2-oxo-5-phenyl-2,3-dihydro-1H-benzo[e][1,4]diazepin-3-yl)-amide;
(S)-6-Morpholin-4-yl-N-(2-oxo-5-phenyl-2,3-dihydro-1H-benzo[e][1,4]diazepin-3-yl)-nicotinamide;
(S)-3-Morpholin-4-ylmethyl-thiophene-2-carboxylic acid (2-oxo-5-phenyl-2,3-dihydro-1H-benzo[e][1,4]diazepin-3-yl)-amide;
(S)-S-Morpholin-4-ylmethyl-thiophene-2-carboxylic acid (2-oxo-5-phenyl-2,3-dihydro-1H-benzo[e][1,4]diazepin-3-yl)-amide;
2-Morpholin-4-yl-N-(2-oxo-5-phenyl-2,3-dihydro-1H-benzo[e][1,4]diazepin-3-yl)-benzamide;
(S)-5-Phenyl-oxazole-4 carboxylic acid (2-oxo-5-phenyl-2,3-dihydro-1H-benzo[e][1,4]diazepin-3-yl)-amide;
1-(2-Oxo-5-phenyl-2,3-dihydro-1H-benzo[e][1,4]diazepin-3-yl)-3-(4-phenoxy-phenyl)-urea an N-oxide of any of the above compounds;

or a pharmaceutically acceptable salt thereof.

**20.** A composition according to claim 1, wherein the benzodiazepine derivative of formula (V) is (S)-5-(1,1-Dioxo-1λ6-thiomorpholin-4-ylmethyl)-furan-2-carboxylic acid (2-oxo-5-phenyl-2,3-dihydro-1H-benzo[e][1,4]diazepin-3-yl)-amide or(S)-2-Chloro-4-morpholin-4-yl-N-(2-oxo-5-phenyl-2,3-dihydro-1H-benzo[e][1,4]diazepin-3-yl)-benzamide or a pharmaceutically acceptable salt thereof.

**21.** A composition according to claim 20, wherein the benzodiazepine derivative of formula (V) is (S)-5-(1,1-Dioxo-1λ6-thiomorpholin-4-ylmethyl)-furan-2-carboxylic acid (2-oxo-5-phenyl-2,3-dihydro-1H-benzo[e][1,4]diazepin-3-yl)-amide or a pharmaceutically acceptable salt thereof.

**22.** A composition according to any one of the preceding claims wherein component (a) is a compound of formula (I), or a pharmaceutically acceptable salt thereof,

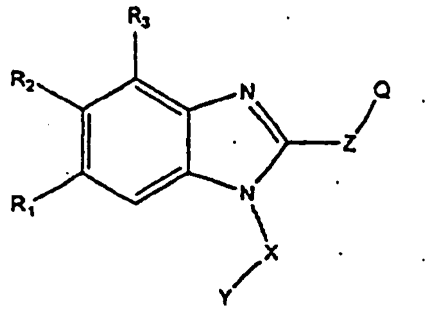

(I)

wherein:

- X is H or $C_{1-6}$ alkyl; said $C_{1-6}$ alkyl being optionally substituted with halogen, $OCOR_4$ or $S(O)_n$-$C_{1-6}$ alkyl;
- Y is $R_4$, $NR_4R_5$, $NCOR_4$, =N-$OR_4$, -$CONHR_4$, $COOR_4$, -$OR_4$, aryl, heteroaryl, cyclyl or heterocyclyl, where R4

and R5 are H or $C_{1-6}$ alkyl;
- Z is $CR_6R_7$, where $R_6$ and $R_7$ are independently H, or straight, branched or cyclic $C_{1-6}$ alkyl;
- n is 1-6;
- $R_1$ is $CONR_4R_5$. $CO_2R_4$ or $C_{1-6}$ alkyl, said $C_{1-6}$ alkyl can be optionally substituted with $OR_4$ or $NR_8R_9$;
- $R_8$ and $R_9$ are each independently H, $C_{1-6}$ alkyl, $SO_2R_5$ $CO_2R_4$ or $CON_4$;
- $R_2$ is selected from the group consisting of $NH_2$, $CONR_6R_7$, heteroaryl, $C_{2-6}$ alkenyl, $CO_2R_4$, $N=CPh_2$, C(=NH) $NH_2$ and $C_{1-6}$ alkyl; said alkyl optionally substituted with a member selected from the group consisting of halogen, CN, $NR_{10}R_{11}$, OSO2$R_4$ and $OR_4$;
- $R_{10}$ and $R_{11}$ are each independently selected from the group consisting of H, $C_{1-6}$ alkyl, $C_{3-6}$ cycloalkyl, $CO_2R_4$, $COR_4$ and $SO_2R_4$;
- $R_3$ is selected from the group consisting of (1) $CO_2R_9$; (2) $C_{1-6}$ alkyl optionally substituted with CN, $OR_4$ or $NR_6R_7$; and (3) $C_{2-6}$ alkenyl substituted with CN;
- Q is a member selected from the group consisting of

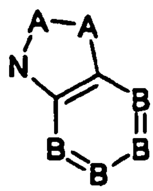

A is C or N, optionally substituted with H, halogen, straight, branched or cyclic $C_{1-6}$ alkyl, C2-6 alkenyl, $CO_2R_4$, aryl or $C_{3-6}$ cycloalkyl. Where A is carbon, it may also be optionally substituted by O or S via a double bond;
B is C or N; where B is C it may be optionally substituted by H, $C_{1-6}$ alkyl, $NO_2$, CN, halogen, $COR_4$, $COOR_4$, $CONHR_4C(=NH)NH_2$ or $C(=NOH)NH_2$.

**23.** A composition according to claim 22 wherein component (a) is a compound of general formula (I), as defined above, or a pharmaceutically acceptable salt thereof, wherein at least two of $R_1$, $R_2$ and $R_3$ are hydrogen, and the other is hydrogen or $-C(NH)-NH_2$ and/or -X-Y is H, or X is a $C_1$-$C_6$ alkylene group which is unsubstituted or substituted by a hydroxy group and Y is H, OH, CN, -NR'R", -COR', $-SO_2R'$ or phenyl, wherein R' and R" are the same or different and represent a $C_1$-$C_4$ alkyl group and/or Z is $-CH_2-$ and/or Q is a moiety

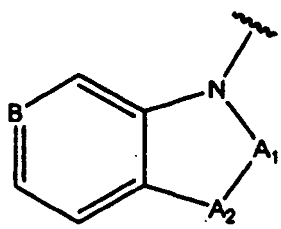

or

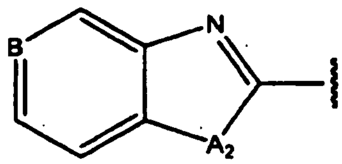

wherein B is -CH- or -N-, $A_1$ is -C(O)- or -NH- and $A_2$ is $-CH_2-$, -CHR'- or NR"-, wherein R' is a halogen atom and R" represents a hydrogen atom or a $C_1$-$C_4$ alkyl, $C_2$-$C_4$ alkenyl, $C_3$-$C_6$ cycloalkyl, $-SO_2-(C_1-C_6$ alkyl), $-SO_2-N(C_1-C_6$ alkyl$)_2$ or -(CO-NH),-(C,-Co alkyl)-phenyl group, wherein a is 0 or 1, which group is unsubstituted or is substituted with a hydroxy or cyano substituent.

**24.** A composition according to claims 1 to 21 wherein component (a) is a compound of formula (II), or a pharmaceutically acceptable salt thereof,

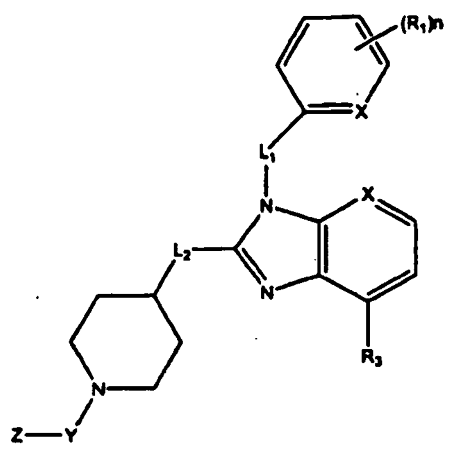

(II)

wherein:

- $L_1$ is $-CH_2-$ or $-CHR_2-CO-$
- each X is the same or different and CH or N;
- each $R_1$ is the same or different and is $C_{1-6}$ alkyl, halogen, hydroxy, phenyl or $(CH_2)_m=NH_2$;
- n is 1 or 2; 2;
- $R_2$ is $C_{1-6}$ alkoxy or $C_{1-6}$alkoxy-phenyl;
- $R_3$ is $C_{1-6}$alkyl;
- $L_2$ is $-CH_2-$ or -NH-;
- Y is $C_{1-6}$alkyl or $C_{1-6}$ alkenyl;
- Z is H, $N(R_4)_2$, $-C(=O)-R_5$, $-C(=CH_2)-R_5$, $-CH(OH)-R_5$, $-CH(CH_3)-R_5$, $-CH(OCH3)-R_5$;
- each $R_4$ is the same or different and is H, $C_{1-6}$ alkyl;
- $R_5$ is $C_{1-6}$ alkyl-carbonyl, amino, hydroxyl, aryl, heteroaryl, carbocyclyl, heterocyclyl; and
- m=1-6

**25.** A composition according to anyone of claims 1 to 21, wherein component (a) is:

1-Cyclopropyl-3-[1-(4-hydroxy-butyl)-1H-benzoimidazol-2-ylmethyl]-1,3-dihydro-imidazo[4,5-c]pyridin-2-one
{2-[2-(1,2-Dihydro-benzotriazol-1-ylmethyl)-benzoimidazol-1-yl]]ethyl}-diethylamine
{2-[2-(3-Iodo-2,3-dihydro-indazol-1-ylmethyl)-benamidazol-1-yl]-ethyl}-dimethylamine
1-Isopropenyl-3-[1-(3-methyl-butyl)-1H-benzoimidazol-2-ylmethyl]-1,3-dihydro-benzoimidazol-2-one
1-(4-Hydroxy-benzyl)-3-[1-(3-methyl-butyl)-1H-benzoimidazol-2-ylmethyl]-1,3-dihydro-benzoimidazol-2-one
1-Isopmpenyl-3-[1-(3-oxo-butyl)-1H-benzoimidazol-2-ylmethyl]-1,3-dihydro-benzoimidazol-2-one
1-Ethyl-3-[1-(2-hydroxy-2-phenyl-ethy)-1H-benzoimidazol-2-ylmethyl]-1,3-dibydro-benzoimidazol-2-one
1-Ethyl-3-[1-(4-hydroxy-butyl)-1H-benzoimidazol-2-ylmethyl]-1,3-dihydro-benzoimidazol-2-one
7-[2-(3-Isopropenyl-2-oxo-2,3-dihydrobenzoimidazol-1-ylmethyl)-benzoimidazol-1-yl]-heptanenitril
5- {3-[1-(3-Methanesulfonyl-propyl)-1H-benzoimidazol-2-ylmethyl]-2-oxo-2,3-dihydro-benzoimidazol-1-yl}-pentanenitrile
3-[1-(3-Methyl-butyl)-1H-benzoimidazol-2-ylmethyl]-2-oxo-2,3-dihydro-benzoimidazol-1-carboxylic acid benzylamide
1-Methanesulfonyl-3-[1-(3-methyl-butyl)-1H-benzoimidazol-2-ylmethyl]-1,3-dihydro-benzoimidazol-2-one
3-[1-(3-Methyl-butyl)-1H-benzoimidazol-2-ylmethyl]-2-oxo-2,3-dihydro-benzoimidazol-1-sulfonic acid dimethylamide
1-Isopropenyl-3-(1-propyl-1H-benzoimidazol-2-ylmethyl)-1,3-dihydro-imidazo[4,5-c]pyridine-2-one
Bis(5-amidino-2-benzimidazolyl)-methane
2-{2-[1-[1-(2-Amino-ethyl)-piperidin-4-ylamino]-4-methyl-benzoimidazol-1-ylmethyl}-6-methyl-pyridin-3-ol

or a pharmaceutically acceptable salt thereof.

**26.** A composition according to any one of claims 1 to 21, wherein component (a) is 1-cyclopropyl-3-[1-(4-hydroxy-butyl)-1H-benzoimidazol-2-ylmethyl]-1,3-dihydro-imidazo[4,5-c]pyridin-2-one, {2-[2-(1,2-dihydro-benzotriazol-1-ylmethyl)-benzoimidazol-1-yl]]ethyl}-diethyl-amine, {2-[2-(3-iodo-2,3-dihydro-indrol-1-ylmethyl)-benimidazol-1-yl]-ethyl}-dimethyl-amine or a pharmaceutically acceptable salt thereof.

**27.** A composition according to any one of claims 1 to 21, wherein component (a) is 1-cyclopropyl-3-[1-(4-hydroxybutyl)-1H-benzoimidazol-2-ylmethyl]-1,3-dihydro-imidazo[4,5-c]pyridin-2-one or 1-Isopropenyl-3-(1-propyl-1H-benzoimidazol-2-ylmethyl)-1,3-dihydro-inliduo[4,5-c]pyridine-2-one or a pharmaceutically acceptable salt thereof.

**28.** A composition according to any one of the preceding claims wherein component (a) is present in an amount of from 0.025 wt% to 10 wt%.

**29.** A composition according to any one of the preceding claims wherein component (b) is present in an amount of 0.025 wt% to 10 wt%.

**30.** A composition according to any one of the preceding claims, for use in the treatment of the human or animal body.

**31.** Use of:

(a) an RSV fusion protein inhibitor as defined in any one of claims 1 and 22 to 28; and
(b) a benzodiazepine derivative defined in any one of claims 1 to 21,

in the manufacture of a medicament for use in treating or preventing an RSV infection.

**32.** Use according to claim 31, wherein the medicament is a composition as defined in claim 28 or 29.

**33.** A product comprising:

(a) an RSV fusion protein inhibitor as defined in any one of claims 1 and 22 to 27; and
(b) a benzodiazepine derivative as defined in any one of claims 1 to 21;

for separate, simultaneous or sequential use in the treatment of the human or animal body.

**34.** A product according to claim 33 for separate, simultaneous or sequential use in treating or preventing an RSV infection.

**35.** Use of an RSV fusion protein inhibitor as defined in anyone of claims 22 to 27, in the manufacture of a medicament for use in treating or preventing an RSV infection, by co-administration with a benzodiazepine derivative as defined in any one of claims 1 to 21.

**36.** Use of a benzodiazepine derivative as defined in any one of claims 1 to 21, in the manufacture of a medicament for use in treating or preventing an RSV infection, by co-administration with an RSV fusion protein inhibitor as defined in any one of claims 22 to 27.

## Patentansprüche

**1.** Pharmazeutische Zusammensetzung, umfassend einen pharmazeutisch annehmbaren Träger und Verdünnungsmittel und:

(a) einen Inhibitor für das RSV-Fusionsprotein; und
(b) ein Benzodiazepinderivat, das in der Lage ist, die RSV-Replikation zu inhibieren,

worin die Komponente (b) eine Verbindung der Formel (V) oder ein pharmazeutisch annehmbares Salz davon ist:

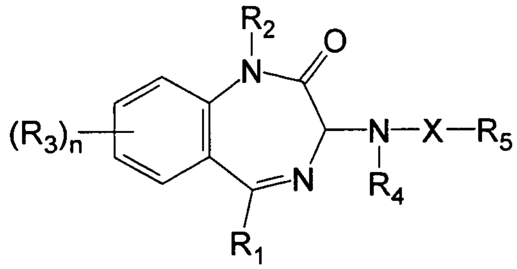

(V)

worin:

- $R^1$ $C_{1-6}$-Alkyl, Aryl oder Heteroaryl darstellt,
- $R^2$ Wasserstoff oder $C_{1-6}$-Alkyl darstellt,
- alle $R^3$ gleich oder verschieden sind und Halogen, Hydroxy, $C_{1-6}$-Alkyl, $C_{1-6}$-Alkoxy, $C_{1-6}$-Alkylthio, $C_{1-6}$-Halogenalkyl, $C_{1-6}$-Halogenalkoxy, Amino, Mono($C_{1-6}$-alkyl)-amino, Di($C_{1-6}$-alkyl)amino, Nitro, Cyano, $-CO_2R'$, -CONR'R", -NH-CO-R', -S(O)R', $-S(O)_2R'$, $-NH-S(O)_2R'$, -S(O)NR'R" oder $-S(O)_2NR'R"$ darstellen, worin R' und R" jeweils gleich oder verschieden sind und Wasserstoff oder $C_{1-6}$-Alkyl darstellen;
- n 0 bis 3 ist;
- $R^4$ Wasserstoff oder $C_{1-6}$-Alkyl darstellt;
- X -CO-, -CO-NR'-, -S(O)- oder $-S(O)_2-$ darstellt, worin R' Wasserstoff oder eine $C_{1-6}$-Alkylgruppe ist; und
- $R^5$ eine Aryl-, Heteroaryl- oder Heterocyclylgruppe darstellt, die mit einer $C_{1-6}$-Hydroxyalkylgruppe oder einer -($C_{1-4}$-Alkyl)-$X_1$-($C_{1-4}$-alkyl)-$X_2$-($C_{1-4}$-alkyl)-Gruppe substituiert ist, worin $X_1$ -O-, -S- oder -NR'- darstellt, worin R' H oder eine $C_{1-4}$-Alkylgruppe darstellt und $X_2$ -CO-, -SO- oder $-SO_2-$ darstellt, oder $R^5$ -$A_1$-Y-$A_2$ darstellt, worin:

  - $A_1$ eine Aryl-, Heteroaryl-, Carbocyclyl- oder Heterocyclylgruppe ist;
  - Y eine direkte Bindung oder eine $C_{1-4}$-Alkylen-, $-SO_2-$, -CO-, -O-, -S- oder -NR'-Einheit darstellt, worin R' eine $C_{1-6}$-Alkylgruppe ist; und
  - $A_2$ eine Aryl-, Heteroaryl-, Carbocyclyl- oder Heterocyclylgruppe ist.

**2.** Zusammensetzung gemäss Anspruch 1, worin $R^1$ $C_{1-2}$-Alkyl oder Phenyl ist.

**3.** Zusammensetzung gemäss entweder Anspruch 1 oder Anspruch 2, worin $R^2$ Wasserstoff ist.

**4.** Zusammensetzung gemäss einem der Ansprüche 1 bis 3, worin $R^3$ Halogen, Hydroxy, $C_{1-4}$-Alkyl, $C_{1-4}$-Alkoxy, $C_{1-4}$-Alkylthio, $C_{1-4}$-Halogenalkyl, $C_{1-4}$-Halogenalkoxy, Amino, Mono($C_{1-4}$-alkyl)amino oder Di($C_{1-4}$-alkyl)amino ist.

**5.** Zusammensetzung gemäss Anspruch 4, worin $R^3$ Fluor, Chlor, Brom, $C_{1-2}$-Alkyl, $C_{1-2}$-Alkoxy, $C_{1-2}$-Alkylthio, $C_{1-2}$-Halogenalkyl, $C_{1-2}$-Halogenalkoxy, Amino, Mono($C_{1-2}$-alkyl)amino oder Di($C_{1-2}$-alkyl)amirio ist.

**6.** Zusammensetzung gemäss einem der Ansprüche 1 bis 5, worin $R^4$ Wasserstoff oder $C_{1-2}$-Alkyl ist.

**7.** Zusammensetzung gemäss einem der Ansprüche 1 bis 6, worin X -CO- oder -CO-NR'- ist, worin R' Wasserstoff oder eine $C_{1-2}$-Alkylgruppe darstellt.

**8.** Zusammensetzung gemäss einem der Ansprüche 1 bis 7, worin $R^5$ ein 5- oder 6-gliedriges Heterocyclyl, Aryl oder ein Heteroarylring, der mit einer $C_{1-6}$-Hydroxyalkylgruppe substituiert ist, oder eine -($C_{1-4}$-Alkyl)-$X_1$-($C_{1-4}$-alkyl)-$X_2$-($C_{1-4}$-alkyl)-Gruppe ist, worin $X_1$ und $X_2$ wie in Anspruch 1 definiert sind.

**9.** Zusammensetzung gemäss Anspruch 8, worin $R^5$ eine 5- oder 6-gliedrige Heteroarylgruppe ist, die mit einem $-CH_2-OH-$ oder -($C_{1-4}$-Alkyl)-NR'-($C_{1-4}$-alkyl)-$S(O)_2$-($C_{1-4}$-alkyl)-Substituenten, worin R' Wasserstoff oder $C_{1-2}$-Alkyl ist, substituiert ist.

**10.** Zusammensetzung gemäss Ansprüchen 1 bis 9, worin $A_1$ eine Aryl- oder Heteroarylgruppe ist.

**11.** Zusammensetzung gemäss Anspruch 10, worin $A_1$ eine Phenylgruppe, eine monocyclische 5- oder 6-gliedrige Heteroarylgruppe oder eine 5- bis 6-gliedrige Heteroarylgruppe, die an eine monocyclische Oxosubstituierte 5- bis

6-gliedrige Heterocyclylgruppe kondensiert ist, darstellt.

**12.** Zusammensetzung gemäss Ansprüchen 1 bis 11, worin $A_1$ unsubstituiert oder mit 1 oder 2 Substituenten, ausgewählt aus Halogen, Cyano, Nitro, $C_{1-4}$-Alkyl, $C_{1-4}$-Halogenalkyl und $C_{1-4}$-Alkoxy, substituiert ist.

**13.** Zusammensetzung gemäss Ansprüchen 1 bis 12, worin Y eine direkte Bindung, eine $C_{1-2}$-Alkylengruppe, $-SO_2$- oder -0- darstellt.

**14.** Zusammensetzung gemäss Ansprüchen 1 bis 13, worin $A_2$ eine Phenyl-, eine 5- bis 6-gliedrige Heteroaryl-, eine 5- bis 6-gliedrige Heterocyclyl- oder eine $C_{3-6}$-Cycloalkylgruppe ist.

**15.** Zusammensetzung gemäss Ansprüchen 1 bis 14, worin, wenn $A_2$ eine Heterocyclylgruppe ist, diese über ein N-Atom an die Y-Einheit angefügt ist.

**16.** Zusammensetzung gemäss Ansprüchen 1 bis 15, worin $A_2$ unsubstituiert oder mit 1 oder 2 Substituenten substituiert ist, die aus $C_{1-4}$-Alkyl- und Halogensubstituenten ausgewählt sind, wenn $A_2$ eine Heteroaryl- oder Arylgruppe ist, und die aus $C_{1-4}$-Alkyl, Halogen und Oxosubstituenten ausgewählt sind, wenn $A_2$ eine carbocyclische oder heterocyclische Gruppe ist.

**17.** Zusammensetzung gemäss Ansprüchen 1 bis 16, worin $A_2$ eine Piperazinyl-, Pyridyl-, Morpholinyl-, Pyrrolidinyl-, Piperidinyl-, Pyrazinyl-, Cyclopropyl-, Phenyl- oder S,S-Dioxo-thiomorpholino-Gruppe, die unsubstituiert oder mit einer $C_{1-2}$-Alkylgruppe substituiert ist, darstellt.

**18.** Zusammensetzung gemäss einem der Ansprüchen 1 bis 17, worin das Benzodiazepinderivat der Formel (V) ein Benzodiazepinderivat der Formel (Va) ist:

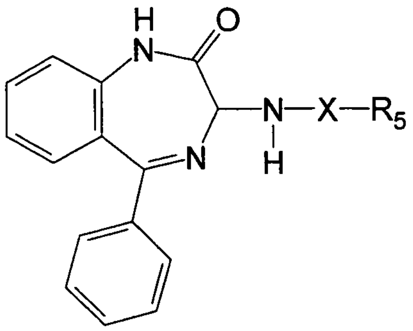

(Va)

worin

- X -CO- oder -CO-NH- ist; und
- $R^5$ eine 5- bis 6-gliedrige Heteroarylgruppe ist, z.B. eine Furanylgruppe, die mit $-CH_2$-OH oder -($C_{1-4}$-Alkyl)-N($CH_3$)-($C_{1-4}$-alkyl)-$SO_2$-($C_{1-4}$-alkyl) substituiert ist, oder $R^5$ stellt -$A_1$-Y-$A_2$ dar, worin:
  - $A_1$ eine Phenyl-, Pyridyl-, Furanyl-, Thiazolyl-, Oxazolyl-, Isoxazolyl-, Thienyl- oder 1H-Imidazo[4,5-b]pyridin-2-(3H)-on-Einheit, die unsubstituiert oder mit 1 oder 2 Substituenten, ausgewählt aus Halogen, Cyano, $C_{1-2}$-Alkyl, $C_{1-2}$-Halogenalkyl und $C_{1-2}$-Alkoxy-Substituenten, substituiert ist, darstellt;
  - Y eine direkte Bindung, eine $C_{1-2}$-Alkylengruppe, $-SO_2$- oder -O- ist; und
  - $A_2$ eine Piperazinyl-, Pyridyl-, Morpholinyl-, Pyrrolidinyl-, Piperidinyl-, Pyrazinyl-, Cyclopropyl-, Phenyl- oder S,S-Dioxo-thiomorpholino-Gruppe, die unsubstituiert oder mit einer $C_{1-2}$-Alkylgruppe substituiert ist, darstellt.

**19.** Zusammensetzung gemäss Anspruch 1, worin das Benzodiazepinderivat der Formel (V) ist:

6-(4-Methylpiperazin-1-yl)-N-(2-oxo-5-phenyl-2,3-dihydro-1H-benzo[e][1,4]diazepin-3-yl)-nikotinamid;
3,4,5,6-Tetrahydro-2H-[1,2']bipyridinyl-5'-carbonsäure-(2-oxo-5-phenyl-2,3-dihydro-1H-benzo[e][1,4]diazepin-3-yl)-amid;

(S)-2-(1,1-Dioxo-1λ6-thiomorpholin-4-yl)-N-(2-oxo-5-phenyl-2,3-dihydro-1H-benzo[e][1,4]diazepin-3-yl)-benzamid;
(S)-2-Chlor-4-morpholin-4-yl-N-(2-oxo-5-phenyl-2,3-dihydro-1H-benzo[e][1,4]diazepin-3-yl)-benzamid;
(S)-2-(1,1-Dioxo-1λ6-thiomorpholin-4-yl)-4-fluor-(2-oxo-5-phenyl-2,3-dihydro-1H-benzo[e][1,4]diazepin-3-yl)-benzamid;
(S)-5-Chlor-2-(1,1-dioxo-1λ6-thiomorpholin-4-yl)-N-(2-oxo-5-phenyl-2,3-dihydro-1H-benzo[e][1,4]diazepin-3-yl)-benzamid;
(S)-2-(1,1-Dioxo-1λ6-thiomorpholin-4-yl)-5-fluor-N-(2-oxo-5-phenyl-2,3-dihydro-1H-benzo[e][1,4]diazepin-3-yl)-benzamid;
(S)-5-(4-Methyl-piperazin-1-ylmethyl)-furan-2-carbonsäure-(2-oxo-5-phenyl-2,3-dihydro-1H-benzo[e][1,4]diazepin-3-yl)-amid;
(S)-5-Pyrrolidin-1-ylmethyl-furan-2-carbonsäure-(2-oxo-5-phenyl-2,3-dihydro-1H-benzo[e][1,4]diazepin-3-yl)-amid;
(S)-5-Piperidin-1-ylmethyl-furan-2-carbonsäure-(2-oxo-5-phenyl-2,3-dihydro-1H-benzo[e][1,4]diazepin-3-yl)-amid;
(S)-5-Dimethylaminomethyl-furan-2-carbonsäure-(2-oxo-5-phenyl-2,3-dihydro-1H-benzo[e][1,4]diazepin-3-yl)-amid;
(S)-4-Fluor-N-(2-oxo-5-phenyl-2,3-dihydro-1H-benzo[e][1,4]diazepin-3-yl)-2-piperidin-1-yl-benzamid;
(S)-4-Fluor-2-morpholino-4-yl-N-(2-oxo-5-phenyl-2,3-dihydro-1H-benzo[e][1,4]diazepin-3-yl)-benzamid;
(S)-4-Cyano-N-(2-oxo-5-phenyl-2,3-dihydro-1H-benzo[e][1,4]diazepin-3-yl)-2-pyrrolidin-1-yl-benzamid;
(S)-4-Cyano-N-(2-oxo-5-phenyl-2,3-dihydro-1H-benzo[e][1,4]diazepin-3-yl)-piperidin-1-yl-benzamid;
(S)-N-(2-Oxo-5-phenyl-2,3-dihydro-1H-benzo[e][1,4]-diazepin-3-yl)-2-pyrrolidin-1-yl-4-trifluormethyl-benzamid;
(S)-N-(2-Oxo-5-phenyl-2,3-dihydro-1H-benzo[e][1,4]-diazepin-3-yl)-2-piperidin-1-yl-4-trifluormethyl-benzamid;
(S)-2-Morpholin-4-yl-N-(2-oxo-5-phenyl-2,3-dihydro-1H-benzo[e][1,4]diazepin-3-yl)-4-trifluormethyl-benzamid;
(S)-N-(2-Oxo-5-phenyl-2,3-dihydro-1H-benzo[e][1,4]-diazepin-3-yl)-2-pyrrolidin-1-yl-5-trifluormethyl-benzamid;
(S)-2-Morpholin-4-yl-N-(2-oxo-5-phenyl-2,3-dihydro-1H-benzo[e][1,4]diazepin-3-yl)-5-trifluormethyl-benzamid;
(S)-2-Morpholin-4-yl-N-(2-oxo-5-phenyl-2,3-dihydro-1H-benzo[e][1,4]diazepin-3-yl)-nikotinamid;
(S)-2-(1,1-Dioxo-1λ6-thiomorpholin-4-yl)-N-(2-oxo-5-phenyl-2,3-dihydro-1H-benzo[e][1,4]diazepin-3-yl)-nikotinamid;
(S)-2-(1,1-Dioxo-1λ6-thiomorpholin-4-yl)-2-methyl-N-(2-oxo-5-phenyl-2,3-dihydro-1H-benzo[e][1,4]diazepin-3-yl)-benzamid;
(S)-2-(1,1-Dioxo-1λ6-thiomorpholin-4-yl)-4-methyl-N-(2-oxo-5-phenyl-2,3-dihydro-1H-benzo[e][1,4]diazepin-3-yl)benzamid;
(S)-2-(1,1-Dioxo-1λ6-thiomorpholin-4-yl)-6-methyl-N-(2-oxo-5-phenyl-2,3-dihydro-1H-benzo[e][1,4]diazepin-3-yl)-benzamid;
(S)-2-Chlor-6-(1,1-dioxo-1λ6-thiomorpholin-4-yl)-N-(2-oxo-5-phenyl-2,3-dihydro-1H-benzo[e][1,4]diazepin-3-yl)-benzamid;
(S)-3-Cyclopropyl-2-oxo-2,3-dihydro-imidazo[4,5-b]-pyridin-1-carbonsäure-(2-oxo-5-phenyl-2,3-dihydro-1H-benzo[e][1,4]diazepin-3-yl)-amid;
(S)-3-(4-Methyl-piperazin-1-sulfonyl)-N-(2-oxo-5-phenyl-2,3-dihydro-1H-benzo[e][1,4]diazepin-3-yl)-benzamid;
(S)-4-(4-Methyl-piperazin-1-yl)-N-(2-oxo-5-phenyl-2,3-dihydro-1H-benzo[e][1,4]diazepin-3-yl)-benzamid;
(S)-N-(2-oxo-5-phenyl-2,3-dihydro-1H-benzo[e][1,4]-diazepin-3-yl)-3-(piperidin-1-sulfonyl)-benzamid;
(S)-3-(Morpholin-4-sulfonyl)-N-(2-oxo-5-phenyl-2,3-dihydro-1H-benzo[e][1,4]diazepin-3-yl)-benzamid;
(S)-5-Morpholin-4-ylmethyl-furan-2-carbonsäure-(2-oxo-5-phenyl-2,3-dihydro-1H-benzo[e][1,4]diazepin-3-yl)-amid;
(S)-5-Hydroxymethyl-furan-2-carbonsäure-(2-oxo-5-phenyl-2,3-dihydro-1H-benzo[e][1,4]diazepin-3-yl)-amid;
(S)-5-(1,1-Dioxo-1λ6-thiomorpholin-4-ylmethyl)-furan-2-carbonsäure-(2-oxo-5-phenyl-2,3-dihydro-1H-benzo [e][1,4]diazepin-3-yl)-amid;
(S)-2-Chlor-4-(1,1-dioxo-1λ6-thiomorpholin-4-yl)-N-(2-oxo-5-phenyl-2,3-dihydro-1H-benzo[e][1,4]diazepin-3-yl)-benzamid;
(S)-2-Chlor-5-(1,1-dioxo-1λ6-thiomorpholin-4-yl)-N-(2-oxo-5-phenyl-2,3-dihydro-1H-benzo[e][1,4]diazepin-3-yl)-benzamid;
(S)-5-{[(2-Methansulfonyl-ethyl)-methylamino]-methyl}-furan-2-carbonsäure-(2-oxo-5-phenyl-2,3-dihydro-1H-

benzo[e][1,4]diazepin-3-yl)-amid;
(S)-2-Pyridin-3-yl-thiazol-4-carbonsäure-(2-oxo-5-phenyl-2,3-dihydro-1H-benzo[e][1,4]diazepin-3-yl)-amid;
(S)-2-Pyridin-4-yl-thiazol-4-carbonsäure-(2-oxo-5-phenyl-2,3-dihydro-1H-benzo[e][1,4]diazepin-3-yl)-amid;
(S)-4-Methyl-2-pyrazin-2-yl-thiazol-5-carbonsäure-(2-oxo-5-phenyl-2,3-dihydro-1H-benzo[e][1,4]diazepin-3-yl)-amid;
(S)-2-Morpholin-4-ylmethyl-furan-3-carbonsäure-(2-oxo-5-phenyl-2,3-dihydro-1H-benzo[e][1,4]diazepin-3-yl)-amid;
(S)-3-Morpholin-4-ylmethyl-N-(2-oxo-5-phenyl-2,3-dihydro-1H-benzo[e][1,4]diazepin-3-yl)-benzamid;
(S)-5-Morpholin-4-ylmethyl-isoxazol-3-carbonsäure-(2-oxo-5-phenyl-2,3-dihydro-1H-benzo[e][1,4]diazepin-3-yl)-amid;
(S)-3-Morpholin-4-ylmethyl-furan-2-carbonsäure-(2-oxo-5-phenyl-2,3-dihydro-1H-benzo[e][1,4]diazepin-3-yl)-amid;
(S)-5-Pyridin-2-yl-thiophen-2-carbonsäure-(2-oxo-5-phenyl-2,3-dihydro-1H-benzo[e][1,4]diazepin-3-yl)-amid;
(S)-2-Methyl-4-(morpholin-4-sulfonyl)-furan-3-carbonsäure-(2-oxo-5-phenyl-2,3-dihydro-1H-benzo[e][1,4]diazepin-3-yl)-amid;
(S)-6-Morpholin-4-yl-N-(2-oxo-5-phenyl-2,3-dihydro-1H-benzo[e][1,4]diazepin-3-yl)-nikotinamid;
(S)-3-Morpholin-4-ylmethyl-thiophen-2-carbonsäure-(2-oxo-5-phenyl-2,3-dihydro-1H-benzo[e][1,4]diazepin-3-yl)-amid;
(S)-5-Morpholin-4-ylmethyl-thiophen-2-carbonsäure-(2-oxo-5-phenyl-2,3-dihydro-1H-benzo[e][1,4]diazepin-3-yl)-amid;
2-Morpholin-4-yl-N-(2-oxo-5-phenyl-2,3-dihydro-1H-benzo[e][1,4]diazepin-3-yl)-benzamid;
(S)-5-Phenyl-oxazol-4-carbonsäure-(2-oxo-5-phenyl-2,3-dihydro-1H-benzo[e][1,4]diazepin-3-yl)-amid;
1-(2-Oxo-5-phenyl-2,3-dihydro-1H-benzo[e][1,4]-diazepin-3-yl)-3-(4-phenoxyphenyl)-harnstoff;
ein N-Oxid jeder der obigen Verbindungen;

oder ein pharmazeutisch annehmbares Salz davon.

**20.** Zusammensetzung gemäss Anspruch 1, worin das Benzodiazepinderivat der Formel (V) (S)-5-(1,1-Dioxo-1λ6-thiomorpholin-4-ylmethyl)-furan-2-carbonsäure(2-oxo-5-phenyl-2,3-dihydro-1H-benzo[e][1,4]diazepin-3-yl)-amid oder (S)-2-Chlor-4-morpholin-4-yl-N-(2-oxo-5-phenyl-2,3-dihydro-1H-benzo[e][1,4]diazepin-3-yl)-benzamid oder ein pharmazeutisch annehmbares Salz davon ist.

**21.** Zusammensetzung gemäss Anspruch 20, worin das Benzodiazepinderivat der Formel (V) (S)-5-(1,1-Dioxo-1λ6-thiomorpholin-4-ylmethyl)-furan-2-carbonsäure(2-oxo-5-phenyl-2,3-dihydro-1H-benzo[e][1,4]diazepin-3-yl)-amid oder ein pharmazeutisch annehmbares Salz davon ist.

**22.** Zusammensetzung gemäss einem der vorhergehenden Ansprüche, worin die Komponente (a) eine Komponente der Formel (I) oder ein pharmazeutisch annehmbares Salz davon ist:

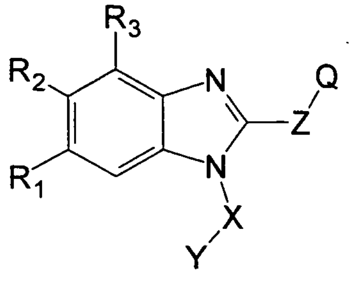

(I)

worin:

- X H oder $C_{1-6}$-Alkyl ist; wobei das $C_{1-6}$-Alkyl gegebenenfalls mit Halogen, $OCOR_4$ oder $S(O)_n$-$C_{1-6}$-Alkyl substituiert ist;
- Y $R_4$, $NR_4R_5$, $NCOR_4$, $=N-OR_4$, $-CONHR_4$, $COOR_4$, $-OR_4$, Aryl, Heteroaryl, Cyclyl oder Heterocyclyl ist, worin $R_4$ und $R_5$ H oder $C_{1-6}$-Alkyl sind;
- Z $CR_6R_7$ ist, worin $R_6$ und $R_7$ unabhängig voneinander H oder geradkettiges, verzweigtes oder cyclisches

$C_{1-6}$-Alkyl sind;
- n 1 bis 6 ist;
- $R_1$ $CONR_4R_5$, $CO_2R_4$ oder $C_{1-6}$-Alkyl ist, wobei das $C_{1-6}$-Alkyl gegebenenfalls mit $OR_4$ oder $NR_8R_9$ substituiert sein kann;
- $R_8$ und $R_9$ jeweils unabhängig voneinander H, $C_{1-6}$-Alkyl, $SO_2R_5$, $CO_2R_4$ oder $COR_4$ sind;
- $R_2$ ausgewählt ist aus der Gruppe bestehend aus $NH_2$, $CONR_6R_7$, Heteroaryl, $C_{2-6}$-Alkenyl, $CO_2R_4$, $N=CPh_2$, $C(=NH)NH_2$ und $C_{1-6}$-Alkyl; wobei das Alkyl gegebenenfalls mit einem Mitglied, ausgewählt aus der Gruppe bestehend aus Halogen, CN, $NR_{10}R_{11}$, $OSO_2R_4$ und $OR_4$, substituiert ist;
- $R_{10}$ und $R_{11}$ jeweils unabhängig ausgewählt sind aus der Gruppe bestehend aus H, $C_{1-6}$-Alkyl, $C_{3-6}$-Cycloalkyl, $CO_2R_4$, $COR_4$ und $SO_2R_4$;
- $R_3$ ausgewählt ist aus der Gruppe bestehend aus (1) $CO_2R_9$; (2) $C_{1-6}$-Alkyl, das gegebenenfalls mit CN, $OR_4$ oder $NR_6R_7$ substituiert ist; und (3) $C_{2-6}$-Alkenyl, das mit CN substituiert ist;
- Q ein Element ist, ausgewählt aus der Gruppe bestehend aus:

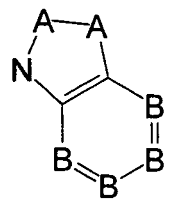

A C oder N ist, das gegebenenfalls mit H, Halogen, geradkettigem, verzweigtem oder cyclischem $C_{1-6}$-Alkyl, $C_{2-6}$-Alkenyl, $CO_2R_4$, Aryl oder $C_{3-6}$-Cycloalkyl substituiert ist; wenn A Kohlenstoff ist, kann es auch gegebenenfalls über eine Doppelbindung mit O oder S substituiert sein;
B C oder N ist; wenn B C ist, kann es gegebenenfalls mit H, $C_{1-6}$-Alkyl, $NO_2$, CN, Halogen, $COR_4$, $COOR_4$, $CONHR_4C(=NH)NH_2$ oder $C(=NOH)NH_2$ substituiert sein.

**23.** Zusammensetzung gemäss Anspruch 22, worin die Komponente (a) eine Verbindung der allgemeinen Formel (I), wie oben definiert, oder ein pharmazeutisch annehmbares Salz davon ist, worin zumindest zwei von $R_1$, $R_2$ und $R_3$ Wasserstoff sind und das andere Wasserstoff oder $-C(NH)-NH_2$ ist, und/oder -X-Y H ist, oder X eine $C_{1-6}$-Alkylengruppe ist, die unsubstituiert oder mit einer Hydroxygruppe substituiert ist, und Y H, OH, CN, -NR'R'', -COR', $-SO_2R'$ oder Phenyl ist, worin R' und R'' gleich oder verschieden sind und eine $C_{1-4}$-Alkylgruppe darstellen, und/oder Z $-CH_2-$ ist und/oder Q eine Einheit ist:

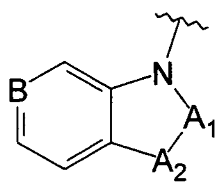
oder
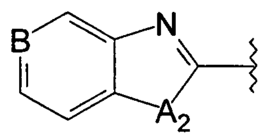

worin B -CH- oder -N- ist, $A_1$ -C(O)- oder -NH- ist und $A_2$ $-CH_2-$, -CHR'- oder -NR''- ist, worin R' ein Halogenatom ist und R'' ein Wasserstoffatom oder eine $C_{1-4}$-Alkyl-, $C_{2-4}$-Alkenyl-, $C_{3-6}$-Cycloalkyl-, $-SO_2-(C_{1-6}\text{-Alkyl})-$, $-SO_2-N(C_{1-6}\text{-Alkyl})_2-$ oder $-(CO-NH)_a-(C_{1-4}\text{-Alkyl})$-phenyl-Gruppe darstellt, worin a 0 oder 1 ist, wobei die Gruppe unsubstituiert oder mit einem Hydroxy- oder Cyanosubstituenten substituiert ist.

**24.** Zusammensetzung gemäss Ansprüchen 1 bis 21, worin die Komponente (a) eine Verbindung der Formel (II) oder ein pharmazeutisch annehmbares Salz davon ist:

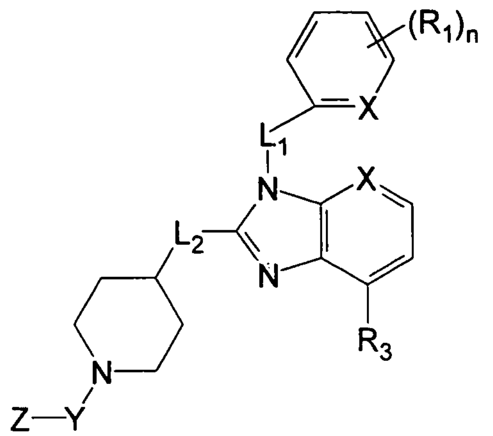

( II )

worin:

- $L_1$ $-CH_2-$ oder $-CHR_2-CO-$ ist;
- jedes X gleich oder verschieden und CH oder N ist;
- jedes $R_1$ gleich oder verschieden und $C_{1-6}$-Alkyl, Halogen, Hydroxy, Phenyl oder $(CH_2)_m=NH_2$ ist;
- n 1 oder 2 ist;
- $R_2$ $C_{1-6}$-Alkoxy oder $C_{1-6}$-Alkoxyphenyl ist;
- $R_3$ $C_{1-6}$-Alkyl ist;
- $L_2$ $-CH_2-$ oder -NH- ist;
- Y $C_{1-6}$-Alkyl oder $C_{1-6}$-Alkenyl ist;
- Z H, $N(R_4)_2$, $-C(=O)-R_5$, $-C(=CH_2)-R_5$, $-CH(OH)-R_5$, $-CH(CH_3)-R_5$ oder $-CH(OCH_3)-R_5$ ist;
- jedes $R_4$ gleich oder verschieden und H oder $C_{1-6}$-Alkyl ist;
- $R_5$ $C_{1-6}$-Alkyl-carbonyl, Amino, Hydroxyl, Aryl, Heteroaryl, Carbocyclyl, oder Heterocyclyl ist; und
- m = 1 bis 6 ist.

**25.** Zusammensetzung gemäss irgendeinem der Ansprüche 1 bis 21, worin die Komponente (a) ist:

1-Cyclopropyl-3-[1-(4-hydroxy-butyl)-1H-benzoimidazol-2-ylmethyl]-1,3-dihydroimidazo[4,5-c]pyridin-2-on;
{2-[2-(1,2-Dihydro-benzotriazol-1-ylmethyl)-benzoimidazol-1-yl]ethyl}-diethylamin;
{2-[2-(3-Iod-2,3-dihydro-indazol-1-ylmethyl)-benzimidazol-1-yl]-ethyl}-dimethylamin;
1-Isopropenyl-3-[1-(3-methylbutyl)-1H-benzoimidazol-2-ylmethyl]-1,3-dihydro-benzoimidazol-2-on;
1-(4-Hydroxybenzyl)-3-[1-(3-methylbutyl)-1H-benzoimidazol-2-ylmethyl]-1,3-dihydro-benzoimidazol-2-on;
1-Isopropenyl-3-[1-(3-oxobutyl)-1H-benzoimidazol-2-ylmethyl]-1,3-dihydro-benzoimidazol-2-on;
1-Ethyl-3-[1-(2-hydroxy-2-phenyl-ethyl)-1H-benzoimidazol-2-ylmethyl]-1,3-dihydro-benzoimidazol-2-on;
1-Ethyl-3-[1-(4-hydroxybutyl)-1H-benzoimidazol-2-ylmethyl]-1,3-dihydro-benzoimidazol-2-on;
7-[2-(3-Isopropenyl-2-oxo-2,3-dihydrobenzoimidazol-1-ylmethyl)-benzoimidazol-1-yl]-heptannitril;
5-{3-[1-(3-Methansulfonyl-propyl)-1H-benzoimidazol-2-ylmethyl]-2-oxo-2,3-dihydro-benzoimidazol-1-yl}-pentannitril;
3-[1-(3-Methylbutyl)-1H-benzoimidazol-2-ylmethyl]-2-oxo-2,3-dihydro-benzoimidazol-1-carbonsäurebenzylamid;
1-Methansulfonyl-3-[1-(3-methylbutyl)-1H-benzoimidazol-2-ylmethyl}-1,3-dihydro-benzoimidazol-2-on;
3-{1-(3-Methylbutyl)-1H-benzoimidazol-2-ylmethyl]-2-oxo-2,3-dihydro-benzoimidazol-1-sulfonsäuredimethylamid;
1-Isopropenyl-3-(1-propyl-1H-benzoimidazol-2-ylmethyl)-1,3-dihydro-imidazo[4,5-c]pyridin-2-on; Bis-(5-amidino-2-benzimidazolyl)-methan;
2-{2-[1-[1-(2-Aminoethyl)-piperidin-4-ylamino]-4-methyl-benzoimidazol-1-ylmethyll-6-methylpyridin-3-ol

oder ein pharmazeutisch annehmbares Salz davon.

**26.** Zusammensetzung gemäss irgendeinem der Ansprüche 1 bis 21, worin die Komponente (a) 1-Cyclopropyl-3-[1-(4-hydroxybutyl)-1H-benzoimidazol-2-ylmethyl]-1,3-dihydroimidazo[4,5-c]pyridin-2-on, {2-[2-(1,2-Dihydro-benzotriazol-1-ylmethyl)-benzoimidazol-1-yl]ethyl}-diethylamin, {2-[2-(3-Iod-2,3-dihydro-indazol-1-ylmethyl)-benzimidazol-1-yl]ethyl}-dimethylamin oder ein pharmazeutisch annehmbares Salz davon ist.

**27.** Zusammensetzung gemäss irgendeinem der Ansprüche 1 bis 21, worin die Komponente (a) 1-Cyclopropyl-3-[1-(4-hydroxybutyl)-1H-benzoimidazol-2-ylmethyl]-1,3-dihydroimidazo[4,5-c]pyridin-2-on oder 1-Isopropenyl-3-(1-propyl-1H-benzoimidazol-2-ylmethyl)-1,3-dihydroimidazo[4,5-c]pyridin-2-on oder ein pharmazeutisch annehmbares Salz davon ist.

**28.** Zusammensetzung gemäss irgendeinem der vorhergehenden Ansprüche, worin die Komponente (a) in einer Menge von 0,025 bis 10 Gew.% vorhanden ist.

**29.** Zusammensetzung gemäss irgendeinem der vorhergehenden Ansprüche, worin die Komponente (b) in einer Menge von 0,025 bis 10 Gew.% vorhanden ist.

**30.** Zusammensetzung gemäss irgendeinem der vorhergehenden Ansprüche, zur Verwendung bei der Behandlung eines menschlichen oder tierischen Körpers.

**31.** Verwendung:

(a) eines RSV-Fusionsproteininhibitors, wie in einem der Ansprüche 1 und 22 bis 28 definiert; und
(b) eines Benzodiazepinderivats, wie in einem der Ansprüche 1 bis 21 definiert,

bei der Herstellung eines Medikaments zur Verwendung bei der Behandlung oder Vorbeugung einer RSV-Infektion.

**32.** Verwendung gemäss Anspruch 31, worin das Medikament eine Zusammensetzung, wie in den Ansprüchen 28 oder 29 definiert, ist.

**33.** Produkt, umfassend:

(a) einen RSV-Fusionsproteininhibitor, wie in einem der Ansprüche 1 und 22 bis 27 definiert; und
(b) ein Benzodiazepinderivat, wie in einem der Ansprüche 1 bis 21 definiert,

zur getrennten, gleichzeitigen oder sequentiellen Verwendung bei der Behandlung eines menschlichen oder tierischen Körpers.

**34.** Produkt gemäss Anspruch 33, zur getrennten, gleichzeitigen oder sequentiellen Verwendung bei der Behandlung oder Vorbeugung einer RSV-Infektion.

**35.** Verwendung eines RSV-Fusionsproteininhibitors, wie in einem der Ansprüche 22 bis 27 definiert, bei der Herstellung eines Medikaments zur Verwendung bei der Behandlung oder Vorbeugung einer RSV-Infektion, durch gleichzeitige Verabreichung mit einem Benzodiazepinderivat, wie in einem der Ansprüche 1 bis 21 definiert.

**36.** Verwendung eines Benzodiazepinderivats, wie in einem der Ansprüche 1 bis 21 definiert, bei der Herstellung eines Medikaments zur Verwendung bei der Behandlung oder Vorbeugung einer RSV-Infektion, durch gleichzeitige Verabreichung mit einem RSV-Fusionsproteininhibitor, wie in einem der Ansprüche 22 bis 27 definiert.

## Revendications

**1.** Composition pharmaceutique qui comprend un support ou diluant pharmaceutiquement acceptable et :

(a) un inhibiteur de la protéine de fusion du RSV ; et
(b) un dérivé de benzodiazépine apte à inhiber la réplication du RSV,

dans laquelle le composant (b) est un composé de formule (V), ou un sel pharmaceutiquement acceptable de celui-ci,

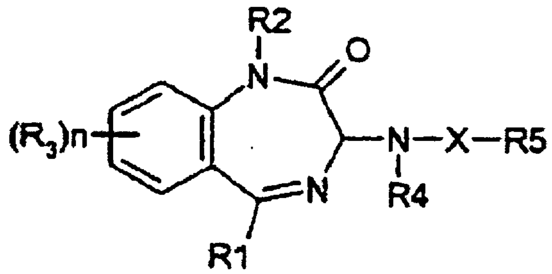

(V)

dans lequel

- $R^1$ représente un alkyle en $C_1$-$C_6$, un aryle ou un hétéroaryle ;
- $R^2$ représente un hydrogène ou un alkyle en $C_1$-$C_6$;
- chaque $R^3$ est identique ou différent et représente un halogène, un hydroxy, un alkyle en $C_1$-$C_6$, un alcoxy en $C_1$-$C_6$, un alkylthio en $C_1$-$C_6$, un haloalkyle en $C_1$-$C_6$, un haloalcoxy en $C_1$-$C_6$, un amino, un mono (alkyle en $C_1$-$C_6$)amino, un di (alkyle en $C_1$-$C_6$)amino, un nitro, un cyano, un $-CO_2R'$, un -CONR'R", un -NH-CO-R', un -S(O)R', un $-S(O)_2R'$, un- $NH-S(O)_2R'$, un -S(O)NR'R" ou un $-S(O)_2NR'R"$, dans lequel chaque R' et R" est identique ou différent et représente un hydrogène ou un alkyle en $C_1$-$C_6$ ;
- n est de 0 à 3 ;
- $R^4$ représente un hydrogène ou un alkyle en $C_1$-$C_6$ ;
- X représente un -CO-, un -CO-NR'-, un -S(O)- ou un
- $S(O)_2$-, dans lequel R' est un hydrogène ou un groupe alkyle en $C_1$-$C_6$ ; et
- $R^5$ représente un groupe aryle, hétéroaryle ou hétérocyclyle qui est substitué par un groupe hydroxyalkyle en $C_1$-$C_6$ ou un groupe -(alkyle en $C_1$-$C_4$)-$X_1$-(alkyle en $C_1$-$C_4$)-$X_2$-(alkyle en $C_1$-$C_4$), dans lequel $X_1$ représente un -O-, un -S- ou un -NR'-, dans lequel R' représente un H ou un groupe alkyle en $C_1$-$C_4$ et $X_2$ représente un -CO-, un -SO- ou un $-SO_2$-, ou $R_5$ représente un $-A_1$-Y-$A_2$, dans lequel :

  - $A_1$ est un groupe aryle, hétéroaryle, carbocyclyle ou hétérocyclyle ;
  - Y représente une liaison directe ou un groupe caractéristique alkylène en $C_1$-$C_4$, $-SO_2$-, -CO-, -O-, -S- ou -NR'-, dans lequel R' est un groupe alkyle en $C_1$-$C_6$ ; et
  - $A_2$ est un groupe aryle, hétéroaryle, carbocyclyle ou hétérocyclyle.

**2.** Composition selon la revendication 1 dans laquelle $R^1$ est un alkyle en $C_1$-$C_2$ ou un phényle.

**3.** Composition selon la revendication 1 ou la revendication 2 dans laquelle $R^2$ est un hydrogène.

**4.** Composition selon l'une quelconque des revendications 1 à 3 dans laquelle $R^3$ est un halogène, un hydroxy, un alkyle en $C_1$-$C_4$, un alcoxy en $C_1$-$C_4$, un alkylthio en $C_1$-$C_4$, un haloalkyle en $C_1$-$C_4$, un haloalcoxy en $C_1$-$C_4$, un amino, un mono(alkyle en $C_1$-$C_4$)amino ou un di(alkyle en $C_1$-$C_4$) amino.

**5.** Composition selon la revendication 4 dans laquelle $R^3$ est un fluor, un chlore, un brome, un alkyle en $C_1$-$C_2$, un alcoxy en $C_1$-$C_2$, un alkylthio en $C_1$-$C_2$, un haloalkyle en $C_1$-$C_2$, un haloalcoxy en $C_1$-$C_2$, un amino, un mono(alkyle en $C_1$-$C_2$)amino ou un di(alkyle en $C_1$-$C_2$)amino.

**6.** Composition selon l'une quelconque des revendications 1 à 5, dans laquelle $R^4$ est un hydrogène ou un alkyle en $C_1$-$C_2$.

**7.** Composition selon l'une quelconque des revendications 1 à 6, dans laquelle X est un -CO- ou un -CO-NR'- dans lequel R' représente un hydrogène ou un alkyle en $C_1$-$C_2$.

**8.** Composition selon l'une quelconque des revendications 1 à 7, dans laquelle $R^5$ est un cycle hétérocyclyle, aryle ou hétéroaryle à 5 ou 6 éléments qui est substitué par un groupe hydroxyalkyle en $C_1$-$C_6$ ou un groupe - (alkyle en $C_1$-$C_4$)-$X_1$-(alkyle en $C_1$-$C_4$)-$X_2$-(alkyle en $C_1$-$C_4$), dans lequel $X_1$ et $X_2$ sont tels que définis dans la revendication 1.

**9.** Composition selon la revendication 8, dans laquelle $R^5$ est un groupe hétéroaryle à 5 ou 6 éléments qui est substitué par un substituant $-CH_2-OH-$ ou -(alkyle en $C_1$-$C_4$)-NR'-(alkyle en $C_1$-$C_4$)-$S(O)_2$-(alkyle en $C_1$-$C_4$), dans lequel R' est un hydrogène ou un alkyle en $C_1$-$C_2$.

**10.** Composition selon les revendications 1 à 9, dans laquelle $A_1$ est un groupe aryle ou hétéroaryle.

**11.** Composition selon la revendication 10, dans laquelle $A_1$ est un groupe phényle, un groupe hétéroaryle à 5 ou 6 éléments monocyclique ou un groupe hétéroaryle à 5 à 6 éléments fusionné à un groupe hétérocyclyle à 5 à 6 éléments substitué par un oxo monocyclique.

**12.** Composition selon les revendications 1 à 11, dans laquelle $A_1$ est non substitué ou substitué par 1 ou 2 substituants choisis parmi des substituants halogène, cyano, nitro, alkyle en $C_1$-$C_4$, haloalkyle en $C_1$-$C_4$ et alcoxy en $C_1$-$C_4$.

**13.** Composition selon les revendications 1 à 12, dans laquelle Y représente une liaison directe, un groupe alkylène en $C_1$-$C_2$, un $-SO_2-$ ou un -0-.

**14.** Composition selon les revendications 1 à 13, dans laquelle $A_2$ est un groupe phényle, hétéroaryle à 5 à 6 éléments, hétérocyclyle à 5 à 6 éléments ou cycloalkyle en $C_3$-$C_6$.

**15.** Composition selon les revendications 1 à 14, dans laquelle lorsque $A_2$ est un groupe hétérocyclyle, il est attaché au groupe caractéristique Y par l'intermédiaire d'un atome de N.

**16.** Composition selon les revendications 1 à 15, dans laquelle $A_2$ est non substitué ou substitué par 1 ou 2 substituants qui sont choisis parmi des substituants alkyle en $C_1$-$C_4$ et halogène lorsque $A_2$ est un groupe hétéroaryle ou aryle et qui sont choisis parmi des substituants alkyle en $C_1$-$C_4$, halogène et oxo lorsque $A_2$ est un groupe carbocyclique ou hétérocyclyle.

**17.** Composition selon les revendications 1 à 16, dans laquelle $A_2$ est un groupe pipérazinyle, pyridyle, morpholinyle, pyrrolidinyle, pipéridinyle, pyrazinyle, cyclopropyle, phényle ou S,S-dioxo-thiomorpholino, qui est non substitué ou substitué par un groupe alkyle en $C_1$-$C_2$.

**18.** Composition selon l'une quelconque des revendications 1 à 17 dans laquelle le dérivé de benzodiazépine de formule (V) est un dérivé de benzodiazépine de formule (Va) :

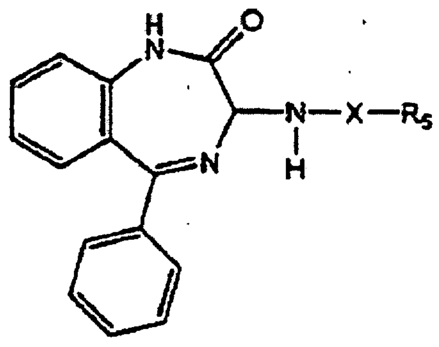

(Va)

dans lequel :

- X est un -CO- ou un -CO-NH- ; et
- $R^5$ est un groupe hétéroaryle à 5 à 6 éléments, par exemple un groupe furanyle, qui est substitué par un $-CH_2-OH$ ou un- (alkyle en $C_1$-$C_4$)-$N(CH_3)$-(alkyle en $C_1$-$C_4$)-$SO_2$-(alkyle en $C_1$-$C_4$), ou bien $R_5$ représente $-A_1-Y-A_2$, dans lequel :

  - $A_1$ est un groupe caractéristique phényle, pyridyle, furanyle, thiazolyle, oxazolyle, isoxazolyle, thiényle ou 1H-imidazo[4,5-b]pyridin-2-(3H)-one, qui est non substitué ou substitué par 1 ou 2 substituants choisis

parmi des substituants halogène, cyano, alkyle en $C_1$-$C_2$, haloalkyle en $C_1$-$C_2$ et alcoxy en $C_1$-$C_2$ ;
- Y est une liaison directe, un groupe alkylène en $C_1$-$C_2$, un -$SO_2$- ou un -O- ; et
- $A_2$ est un groupe pipérazinyle, pyridyle, morpholinyle, pyrrolidinyle, pipéridinyle, pyrazinyle, cyclopropyle, phényle ou S,S-dioxo-thiomorpholino, qui est non substitué ou substitué par un groupe alkyle en $C_1$-$C_2$.

**19.** Composition selon la revendication 1, dans laquelle le dérivé de benzodiazépine de formule (V) est :

le 6-(4-méthyl-pipérazin-1-yl)-N-(2-oxo-5-phényl-2,3-dihydro-1H-benzo[e][1,4]diazépin-3-yl)-nicotinamide ;
le (2-oxo-5-phényl-2,3-dihydro-1H-benzo[e][1,4]diazépin-3-yl)-amide d'un acide 3,4,5,6-tétrahydro-2H-[1,2']bipyridinyl-5'-carboxylique ;
le (S)-2-(1,1-dioxo-1λ6-thiomorpholin-4-yl)-N-(2-oxo-5-phényl-2,3-dihydro-1H-benzo[e][1,4]diazépin-3-yl)-benzamide ;
le (S)-2-chloro-4-morpholin-4-yl-N-(2-oxo-5-phényl-2,3-dihydro-1H-benzo[e][1,4]diazépin-3-yl)-benzamide ;
le (S)-2-(1,1-dioxo-1λ6-thiomorpholin-4-yl)-4-fluoro-(2-oxo-5-phényl-2,3-dihydro-1H-benzo[e][1,4]diazépin-3-yl)-benzamide ;
le (S)-5-chloro-2-(1,1-dioxo-1λ6-thiomorpholin-4-yl)-N-(2-oxo-5-phényl-2,3-dihydro-1H-benzo[e][1,4]diazépin-3-yl)-benzamide ;
le (S)-2-(1,1-dioxo-1λ6-thiomorpholin-4-yl)-5-fluoro-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-5-(4-méthyl-pipérazin-1-ylméthyl)-furan-2-carboxylique ;
le (2-oxo-5-phényl-2,3-dihydro-1H-benzo[e][1,4]diazépin-3-yl)-amide d'un acide (S)-5-pyrrolidin-1-ylméthyl-furan-2-carboxylique ;
le (2-oxo-5-phényl-2,3-dihydro-1H-benzo[e][1,4]diazépin-3-yl)-amide d'un acide (S)-5-pipéridin-1-ylméthyl-furan-2-carboxylique ;
le (2-oxo-5-phényl-2,3-dihydro-1H-benzo[e][1,4]diazépin-3-yl)-amide d'un acide (S)-5-diméthylaminométhyl-furan-2-carboxylique ;
le (S)-4-fluoro-N-(2-oxo-5-phényl-2,3-dihydro-1H-benzo[e][1,4]diazépin-3-yl)-2-pipéridin-1-yl-benzamide ;
le (S)-4-fluoro-2-morpholino-4-yl-N-(2-oxo-5-phényl-2,3-dihydro-1H-benzo[e][1,4]diazépin-3-yl)-benzamide ;
le (S)-4-cyano-N-(2-oxo-5-phényl-2,3-dihydro-1H-benzo[e][1,4]diazépin-3-yl)-2-pyrrolidin-1-yl-benzamide ;
le (S)-4-cyano-N-(2-oxo-5-phényl-2,3-dihydro-1H-benzo[e][1,4]diazépin-3-yl)-pipéridin-1-yl-benzamide :
le (S)-N-(2-oxo-5-phényl-2,3-dihydro-1H-benzo[e][1,4]diazépin-3-yl)-2-pyrrolidin-1-yl-4-trifluorométhyl-benzamide ;
le (S)-N-(2-oxo-5-phényl-2,3-dihydro-1H-benzo[e][1,4]diazépin-3-yl)-2-pipéridin-1-yl-4-trifluorométhyl-benzamide ;
le (S)-2-morpholin-4-yl-N-(2-oxo-5-phényl-2,3-dihydro-1H-benzo[e][1,4]diazépin-3-yl)-4-trifluorométhyl-benzamide ;
le (S)-N-(2-oxo-5-phényl-2,3-dihydro-1H-benzo[e][1,4]diazépin-3-yl)-2-pyrrolidin-1-yl-5-trifluorométhyl-benzamide ;
le (S)-2-morpholin-4-yl-N-(2-oxo-5-phényl-2,3-dihydro-1H-benzo[e][1,4]diazépin-3-yl)-5-trifluorométhyl-benzamide ;
le (S)-2-morpholin-4-yl-N-(2-oxo-5-phényl-2,3-dihydro-1H-benzo[e][1,4]diazépin-3-yl)-nicotinamide ;
le (S)-2-(1,1-dioxo-1λ6-thiomorpholin-4-yl)-N-(2-oxo-5-phényl-2,3-dihydro-1H-benzo[e][1,4]diazépin-3-yl)-nicotinamide ;
le (S)-2-(1,1-dioxo-1λ6-thiomorpholin-4-yl)-2-méthyl-N-(2-oxo-5-phényl-2,3-dihydro-1H-benzo[e][1,4]diazé pin-3-yl)-benzamide ;
le (S)-2-(1,1-dioxo-1λ6-thiomorpholin-4-yl)-4-méthyl-N-(2-oxo-5-phényl-2,3-dihydro-1H-benzo[e][1,4]diazé pin-3-yl)-benzamide ;
le (S)-2-(1,1-dioxo-1λ6-thiomorpholin-4-yl)-6-méthyl-N-(2-oxo-5-phényl-2,3-dihydro-1H-benzo[e][1,4]diazé pin-3-yl)-benzamide ;
le (S)-2-chloro-6-(1,1-dioxo-1λ6-thiomorpholin-4-yl)-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-3-cyclopropyl-2-oxo-2,3-dihydro-imidazo[4,5-b]pyridin-1-carboxylique ;
le (S)-3-(4-méthyl-pipérazine-1-sulfonyl)-N-(2-oxo-5-phényl-2,3-dihydro-1H-benzo[e][1,4]diazépin-3-yl)-benzamide ;
le (S)-4-(4-méthyl-pipérazine-1-yl)-N-(2-oxo-5-phényl-2,3-dihydro-1H-benzo[e][1,4]diazépin-3-yl)-benzamide ;
le (S)-N-(2-oxo-5-phényl-2,3-dihydro-1H-benzo[e][1,4]diazépin-3-yl)-3-(pipéridine-1-sulfonyl)-benzamide ;

le (S)-3-(morpholine-4-sulfonyl)-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-5-morpholin-4-ylméthyl-furan-2-carboxylique ;
le (2-oxo-5-phényl-2,3-dihydro-1H-benzo[e][1,4]diazépin-3-yl)-amide d'un acide (S)-5-hydroxyméthyl-furan-2-carboxylique ;
le (2-oxo-5-phényl-2,3-dihydro-1H-benzo[e][1,4]diazépin-3-yl)-amide d'un acide (S)-5-(1,1-dioxo-1λ6-thiomorpholin-4-ylméthyl)-furan-2-carboxylique ;
le -(S)-2-chloro-4-(1,1-dioxo-1λ6-thiomorpholin-4-yl)-N-(2 oxo-5-phényl-2,3-dihydro-1H-benzo[e][1,4]diazépin-3-yl)-benzamide ;
le (S)-2-chloro-5-(1,1-dioxo-1λ6-thiomorpholin-4-yl)-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-5-{[(2-méthanesulfonyl-éthyl)-méthyl-amino]-méthyl}-furan-2-carboxylique ;
le (2-oxo-5-phényl-2,3-dihydro-1H-benzo[e][1,4]diazépin-3-yl)-amide d'un acide (S)-2-pyridin-3-yl-thiazole-4-carboxylique ;
le (2-oxo-5-phényl-2,3-dihydro-1H-benzo[e][1,4]diazépin-3-yl)-amide d'un acide (S)-2-pyridin-4-yl-thiazole-4-carboxylique ;
le (2-oxo-5-phényl-2,3-dihydro-1H-benzo[e][1,4]diazépin-3-yl)-amide d'un acide (S)-4-méthyl-2-pyrazin-2-yl-thiazole-5-carboxylique ;
le (2-oxo-5-phényl-2,3-dihydro-1H-benzo[e][1,4]diazépin-3-yl)-amide d'un acide (S)-2-morpholin-4-ylméthyl-furan-3-carboxylique ;
le (S)-3-morpholin-4-ylméthyl-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-5-morpholin-4-ylméthyl-isoxazole-3-carboxylique ;
le (2-oxo-5-phényl-2,3-dihydro-1H-benzo[e][1,4]diazépin-3-yl)-amide d'un acide (S)-3-morpholin-4-ylméthyl-furan-2-carboxylique ;
le (2-oxo-5-phényl-2,3-dihydro-1H-benzo[e][1,4]diazépin-3-yl)-amide d'un acide (S)-5-pyridin-2-yl-thiophène-2-carboxylique ;
le (2-oxo-5-phényl-2,3-dihydro-1H-benzo[e][1,4]diazépin-3-yl)-amide d'un acide (S)-2-méthyl-4-(morpholin-4-sulfonyl)-furan-3-carboxylique ;
le (S)-6-morpholin-4-yl-N-(2-oxo-5-phényl-2,3-dihydro-1H-benzo[e][1,4]diazépin-3-yl)-nicotinamide ;
le (2-oxo-5-phényl-2,3-dihydro-1H-benzo[e][1,4]diazépin-3-yl)-amide d'un acide (S)-3-morpholin-4-ylméthyl-thiophène-2-carboxylique ;
le (2-oxo-5-phényl-2,3-dihydro-1H-benzo[e][1,4]diazépin-3-yl)-amide d'un acide (S)-5-morpholin-4-ylméthyl-thiophène-2-carboxylique ;
le 2-morpholin-4-yl-N-(2-oxo-5-phényl-2,3-dihydro-1H-benzo[e][1,4]diazépin-3-yl)-benzamide ;
le (2-oxo-5-phényl-2,3-dihydro-1H-benzo[e][1,4]diazépin-3-yl)-amide d'un acide (S)-5-phényl-oxazole-4-carboxylique ;
la 1-(2-oxo-5-phényl-2,3-dihydro-1H-benzo[e][1,4]diazépin-3-yl)-3-(4-phénoxy-phényl)-urée ;
un N-oxyde de l'un quelconque des composés ci-dessus ;

ou un sel pharmaceutiquement acceptable de ceux-ci.

**20.** Composition selon la revendication 1, dans laquelle le dérivé de benzodiazépine de formule (V) est le (2-oxo-5-phényl-2,3-dihydro-1H-benzo[e][1,4]diazépin-3-yl)-amide d'un acide (S)-5-(1,1-dioxo-1λ6-thiomorpholin-4-ylméthyl)-furan-2-carboxylique ou le (S)-2-chloro-4-morpholin-4-yl-N-(2-oxo-5-phényl-2,3-dihydro-1H-benzo[e][1,4]diazépin-3-yl)-benzamide ou un sel pharmaceutiquement acceptable de ceux-ci.

**21.** Composition selon la revendication 20, dans laquelle le dérivé de benzodiazépine de formule (V) est le (2-oxo-5-phényl-2,3-dihydro-1H-benzo[e][1,4]diazépin-3-yl)-amide d'un acide (S)-5-(1,1-dioxo-1λ6-thiomorpholin-4-ylméthyl)-furan-2-carboxylique ou un sel pharmaceutiquement acceptable de celui-ci.

**22.** Composition selon l'une quelconque des revendications précédentes dans laquelle le composant (a) est un composé de formule (I), ou un sel pharmaceutiquement acceptable de celui-ci,

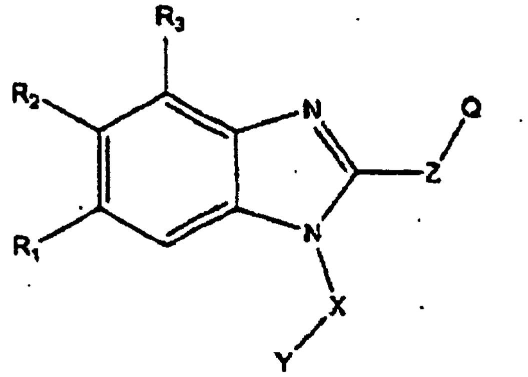

(I)

dans laquelle :

- X est un H ou un alkyle en $C_1$-$C_6$; ledit alkyle en $C_1$-$C_6$ étant facultativement substitué avec un halogène, un $OCOR_4$ ou un $S(O)_n$-alkyle en $C_1$-$C_6$ ;
- Y est un $R_4$, un $NR_4R_5$, un $NCOR_4$, un =N-$OR_4$, un -$CONHR_4$, un $COOR_4$, un -$OR_4$, un aryle, un hétéroaryle, un cyclyle ou un hétérocyclyle, où $R_4$ et $R_5$ sont un H ou un alkyle en $C_1$-$C_6$ ;
- Z est un $CR_6R_7$, où $R_6$ et $R_7$ sont indépendamment un H, un alkyle en $C_1$-$C_6$ à chaîne droite, ramifié ou cyclique ;
- n est 1 à 6 ;
- $R_1$ est un $CONR_4R_5$, un $CO_2R_4$ ou un alkyle en $C_1$-$C_6$, ledit alkyle en $C_1$-$C_6$ peut être facultativement substitué avec un $OR_4$ ou un $NR_8R_9$ ;
- $R_8$ et $R_9$ sont chacun indépendamment un H, un alkyle en $C_1$-$C_6$, un $SO_2R_5$, un $CO_2R_4$ ou $COR_4$ ;
- $R_2$ est choisi parmi le groupe consistant en un $NH_2$, un $CONR_6R_7$, un hétéroaryle, un alcényle en $C_2$-$C_6$, un $CO_2R_4$, un $N=CPh_2$, un $C(=NH)NH_2$ et un alkyle en $C_1$-$C_6$; ledit alkyle facultativement substitué avec un élément choisi parmi le groupe consistant en un halogène, un CN, un $NR_{10}R_{11}$, un $OSO_2R_4$ et un $OR_4$ ;
- $R_{10}$ et $R_{11}$ sont chacun indépendamment choisis parmi le groupe consistant en un H, un alkyle en $C_1$-$C_6$, un cycloalkyle en $C_3$-$C_6$, un $CO_2R_4$, un $COR_4$ et un $SO_2R_4$;
- $R_3$ est choisi parmi le groupe consistant en (1) un $CO_2R_9$ ; (2) un alkyle en $C_1$-$C_6$ facultativement substitué avec un CN, un $OR_4$ ou un $NR_6R_7$ ; et (3) un alcényle en $C_2$-$C_6$ substitué avec un CN ;
- Q est un élément choisi parmi le groupe consistant en

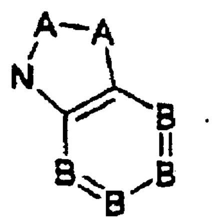

- A est un C ou un N, facultativement substitué avec un H, un halogène, un alkyle en $C_1$-$C_6$ à chaîne droite, ramifié ou cyclique, un alcényle en $C_2$-$C_6$, un $CO_2R_4$ un aryle ou un cycloalkyle en $C_3$-$C_6$. Lorsque A est un carbone, il peut également être facultativement substitué avec un O ou un S par l'intermédiaire d'une liaison double ;
- B est un C ou un N ; lorsque B est un C, il peut être facultativement substitué avec un H, un alkyle en $C_1$-$C_6$, un $NO_2$ un CN, un halogène, un $COR_4$, un $COOR_4$, un $CONHR_4C(=NH)NH_2$ ou un $C(=NOH)NH_2$.

**23.** Composition selon la revendication 22 dans laquelle le composant (a) est un composé de formule générale (I), tel que défini ci-dessus, ou un sel pharmaceutiquement acceptable de celui-ci, dans lequel au moins deux de $R_1$, $R_2$ et $R_3$ sont un hydrogène et l'autre est un hydrogène ou un -C(NH)-$NH_2$ et/ou -X-Y est un H, ou X est un groupe alkylène en $C_1$-$C_6$ qui est non substitué ou substitué par un groupe hydroxy et Y est un H, un OH, un CN, un -NR'R'', un -COR', un -$SO_2$R ou un phényle, dans lequel R' et R'' sont identiques ou différents et représentent un groupe

alkyle en $C_1$-$C_9$ et/ou Z est un $-CH_2-$ et/ou Q est un groupe caractéristique

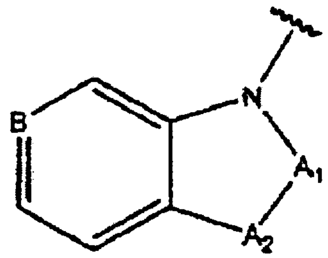

ou

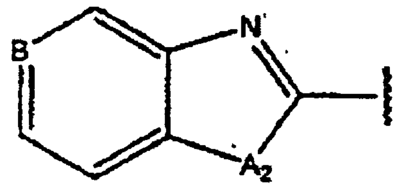

où B est un -CH- ou un -N-, $A_1$ est un -C(O)- ou un -NH- et $A_2$ est un $-CH_2-$, un -CHR'- ou un -NR"-, où R' est un atome d'halogène et R'' représente un atome d'hydrogène ou un groupe alkyle en $C_1$-$C_4$, alcényle en $C_2$-$C_4$, cycloalkyle en $C_3$-$C_6$, $-SO_2-$ (alkyle en $C_1$-$C_6$), $-SO_2-N$ (alkyle en $C_1$-$C_6$)$_2$ ou -(CO-NH)$_a$-(alkyle en $C_1$-$C_4$) -phényle, où a est 0 ou 1, lequel groupe est non substitué ou substitué avec un substituant hydroxy ou cyano.

**24.** Composition selon les revendications 1 à 21 dans laquelle le composant (a) est un composé de formule (II), ou un sel pharmaceutiquement acceptable de celui-ci,

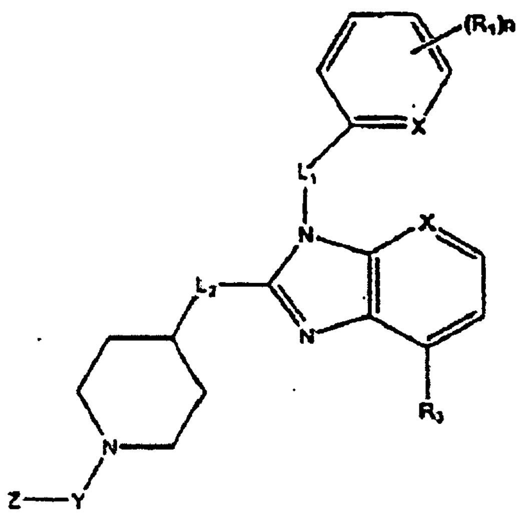

(II)

dans laquelle :

- $L_1$ est un $-CH_2-$ ou un $-CHR_2-CO-$ ;
- chaque X est identique ou différent et est un CH ou un N ;
- chaque $R_1$ est identique ou différent et est un alkyle en $C_1$-$C_6$, un halogène, un hydroxy, un phényle ou un $(CH_2)_m=NH_2$ ;
- n est 1 ou 2 ;
- $R_2$ est un alcoxy en $C_1$-$C_6$ ou un alcoxy en $C_1$-$C_6$-phényle ;
- $R_3$ est un alkyle en $C_1$-$C_6$ ;
- $L_2$ est un $-CH_2-$ ou un -NH- ;
- Y est un alkyle en $C_1$-$C_6$ ou un alcényle en $C_1$-$C_6$ ;
- Z est un H, un $N(R_4)_2$, un $-C(=O)-R_5$, un $-C(=CH_2)-R_5$, un $-CH(OH)-R_5$, un $-CH(CH_3)-R_5$; un $-CH(OCH_3)-R_5$ ;
- chaque $R_4$ est identique ou différent et est un H, un alkyle en $C_1$-$C_6$ ;
- $R_5$ est un alkyle en $C_1$-$C_6$-carbonyle, un amino, un hydroxyle, un aryle, un hétéroaryle, un carbocyclyle, un hétérocyclyle ; et

- m = 1 à 6.

**25.** Composition selon l'une quelconque des revendications 1 à 21, dans laquelle le composant (a) est :

la 1-cyclopropyl-3-[1-(4-hydroxy-butyl)-1H-benzoimidazol-2-ylméthyl]-1,3-dihydro-imidazo[4,5-c]pyridin-2-one ;
la {2-[2-(1,2-dihydro-benzotriazol-1-ylméthyl)benzoimidazol-l-yl]éthyl}-diéthyl-amine ;
la {2-[2-(3-iodo-2,3-dihydro-indazol-1-ylméthyl)benzimidazol-1-y1]éthyl}-diméthyl-amine ;
la 1-isopropényl-3-[1-(3-méthyl-butyl)-1H-benzoimidazol-2-ylméthyl]-1,3-dihydro-benzoimidazol-2-one ;
la 1-(4-hydroxy-benzyl)-3-[1-(3-méthyl-butyl)-1H-benzoimidazol-2-ylméthyl]-1,3-dihydro-benzoimidazol-2-one ;
la 1-isopropényl-3-[1-(3-oxo-butyl)-1H-benzoimidazol-2-ylméthyl]-1,3-dihydro-benzoimidazol-2-one ;
la 1-éthyl-3-[1-(2-hydroxy-2-phényl-éthyl)-1H-benzoimidazol-2-ylméthyl]-1,3-dihydro-benzoimidazol-2-one ;
la 1-éthyl-3-[1-(4-hydroxy-butyl)-1H-benzoimidazol-2-ylméthyl]-1,3-dihydro-benzoimidazol-2-one ;
le 7-[2-(3-isopropényl-2-oxo-2,3-dihydrobenzoimidazol-1-ylméthyl)-benzoimidazol-1-yl]heptanenitrile ;
le 5-{3-[1-(3-méthanesulfonyl-propyl)-1H-benzoimidazol-2-ylméthyl]-2-oxo-2,3-dihydrobenzoimidazol-1-yl}-pentanenitrile ;
le benzylamide d'un acide 3-[1-(3-méthyl-butyl)-1H-benzoimidazol-2-ylméthyl]-2-oxo-2,3-dihydro-benzoimidazol-1-carboxylique ;
la 1-méthanesulfonyl-3-[1-(3-méthyl-butyl)-1H-benzoimidazol-2-ylméthyl]-1,3-dihydro-benzoimidazol-2-one ;
le diméthylamide d'acide 3-[1-(3-méthyl-butyl)-1H-benzoimidazol-2-ylméthyl]-2-oxo-2,3-dihydro-benzoimidazol-1-sulfonique ;
la -l-isopropényl-3-(1-propyl-1H-benzoimidazol-2-ylméthyl] 1,3-dihydro-imidazo[4,5-c]pyridine-2-one ;
le bis(5-amidino-2-benzimidazolyl)-méthane ;
le 2-{2-[1-(2-amino-éthyl)-pipéridin-4-ylamino]-4-méthyl-benzoimidazol-1-ylméthy11-6-méthyl-pyridin-3-ol ;

ou un sel pharmaceutiquement acceptable de ceux-ci.

**26.** Composition selon l'une quelconque des revendications 1 à 21, dans laquelle le composant (a) est la 1-cyclopropyl-3-[1-(4-hydroxy-butyl)-1H-benzoimidazol-2-ylméthyl]-1,3-dihydro-imidazo[4,5-c]pyridin-2-one, la {2-[2-(1,2-dihydro-benzotriazol-1-ylméthyl)benzoimidazol-1-yl]éthyl}-diéthyl-amine, la {2-[2-(3-iodo-2,3-dihydro-indazol-1-ylméthyl)benzimidazol-1-ylléthyll-diméthyl-amine ou un sel pharmaceutiquement acceptable de celles-ci.

**27.** Composition selon l'une quelconque des revendications 1 à 21, dans laquelle le composant (a) est la 1-cyclopropyl-3-[1-(4-hydroxy-butyl)-1H-benzoimidazol-2-ylméthyl]-1,3-dihydro-imidazo[4,5-c]pyridin-2-one ou la 1-isopropényl-3-(1-propyl-1H-benzoimidazol-2-ylméthyl]- 1,3-dihydro-imidazo[4,5-c]pyridine-2-one ou un sel pharmaceutiquement acceptable de celles-ci.

**28.** Composition selon l'une quelconque des revendications précédentes dans laquelle le composant (a) est présent dans une quantité de 0,025% en poids à 10% en poids.

**29.** Composition selon l'une quelconque des revendications précédentes dans laquelle le composant (b) est présent dans une quantité de 0,025% en poids à 10% en poids.

**30.** Composition selon l'une quelconque des revendications précédentes, destinée à une utilisation dans le traitement du corps humain ou animal.

**31.** Utilisation de :

(a) un inhibiteur de la protéine de fusion du RSV tel que défini dans l'une quelconque des revendications 1 et 22 à 28 ; et
(b) un dérivé de benzodiazépine défini dans l'une quelconque des revendications 1 à 21,

dans la fabrication d'un médicament destiné à une utilisation dans le traitement ou la prévention d'une infection par le RSV.

**32.** Utilisation selon la revendication 31, dans laquelle le médicament est une composition telle que définie dans la revendication 28 ou 29.

**33.** Produit comprenant :

(a) un inhibiteur de la protéine de fusion du RSV tel que défini dans l'une quelconque des revendications 1 et 22 à 27 ; et
(b) un dérivé de benzodiazépine défini dans l'une quelconque des revendications 1 à 21 ;

pour une utilisation séparée, simultanée ou séquentielle dans le traitement du corps humain ou animal.

**34.** Produit selon la revendication 33 pour une utilisation séparée, simultanée ou séquentielle dans le traitement ou la prévention d'une infection par le RSV.

**35.** Utilisation d'un inhibiteur de la protéine de fusion du RSV tel que défini dans l'une quelconque des revendications 22 à 27, dans la fabrication d'un médicament destiné à une utilisation dans le traitement ou la prévention d'une infection par le RSV, par co-administration avec un dérivé de benzodiazépine tel que défini dans l'une quelconque des revendications 1 à 21.

**36.** Utilisation d'un dérivé de benzodiazépine tel que défini dans l'une quelconque des revendications 1 à 21, dans la fabrication d'un médicament destiné à une utilisation dans le traitement ou la prévention d'une infection par le RSV, par co-administration avec un inhibiteur de la protéine de fusion du RSV tel que défini dans l'une quelconque des revendications 22 à 27.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 0304050 W
- WO 00195910 A
- WO 00004900 A
- WO 03053344 A
- US 4324794 A
- WO 0100612 A

### Non-patent literature cited in the description

- **Morton, C.J. et al.** *Virology,* 2003, vol. 311, 275-288